# EUROPEAN PATENT SPECIFICATION

(11) **EP 3 679 158 B1**
(45) Date of publication and mention of the grant of the patent: **30.04.2025**
(21) Application number: 18779488.8
(22) Date of filing: 05.09.2018
(51) Int. Cl.: C12Q 1/6883

(54) **SINGLE IMMUNOGLOBULIN INTERLEUKIN-1 RECEPTOR RELATED (SIGIRR) VARIANTS AND USES THEREOF**
MIT SINGLE-IMMUNGLOBULIN-INTERLEUKIN-1-REZEPTOR (SIGIRR) ASSOZIERTE VARIANTEN UND DEREN VERWENDUNG
VARIANTS APPARENTÉS AU RÉCEPTEUR DE L'INTERLEUKINE 1 D'IMMUNOGLOBULINE UNIQUE (SIGIRR) ET LEURS UTILISATIONS

(30) Priority: 06.09.2017 US 201762554857 P
(43) Date of publication of application: 15.07.2020
(73) Proprietor: Regeneron Pharmaceuticals, Inc., Tarrytown, NY 10591 (US)
(72) Inventor: GONZAGA-JAUREGUI, Claudia G., Tarrytown, New York 10591 (US); HOROWITZ, Julie, Tarrytown, New York 10591 (US)
(74) Representative: Vossius & Partner Patentanwälte Rechtsanwälte mbB
(86) International application number: PCT/US2018/049478
(87) International publication number: WO 2019/050899

(56) References cited:
- ANONYMOUS: "Reference SNP Cluster Report rs752858122", DBSNP, 15 November 2018 (2018-11-15), XP055524782, Retrieved from the Internet <URL:https://www.ncbi.nlm.nih.gov/projects/SNP/snp_ref.cgi?do_not_redirect&rs=rs752858122> [retrieved on 20181119]
- ANONYMOUS: "Submitted SNP(ss) Details: ss1711956220", DBSNP, 4 March 2015 (2015-03-04), XP055524781, Retrieved from the Internet <URL:https://www.ncbi.nlm.nih.gov/projects/SNP/snp_ss.cgi?subsnp_id=ss1711956220> [retrieved on 20181119]
- XIONG HUIFANG ET AL: "The sinomenine enteric-coated microspheres suppressed the TLR/NF-[kappa]B signaling in DSS-induced experimental colitis", INTERNATIONAL IMMUNOPHARMACOLOGY, ELSEVIER, AMSTERDAM, NL, vol. 50, 12 July 2017 (2017-07-12), pages 251 - 262, XP085149780, ISSN: 1567-5769, DOI: 10.1016/J.INTIMP.2017.06.033
- THOMASSEN E ET AL: "Identification and characterization of SIGIRR, a molecule representing a novel subtype of the IL-1R superfamily", CYTOKINE, ACADEMIC PRESS LTD, PHILADELPHIA, PA, US, vol. 11, no. 6, 1 June 1999 (1999-06-01), pages 389 - 399, XP002332416, ISSN: 1043-4666, DOI: 10.1006/CYTO.1998.0452
- GARLANDA C ET AL: "TIR8/SIGIRR: an IL-1R/TLR family member with regulatory functions in inflammation and T cell polarization", TRENDS IN IMMUNOLOGY, ELSEVIER LTD. * TRENDS JOURNALS, GB, vol. 30, no. 9, 1 September 2009 (2009-09-01), pages 439 - 446, XP026502000, ISSN: 1471-4906, [retrieved on 20090821], DOI: 10.1016/J.IT.2009.06.001
- ZHAO JUNJIE ET AL: "Human Colon Tumors Express a Dominant-Negative Form of SIGIRR That Promotes Inflammation and Colitis-Associated Colon Cancer in Mice", GASTROENTEROLOGY, vol. 149, no. 7, 2015, pages 1860, XP029318958, ISSN: 0016-5085, DOI: 10.1053/J.GASTRO.2015.08.051
- SHUNJI ISHIHARA ET AL: "Inflammatory bowel disease: review from the aspect of genetics", JOURNAL OF GASTROENTEROLOGY, SPRINGER-VERLAG, TO, vol. 44, no. 11, 6 October 2009 (2009-10-06), pages 1097 - 1108, XP019761471, ISSN: 1435-5922, DOI: 10.1007/S00535-009-0141-8

## Description

### Field

The disclosure relates generally to the field of genetics. More particularly, the disclosure relates to gene alterations and polypeptide variants in the Single Immunoglobulin Interleukin-1 Receptor Related (SIGlRR) that associate with, for example, early-onset inflammatory bowel disease (EO-IBD).

### Background

Various references, including patents, patent applications, accession numbers, technical articles, and scholarly articles are cited throughout the specification.

Inflammatory bowel disease (IBD) is a genetically heterogeneous, chronic inflammatory disorder initiated by the inappropriate immune response to commensal microbiota in the gastrointestinal tract and with an average age of onset at 30 years. Severe, monogenic forms of IBD can present with pediatric age of onset (<18 years) and have been attributed to rare, highly-penetrant variants in about 50 'Mendelian' genes. However, the genetic architecture of early onset inflammatory bowel disease (EO-IBD) is poorly understood, and the majority of patients remain genetically undiagnosed.

Ishihara et al ("Inflammatory bowel disease: review from the aspect of genetics", JOURNAL OF GASTROENTEROLOGY, vol. 44, no. 11, 6 October 2009, pages 1097-1108) mentions that SIGIRR is located in an IBD susceptibility locus at chromosome 11p15.5.

The present disclosure provides novel SIGIRR variants that will aid in understanding the biology of SIGIRR, and will facilitate the diagnosis and treatment of children with early-onset inflammatory bowel disease.

### Summary

The invention is as defined in the appended claims.

The present disclosure provides novel nucleic acid molecules (i.e., genomic DNA, mRNA, and cDNA) encoding SIGIRR variant polypeptides, and SIGIRR variant polypeptides, that have been demonstrated herein to be associated with inflammatory bowel disease, such as early-onset inflammatory bowel disease.

The present disclosure provides isolated nucleic acid molecules comprising a nucleic acid sequence encoding a human SIGIRR protein, wherein the protein is truncated at a position corresponding to position 215 according to SEQ ID NO:9, or the complement of the nucleic acid sequence.

The present disclosure also provides genomic DNA molecules comprising a nucleic acid sequence encoding at least a portion of a human SIGIRR protein, wherein the protein is truncated at a position corresponding to position 215 according to SEQ ID NO:9, or the complement of the nucleic acid sequence.

The present disclosure also provides cDNA molecules comprising a nucleic acid sequence encoding at least a portion of a human SIGIRR protein, wherein the protein is truncated at a position corresponding to position 215 according to SEQ ID NO:9, or the complement of the nucleic acid sequence.

The present disclosure also provides mRNA molecules comprising a nucleic acid sequence encoding at least a portion of a human SIGIRR protein, wherein the protein is truncated at a position corresponding to position 215 according to SEQ ID NO:9, or the complement of the nucleic acid sequence.

The present disclosure also provides vectors comprising any of the isolated nucleic acid molecules disclosed herein.

The present disclosure also provides compositions comprising any of the isolated nucleic acid molecules or vectors disclosed herein and a carrier.

The present disclosure also provides host cells comprising any of the isolated nucleic acid molecules or vectors disclosed herein.

The present disclosure also provides isolated or recombinant polypeptides comprising at least a portion of the human SIGIRR protein, wherein the protein is truncated at a position corresponding to position 215 according to SEQ ID NO:9.

The present disclosure also provides compositions comprising any of the isolated or recombinant polypeptides disclosed herein and a carrier.

The present disclosure also provides a probe or a primer comprising a nucleic acid sequence comprising at least about 5 nucleotides, which hybridizes to a nucleic acid sequence encoding a human SIGIRR protein, wherein the protein is truncated at a position corresponding to position 215 according to SEQ ID NO:9, or which hybridizes to the complement of the nucleic acid sequence encoding the human SIGIRR protein, wherein the protein is truncated at a position corresponding to position 215 according to SEQ ID NO:9.

The present disclosure also provides supports comprising a substrate to which any of the probes disclosed herein hybridize.

The present disclosure also provides an alteration-specific probe or primer comprising a nucleic acid sequence which is complementary to a nucleic acid sequence encoding a SIGIRR protein truncated at a position corresponding to position 215 according to SEQ ID NO:9, wherein the alteration-specific probe or primer comprises a nucleic acid sequence which is complementary to a portion of the nucleic acid molecule encoding any plurality of positions corresponding to positions 186 to 209 or 211 to 215 according to SEQ ID NO:9. In some embodiments, the alteration-specific probe or primer specifically hybridizes to a portion of the nucleic acid molecule encoding a position corresponding to position 186 according to SEQ ID NO:9, or to the complement thereof. The alteration-specific probe or primer does not hybridize to a nucleic acid molecule having a nucleic acid sequence encoding a wild-type SIGIRR protein.

The present disclosure also provides methods for identifying a human subject having inflammatory bowel disease or early-onset inflammatory bowel disease or a risk for developing inflammatory bowel disease or early-onset inflammatory bowel disease, wherein the method comprises detecting in a sample obtained from the subject the presence or absence of: a truncated SIGIRR protein; and/or a nucleic acid molecule encoding a truncated SIGIRR protein; wherein the presence of the truncated SIGIRR protein and/or the nucleic acid molecule encoding the truncated SIGIRR protein indicates that the subject has inflammatory bowel disease or early-onset inflammatory bowel disease or a risk for developing inflammatory bowel disease or early-onset inflammatory bowel disease.

The present disclosure also provides methods for identifying a human subject having inflammatory bowel disease or early-onset inflammatory bowel disease or a risk for developing inflammatory bowel disease or early-onset inflammatory bowel disease, wherein the method comprises detecting in a sample obtained from the subject the presence or absence of: a SIGIRR protein truncated at a position corresponding to position 215 according to SEQ ID NO:9; and/or a nucleic acid molecule encoding a SIGIRR protein truncated at a position corresponding to position 215 according to SEQ ID NO:9; wherein the presence of the truncated SIGIRR protein and/or the nucleic acid molecule encoding the truncated SIGIRR protein indicates that the subject has inflammatory bowel disease or early-onset inflammatory bowel disease or a risk for developing inflammatory bowel disease or early-onset inflammatory bowel disease.

The present disclosure also provides methods for diagnosing inflammatory bowel disease or early-onset inflammatory bowel disease or detecting a risk of developing inflammatory bowel disease or early-onset inflammatory bowel disease in a human subject, comprising: detecting an alteration in a nucleic acid molecule encoding a SIGIRR protein obtained from the human subject, wherein the alteration encodes a truncated SIGIRR protein; and diagnosing the human subject with early-onset inflammatory bowel disease if the subject has one or more symptoms of inflammatory bowel disease or early-onset inflammatory bowel disease, or diagnosing the human subject as at risk for developing inflammatory bowel disease or early-onset inflammatory bowel disease if the subject does not have one or more symptoms of inflammatory bowel disease or early-onset inflammatory bowel disease.

The present disclosure also provides methods for diagnosing inflammatory bowel disease or early-onset inflammatory bowel disease or detecting a risk of developing inflammatory bowel disease or early-onset inflammatory bowel disease in a human subject, comprising: detecting an alteration in a nucleic acid molecule encoding a SIGIRR protein obtained from the human subject, wherein the alteration encodes a SIGIRR protein truncated at a position corresponding to position 215 according to SEQ ID NO:9; and diagnosing the human subject with early-onset inflammatory bowel disease if the subject has one or more symptoms of inflammatory bowel disease or early-onset inflammatory bowel disease, or diagnosing the human subject as at risk for developing inflammatory bowel disease or early-onset inflammatory bowel disease if the subject does not have one or more symptoms of inflammatory bowel disease or early-onset inflammatory bowel disease.

### Brief Description Of The Figures

The accompanying figures, which are incorporated in and constitute a part of this specification, illustrate several aspects and together with the description serve to explain the principles of the present disclosure.

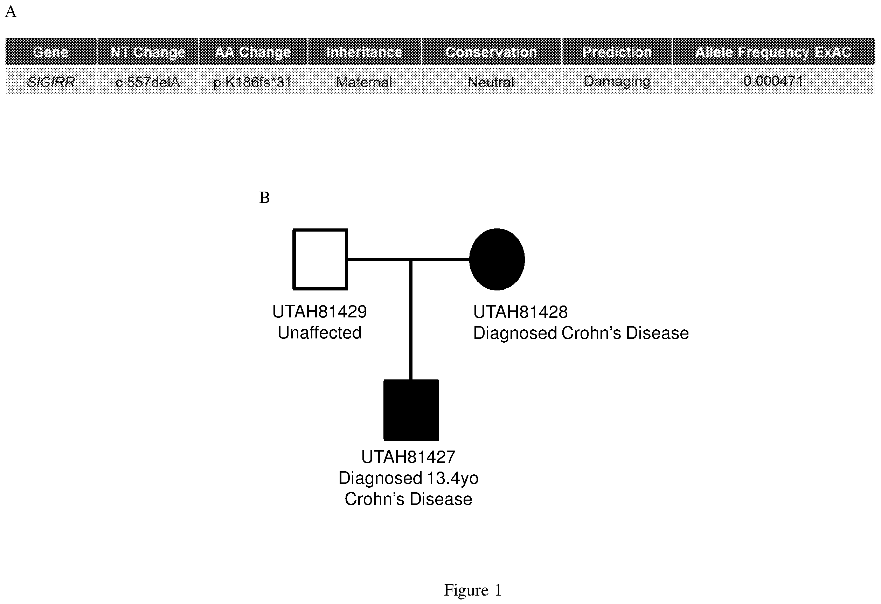

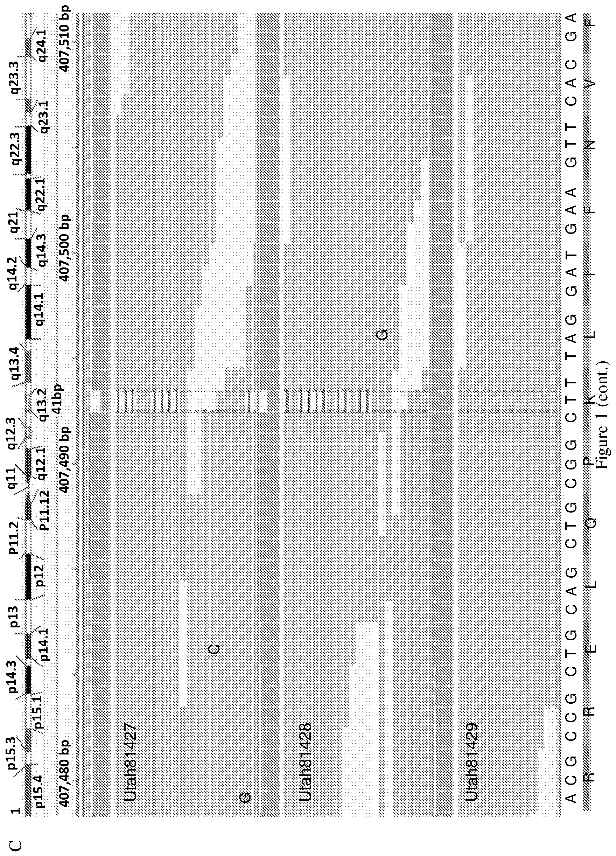

Figure 1, panels A, B, and C, show a truncating variant in SIGIRR with dominant segregation in a family with Crohn's Disease.

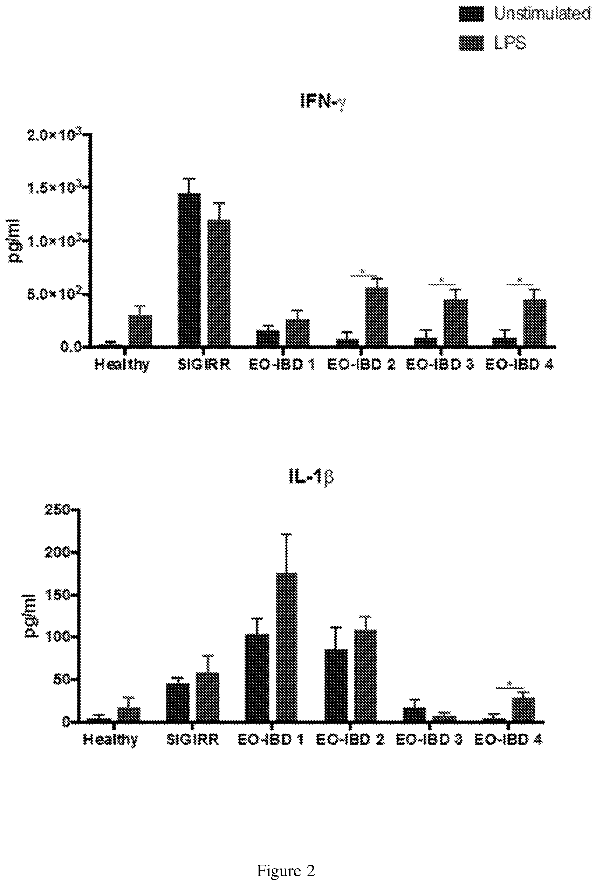

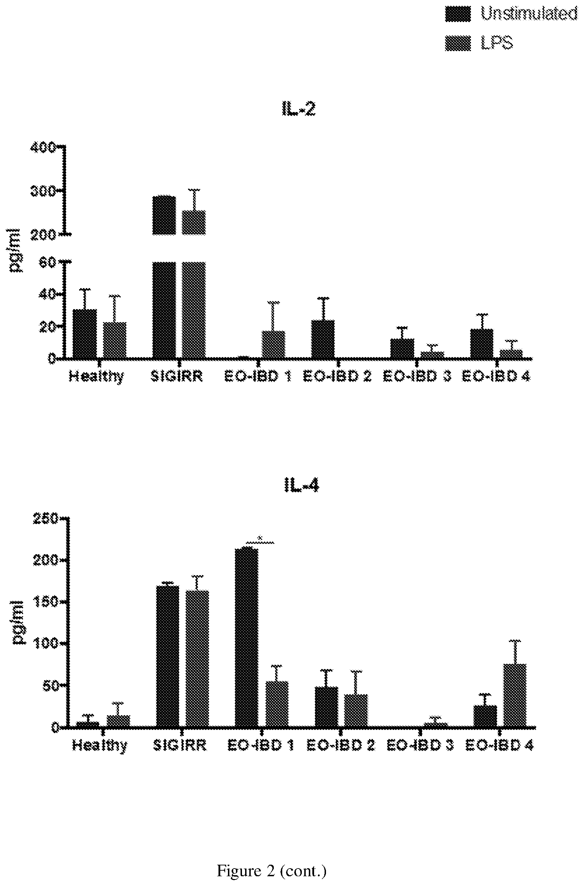

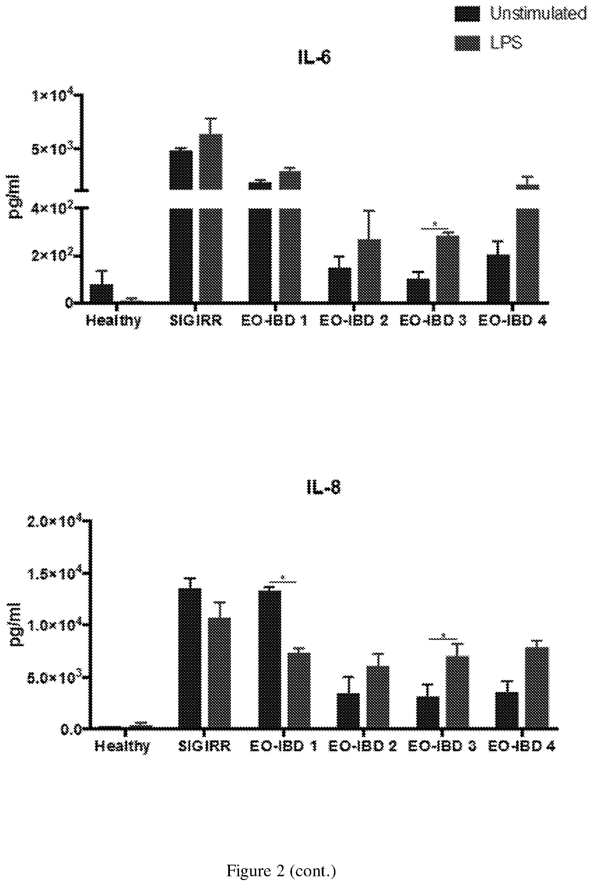

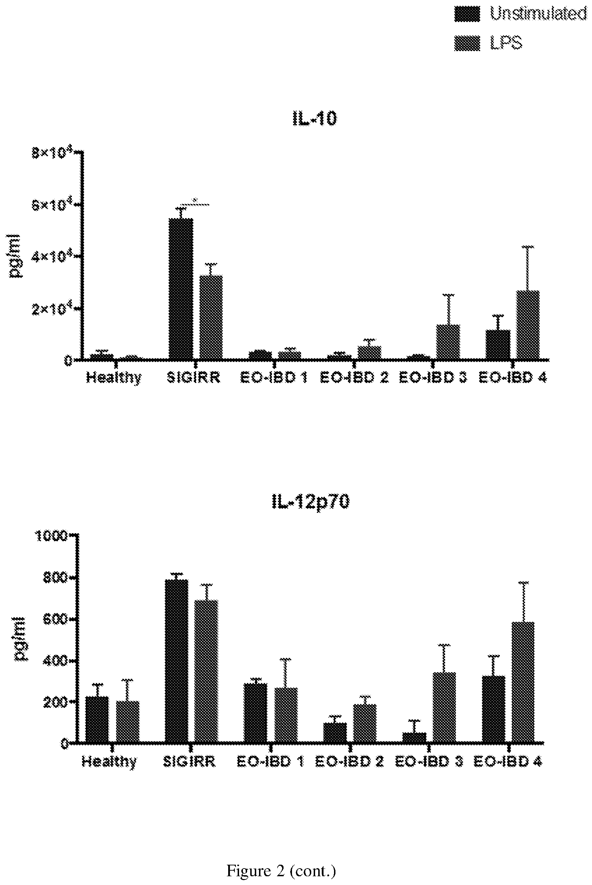

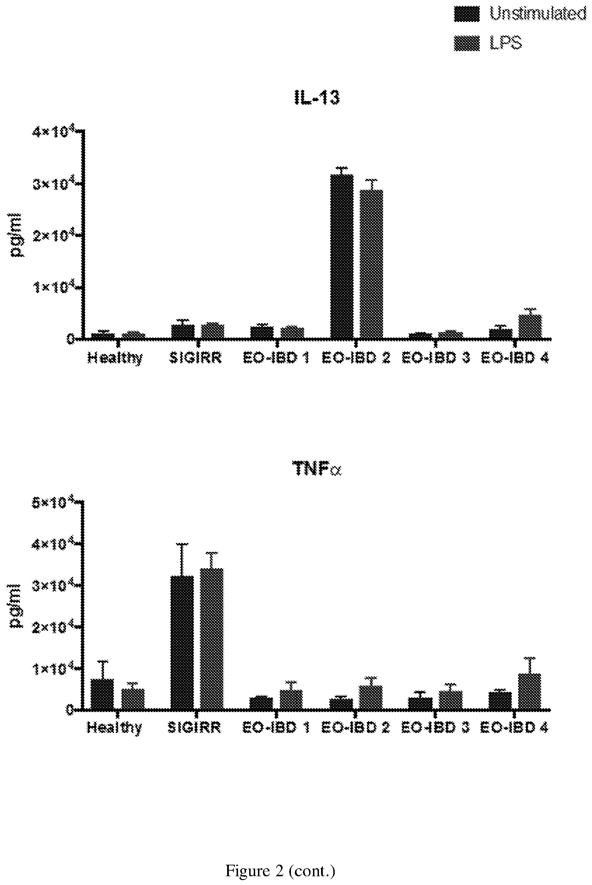

Figure 2 shows results from a Mesoscale Discovery Pro-Inflammatory Cytokine panel performed on cell culture supernatants taken from LCLs generated from healthy controls, the SIGIRR LoF patient, and from 4 EO IBD patients not harboring SIGIRR LoFs unstimulated or treated with 2 mg/ml of LPS for 72 hours.

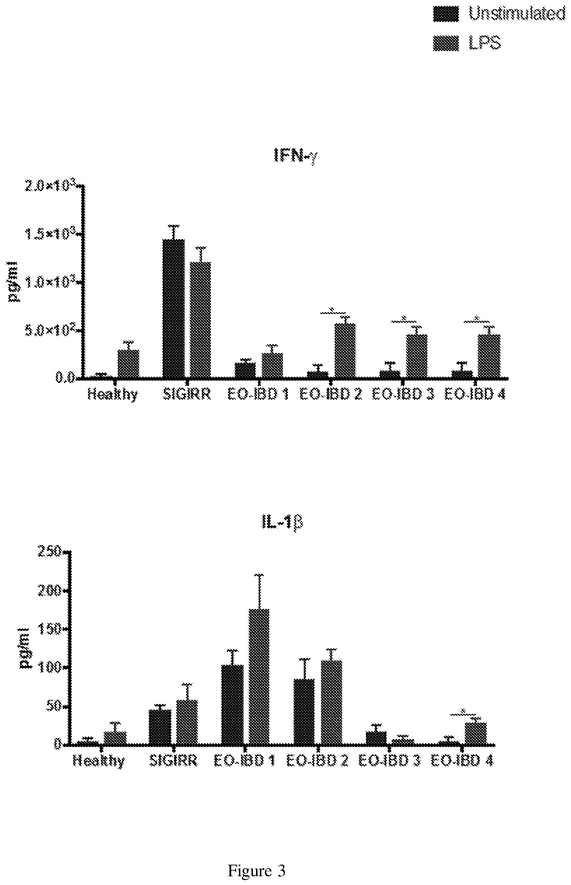

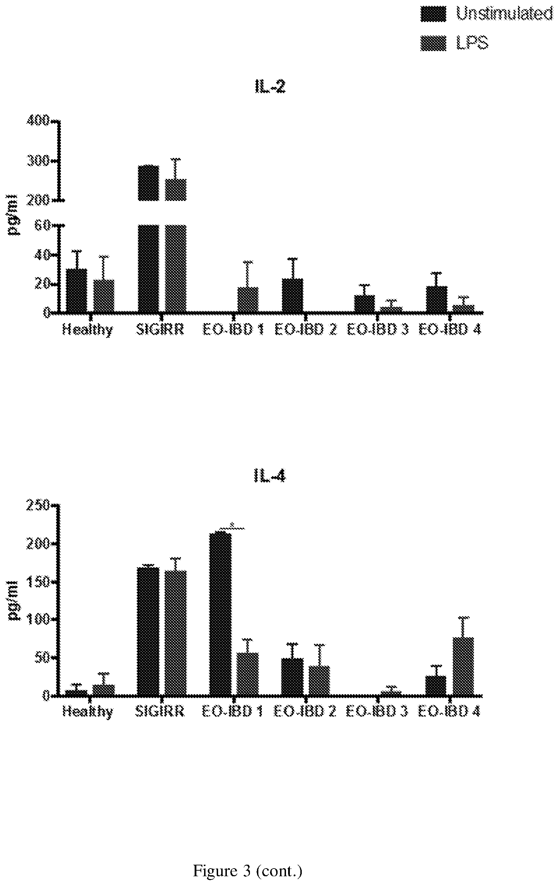

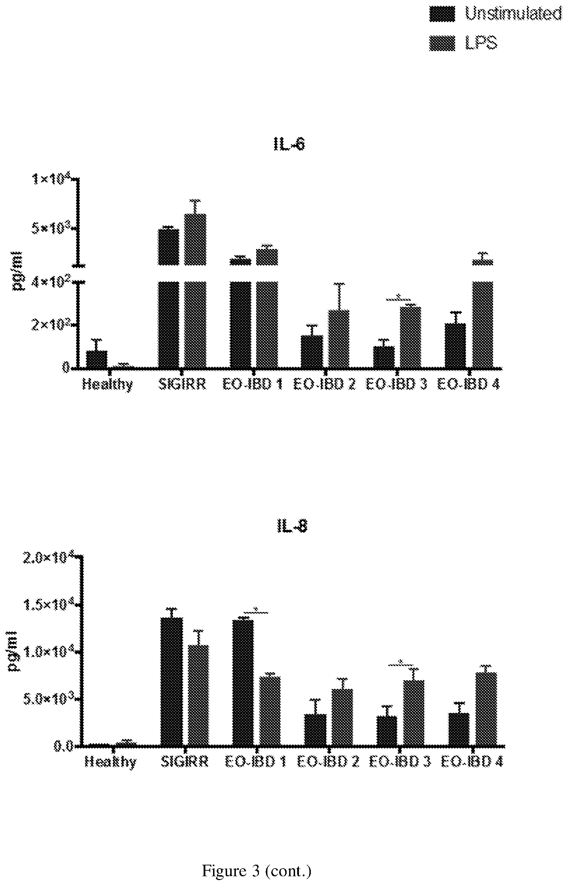

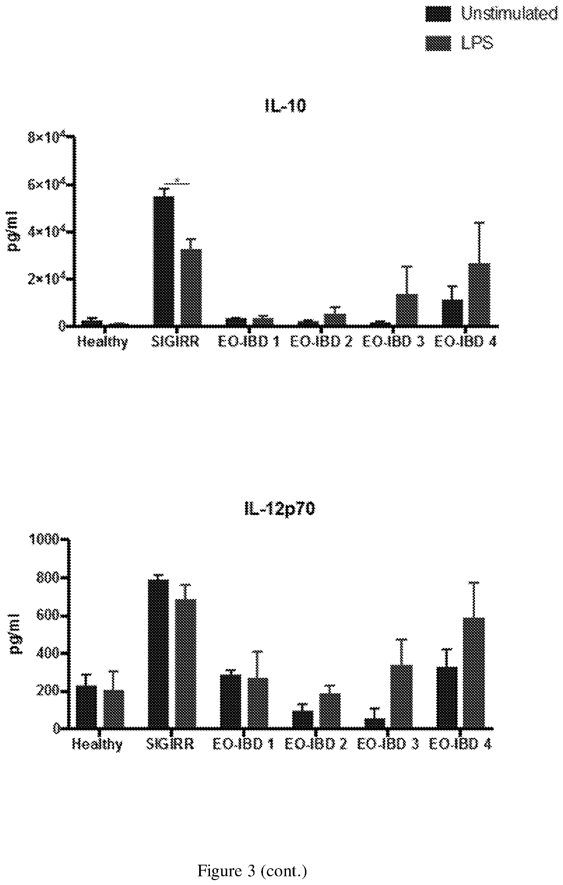

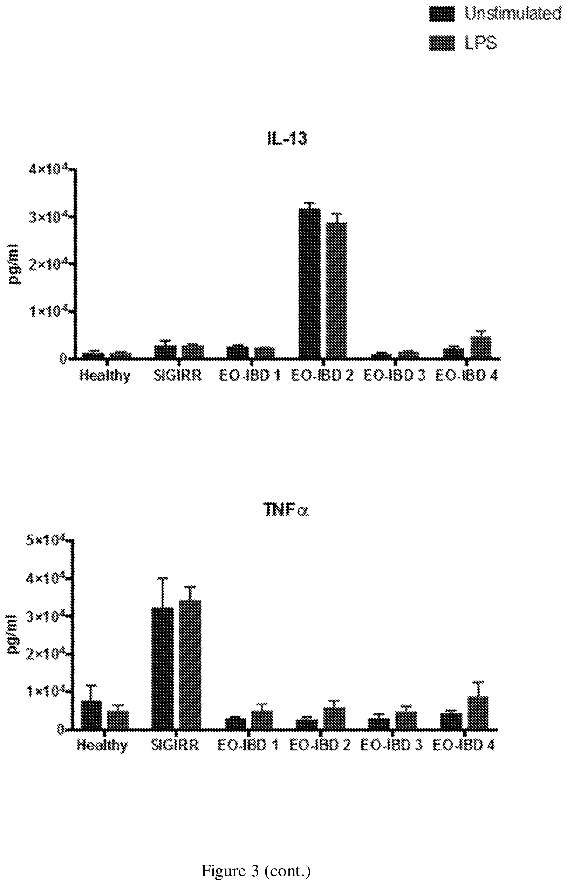

Figure 3 shows results from a Mesoscale Discovery Pro-Inflammatory Cytokine panel performed on cell culture supernatants taken from LCLs generated from healthy controls, the SIGIRR LoF patient, and from 4 EO IBD patients not harboring SIGIRR LoFs unstimulated or treated with 2 mg/ml of anti-IgM/anti-CD40 for 16 hours.

Additional advantages of the present disclosure will be set forth in part in the description which follows, and in part will be apparent from the description, or can be learned by practice of the embodiments disclosed herein. The advantages of the present disclosure will be realized and attained by means of the elements and combinations particularly pointed out in the appended claims. It is to be understood that both the foregoing general description and the following detailed description are exemplary and explanatory only and are not restrictive of the embodiments, as claimed.

### Description

Various terms relating to aspects of disclosure are used throughout the specification and claims. Such terms are to be given their ordinary meaning in the art, unless otherwise indicated. Other specifically defined terms are to be construed in a manner consistent with the definition provided herein.

Unless otherwise expressly stated, it is in no way intended that any method or aspect set forth herein be construed as requiring that its steps be performed in a specific order. Accordingly, where a method claim does not specifically state in the claims or descriptions that the steps are to be limited to a specific order, it is in no way intended that an order be inferred, in any respect. This holds for any possible non-express basis for interpretation, including matters of logic with respect to arrangement of steps or operational flow, plain meaning derived from grammatical organization or punctuation, or the number or type of aspects described in the specification.

As used herein, the singular forms "a," "an" and "the" include plural referents unless the context clearly dictates otherwise.

As used herein, the terms "subject" and "patient" are used interchangeably. A subject may include any animal, including mammals. Mammals include, without limitation, farm animals *(e.g.,* horse, cow, pig), companion animals *(e.g.,* dog, cat), laboratory animals (*e.g.,* mouse, rat, rabbits), and non-human primates. In some embodiments, the subject is a human being.

As used herein, a "nucleic acid," a "nucleic acid molecule," a "nucleic acid sequence," "polynucleotide," or "oligonucleotide" can comprise a polymeric form of nucleotides of any length, may comprise DNA and/or RNA, and can be single-stranded, double-stranded, or multiple stranded. One strand of a nucleic acid also refers to its complement.

As used herein, the phrase "corresponding to" or grammatical variations thereof when used in the context of the numbering of a given amino acid or nucleic acid sequence or position refers to the numbering of a specified reference sequence when the given amino acid or nucleic acid sequence is compared to the reference sequence (e.g., with the reference sequence herein being the nucleic acid molecule or polypeptide of (wild type or full length) SIGIRR). In other words, the residue *(e.g.,* amino acid or nucleotide) number or residue *(e.g.,* amino acid or nucleotide) position of a given polymer is designated with respect to the reference sequence rather than by the actual numerical position of the residue within the given amino acid or nucleic acid sequence. For example, a given amino acid sequence can be aligned to a reference sequence by introducing gaps to optimize residue matches between the two sequences. In these cases, although the gaps are present, the numbering of the residue in the given amino acid or nucleic acid sequence is made with respect to the reference sequence to which it has been aligned.

For example, the phrase "SIGIRR protein truncated at a position corresponding to position 215 according to SEQ ID NO:9" (and similar phrases) means that, if the amino acid sequence of the SIGIRR protein is aligned to the sequence of SEQ ID NO:9, the SIGIRR protein truncates at the position that corresponds to position 215 of SEQ ID NO:9 (e.g., the terminal amino acid of the SIGIRR protein is the amino acid at position 215). Or, in other words, these phrases refer to a SIGIRR protein which has a truncation at a position that is homologous to position 215 of SEQ ID NO:9. Herein, such a protein is also referred to as "a truncated SIGIRR protein" or "a variant SIGIRR protein" or "p.K186fs*31 variant."

A SIGIRR protein truncated at a position corresponding to position 215 according to SEQ ID NO:9 can easily be identified by performing a sequence alignment between the given SIGIRR protein and the amino acid sequence of SEQ ID NO:9. Likewise, a SIGIRR protein having a serine at a position corresponding to position 186 according to SEQ ID NO:9 can easily be identified by performing a sequence alignment between the given SIGIRR protein and the amino acid sequence of SEQ ID NO:9. A variety of computational algorithms exist that can be used for performing a sequence alignment in order to identify a truncation at a position that corresponds to position 215 in SEQ ID NO:9, or to identify a serine at a position that corresponds to position 186 according to SEQ ID NO:9. For example, by using the NCBI BLAST algorithm (Altschul et al., 1997, Nucleic Acids Res., 25, 3389-3402) or CLUSTALW software (Sievers et al., 2014, Methods Mol. Biol., 1079, 105-116) sequence alignments may be performed. However, sequences can also be aligned manually.

It has been observed in accordance with the disclosure that certain variations in SIGIRR associate with a risk of developing early-onset inflammatory bowel disease. In general, the function of this protein is not completely understood, particularly in children under the age of 18. It is believed that no variants of the SIGIRR gene or protein have any known association with early-onset inflammatory bowel disease in human beings. It is further believed that no variants of the *SIGIRR* gene or protein have any known association with early-onset inflammatory bowel disease, specifically in children. A rare variant in the SIGIRR gene segregating with the phenotype of early-onset inflammatory bowel disease in affected family members has been identified in accordance with the present disclosure. For example, a genetic alteration that results in a deletion of an adenine at position 557 of the human *SIGIRR* mRNA or cDNA (e.g., wild type SEQ ID NO:3 and SEQ ID NO:5, respectively), which results in a frameshift producing a SIGIRR protein that is truncated at a position corresponding to position 215 according to SEQ ID NO:9 (e.g., the terminal amino acid is located at position 215), has been observed to indicate that the human having such an alteration may develop early-onset inflammatory bowel disease. Altogether, the genetic analyses described herein suggest that the *SIGIRR* gene and, in particular, truncating or loss of function variants in the *SIGIRR* gene, associate with increased susceptibility to develop early-onset inflammatory bowel disease. Therefore, human subjects having SIGIRR alterations that associate with early-onset inflammatory bowel disease may be treated such that early-onset inflammatory bowel disease is inhibited, the symptoms thereof are reduced, and/or development of symptoms is repressed. Accordingly, the present disclosure provides isolated or recombinant SIGIRR variant genes, including cDNA and mRNA, as well as isolated or recombinant SIGIRR variant polypeptides. Additionally, the disclosure provides methods for leveraging the identification of such variants in subjects to identify or stratify risk in such subjects of developing inflammatory bowel disease or early-onset inflammatory bowel disease, or to diagnose subjects as having inflammatory bowel disease or early-onset inflammatory bowel disease, such that subjects at risk or subjects with active disease may be treated.

The amino acid sequences for two wild type SIGIRR proteins are set forth in SEQ ID NO:7 and SEQ ID NO:8. The wild type SIGIRR protein having SEQ ID NO:7 is 410 amino acids in length, whereas the wild type SIGIRR protein having SEQ ID NO:8 is 504 amino acids in length. Referring to both SEQ ID NO:7 and SEQ ID NO:8, positions 186 to 215 of the wild type proteins comprise the following amino acids in the recited order: Lys-Pro-Gln-Leu-Glu-Arg-Arg-Arg-Gly-Tyr-Lys-Leu-Phe-Leu-Asp-Asp-Arg-Asp-Leu-Leu-Pro-Arg-Ala-Glu-Pro-Ser-Ala-Asp-Leu-Leu (SEQ ID NO:10).

The present disclosure provides nucleic acid molecules encoding SIGIRR variant proteins that associate with inflammatory bowel disease or early-onset inflammatory bowel disease. In some embodiments, the nucleic acid molecules encode a truncated SIGIRR variant protein. For example, the present disclosure provides isolated nucleic acid molecules comprising a nucleic acid sequence encoding a human SIGIRR protein, wherein the protein is truncated at a position corresponding to position 215 according to SEQ ID NO:9, or the complement of the nucleic acid sequence.

In some embodiments, the isolated nucleic acid molecule comprises or consists of a nucleic acid sequence that encodes a truncated SIGIRR protein comprising a serine at a position corresponding to position 186 according to SEQ ID NO:9.

In some embodiments, the isolated nucleic acid molecule comprises or consists of a nucleic acid sequence that encodes a truncated SIGIRR protein comprising the amino acid sequence of SEQ ID NO:11 at the positions corresponding to positions 186 to 215 according to SEQ ID NO:9.

In some embodiments, the nucleic acid molecule comprises or consists of a nucleic acid sequence that encodes a human SIGIRR protein having an amino acid sequence that has at least about 90%, at least about 91%, at least about 92%, at least about 93%, at least about 94%, at least about 95%, at least about 96%, at least about 97%, at least about 98%, or at least about 99% sequence identity to SEQ ID NO:9, or the complement of the nucleic acid sequence. Herein, if reference is made to percent sequence identity, the higher percentages of sequence identity are preferred over the lower ones.

In some embodiments, the isolated nucleic acid molecule comprises or consists of a nucleic acid sequence that encodes a truncated SIGIRR protein, wherein the truncated SIGIRR protein comprises the amino acid sequence of SEQ ID NO:9, or an amino acid sequence that has at least 90% sequence identity to SEQ ID NO:9 and comprises a serine at a position corresponding to position 186 according to SEQ ID NO:9.

The nucleic acid sequence of a wild type SIGIRR genomic DNA is set forth in SEQ ID NO:1. The wild type SIGIRR genomic DNA comprising SEQ ID NO:1 is 11,739 nucleotides in length. Referring to SEQ ID NO:1, position 9962 of the wild type SIGIRR genomic DNA is an adenine.

The present disclosure provides genomic DNA molecules encoding a variant SIGIRR protein. In some embodiments, the genomic DNA molecules encode a truncated SIGIRR protein. In some embodiments, the genomic DNA comprises or consists of a nucleic acid sequence encoding a SIGIRR protein truncated at a position corresponding to position 215 according to SEQ ID NO:9. In some embodiments, the genomic DNA comprises or consists of a nucleic acid sequence encoding a SIGIRR protein truncated at a position corresponding to position 215 according to SEQ ID NO:9, and that comprises a serine at a position corresponding to position 186 according to SEQ ID NO:9. In some embodiments, the genomic DNA comprises or consists of a nucleic acid sequence encoding a SIGIRR protein truncated at a position corresponding to position 215 according to SEQ ID NO:9, and that comprises the following amino acid sequence at positions corresponding to positions 186 to 215 according to SEQ ID NO:9: Ser-Arg-Ser-Trp-Ser-Gly-Val-Gly-Ala-Thr-Ser-Ser-Ser-Trp-Thr-Thr-Ala-Thr-Ser-Cys-Arg-Ala-Leu-Ser-Pro-Pro-Pro-Thr-Ser-Trp (SEQ ID NO:11). In some embodiments, the genomic DNA comprises or consists of a nucleic acid sequence that encodes a variant SIGIRR protein having at least about 90%, at least about 91%, at least about 92%, at least about 93%, at least about 94%, at least about 95%, at least about 96%, at least about 97%, at least about 98%, or at least about 99% sequence identity to SEQ ID NO:9. In some embodiments, the genomic DNA comprises or consists of a nucleic acid sequence encoding a variant SIGIRR protein having SEQ ID NO:9. In some embodiments, the genomic DNA comprises or consists of a nucleic acid sequence that encodes a truncated SIGIRR protein, wherein the truncated SIGIRR protein comprises the amino acid sequence of SEQ ID NO:9, or an amino acid sequence that has at least 90% sequence identity to SEQ ID NO:9 and comprises a serine at a position corresponding to position 186 according to SEQ ID NO:9.

In some embodiments, the genomic DNA comprises or consists of a nucleic acid sequence comprising a guanine at a position corresponding to position 9962 according to SEQ ID NO:2. In contrast, the wild type SIGIRR genomic DNA comprises an adenine at a position corresponding to position 9962 according to SEQ ID NO:1. The alteration in the variant SIGIRR genomic DNA is due to the deletion of this adenine, which produces a one nucleotide base frameshift, thereby resulting in the guanine at a position corresponding to position 9962 of SEQ ID NO:2. In some embodiments, the genomic DNA comprises or consists of a nucleic acid sequence that has at least about 90%, at least about 91%, at least about 92%, at least about 93%, at least about 94%, at least about 95%, at least about 96%, at least about 97%, at least about 98%, or at least about 99% sequence identity to SEQ ID NO:2. In some embodiments, the genomic DNA comprises or consists of a nucleic acid sequence according to SEQ ID NO:2.

In some embodiments, the isolated nucleic acid molecules comprise less than the entire genomic DNA sequence. In some embodiments, the isolated nucleic acid molecules comprise or consist of at least about 15, at least about 20, at least about 25, at least about 30, at least about 35, at least about 40, at least about 45, at least about 50, at least about 60, at least about 70, at least about 80, at least about 90, at least about 100, at least about 200, at least about 300, at least about 400, at least about 500, at least about 600, at least about 700, at least about 800, at least about 900, at least about 1000, at least about 2000, at least about 3000, at least about 4000, at least about 5000, at least about 6000, at least about 7000, at least about 8000, at least about 9000, at least about 10000, at least about 11000, or at least about 11500 contiguous nucleotides of SEQ ID NO:2. In some embodiments, the isolated nucleic acid molecules comprise or consist of at least about 1000 to at least about 2000 contiguous nucleotides of SEQ ID NO:2.

In some embodiments, the isolated nucleic acid molecules comprise or consist of at least about 15, at least about 20, at least about 25, at least about 30, at least about 35, at least about 40, at least about 45, at least about 50, at least about 60, at least about 70, at least about 80, at least about 90, at least about 100, at least about 200, at least about 300, at least about 400, at least about 500, at least about 600, at least about 700, at least about 800, at least about 900, at least about 1000, at least about 1000, at least about 1100, at least about 1200, at least about 1300, at least about 1400, at least about 1500, at least about 1600, at least about 1700, at least about 1800, at least about 1900, at least about 2000, at least about 2100, at least about 2200, at least about 2300, at least about 2400, or at least about 2500 contiguous nucleotides of SEQ ID NO:2. In some embodiments, such contiguous nucleotides may be combined with other nucleic acid molecules of contiguous nucleotides to produce the cDNA molecules described herein.

Such isolated nucleic acid molecules can be used, for example, to express variant *SIGIRR* mRNAs and proteins or as exogenous donor sequences. It is understood that gene sequences within a population can vary due to polymorphisms, such as SNPs. The examples provided herein are only exemplary sequences, and other sequences are also possible.

In some embodiments, the isolated nucleic acid molecules comprise a variant *SIGIRR* minigene, in which one or more nonessential segments encoding SEQ ID NO:9 have been deleted with respect to a corresponding wild type SIGIRR genomic DNA. In some embodiments, the deleted nonessential segment(s) comprise one or more intron sequences. In some embodiments, the SIGIRR minigene has at least about 70%, at least about 75%, at least about 80%, at least about 85%, at least about 90%, at least about 95%, at least about 96%, at least about 97%, at least about 98%, at least about 99%, or 100% sequence identity to a portion of SEQ ID NO:2, wherein the minigene comprises a nucleic acid sequence having a guanine at a position corresponding to position 9962 according to SEQ ID NO:2.

The nucleic acid sequence of a wild type *SIGIRR* mRNA is set forth in SEQ ID NO:3. The wild type SIGIRR mRNA comprising SEQ ID NO:3 is 1230 nucleotides in length. Referring to SEQ ID NO:3, position 557 of the wild type *SIGIRR* mRNA is an adenine.

The present disclosure also provides mRNA molecules encoding a variant SIGIRR protein. In some embodiments, the mRNA molecules encode a truncated SIGIRR protein. In some embodiments, the mRNA comprises or consists of a nucleic acid sequence encoding a SIGIRR protein truncated at a position corresponding to position 215 according to SEQ ID NO:9. In some embodiments, the mRNA comprises or consists of a nucleic acid sequence encoding a SIGIRR protein truncated at a position corresponding to position 215 according to SEQ ID NO:9, and that comprises a serine at a position corresponding to position 186 according to SEQ ID NO:9. In some embodiments, the mRNA comprises or consists of a nucleic acid sequence encoding a SIGIRR protein truncated at a position corresponding to position 215 according to SEQ ID NO:9, and that comprises the following amino acid sequence at positions corresponding to positions 186 to 215 according to SEQ ID NO:9: Ser-Arg-Ser-Trp-Ser-Gly-Val-Gly-Ala-Thr-Ser-Ser-Ser-Trp-Thr-Thr-Ala-Thr-Ser-Cys-Arg-Ala-Leu-Ser-Pro-Pro-Pro-Thr-Ser-Trp (SEQ ID NO:11). In some embodiments, the mRNA comprises or consists of a nucleic acid sequence that encodes a variant SIGIRR protein having at least about 90%, at least about 91%, at least about 92%, at least about 93%, at least about 94%, at least about 95%, at least about 96%, at least about 97%, at least about 98%, or at least about 99% sequence identity to SEQ ID NO:9. In some embodiments, the mRNA comprises or consists of a nucleic acid sequence encoding a variant SIGIRR protein having SEQ ID NO:9. In some embodiments, the mRNA comprises or consists of a nucleic acid sequence that encodes a truncated SIGIRR protein, wherein the truncated SIGIRR protein comprises the amino acid sequence of SEQ ID NO:9, or an amino acid sequence that has at least 90% sequence identity to SEQ ID NO:9 and comprises a serine at a position corresponding to position 186 according to SEQ ID NO:9.

In some embodiments, the mRNA comprises or consists of a nucleic acid sequence comprising a guanine at a position corresponding to position 557 according to SEQ ID NO:4. In contrast, the wild type *SIGIRR* mRNA comprises an adenine at a position corresponding to position 557 according to SEQ ID NO:3. The alteration in the variant SIGIRR mRNA is due to the deletion of this adenine, which produces a one nucleotide base frameshift, thereby resulting in the guanine at a position corresponding to position 557 of SEQ ID NO:4. In some embodiments, the mRNA comprises or consists of a nucleic acid sequence comprising the codons CUA and AGC at positions corresponding to positions 553 to 555 and 556 to 558, respectively, according to SEQ ID NO:4. In contrast, the wild type *SIGIRR* mRNA comprises the codons CUA and AAG at positions corresponding to positions 553 to 555 and 556 to 558, respectively, according to SEQ ID NO:3. In some embodiments, the mRNA comprises or consists of a nucleic acid sequence that has at least about 90%, at least about 91%, at least about 92%, at least about 93%, at least about 94%, at least about 95%, at least about 96%, at least about 97%, at least about 98%, or at least about 99% sequence identity to SEQ ID NO:4. In some embodiments, the mRNA comprises or consists of a nucleic acid sequence according to SEQ ID NO:4.

In some embodiments, the isolated nucleic acid molecule comprises less nucleotides than the entire SIGIRR mRNA sequence. In some embodiments, the isolated nucleic acid molecules comprise or consist of at least about 5, at least about 8, at least about 10, at least about 12, at least about 15, at least about 20, at least about 25, at least about 30, at least about 35, at least about 40, at least about 45, at least about 50, at least about 60, at least about 70, at least about 80, at least about 90, at least about 100, at least about 200, at least about 300, at least about 400, at least about 500, or at least about 600 contiguous nucleotides of SEQ ID NO:4. In some embodiments, the isolated nucleic acid molecules comprise or consist of at least about 200 to at least about 500 contiguous nucleotides of SEQ ID NO:4. In this regard, the longer mRNA molecules are preferred over the shorter ones. In some embodiments, the isolated nucleic acid molecules comprise or consist of at least about 50, at least about 60, at least about 70, at least about 80, at least about 90, at least about 100, at least about 200, at least about 300, at least about 400, or at least about 500 contiguous nucleotides of SEQ ID NO:4. In this regard, the longer mRNA molecules are preferred over the shorter ones. In some embodiments, such mRNA molecules include the codon that encodes the serine at the position that corresponds to position 186 according to SEQ ID NO:9. In some embodiments, such mRNA molecules include the guanine at the position corresponding to position 557 according to SEQ ID NO:4. In some embodiments, such mRNA molecules include the codons CUA and AGC at positions corresponding to positions 553 to 555 and 556 to 558, respectively, according to SEQ ID NO:4.

The nucleic acid sequence of a wild type *SIGIRR* cDNA is set forth in SEQ ID NO:5. The wild type *SIGIRR* cDNA comprising SEQ ID NO:5 is 1233 nucleotides in length, including the stop codon. Referring to SEQ ID NO:5, position 557 of the wild type *SIGIRR* cDNA is an adenine.

The present disclosure also provides cDNA molecules encoding a variant SIGIRR protein. In some embodiments, the cDNA molecules encode a truncated SIGIRR protein. In some embodiments, the cDNA comprises or consists of a nucleic acid sequence encoding a SIGIRR protein truncated at a position corresponding to position 215 according to SEQ ID NO:9. In some embodiments, the cDNA comprises or consists of a nucleic acid sequence encoding a SIGIRR protein truncated at a position corresponding to position 215 according to SEQ ID NO:9, and that comprises a serine at a position corresponding to position 186 according to SEQ ID NO:9. In some embodiments, the cDNA comprises or consists of a nucleic acid sequence encoding a SIGIRR protein truncated at a position corresponding to position 215 according to SEQ ID NO:9, and that comprises the following amino acid sequence at positions corresponding to positions 186 to 215 according to SEQ ID NO:9: Ser-Arg-Ser-Trp-Ser-Gly-Val-Gly-Ala-Thr-Ser-Ser-Ser-Trp-Thr-Thr-Ala-Thr-Ser-Cys-Arg-Ala-Leu-Ser-Pro-Pro-Pro-Thr-Ser-Trp (SEQ ID NO:11). In some embodiments, the cDNA comprises or consists of a nucleic acid sequence that encodes a variant SIGIRR protein having at least about 90%, at least about 91%, at least about 92%, at least about 93%, at least about 94%, at least about 95%, at least about 96%, at least about 97%, at least about 98%, or at least about 99% sequence identity to SEQ ID NO:9. In some embodiments, the cDNA comprises or consists of a nucleic acid sequence encoding a variant SIGIRR protein having SEQ ID NO:9. In some embodiments, the cDNA comprises or consists of a nucleic acid sequence that encodes a truncated SIGIRR protein, wherein the truncated SIGIRR protein comprises the amino acid sequence of SEQ ID NO:9, or an amino acid sequence that has at least 90% sequence identity to SEQ ID NO:9 and comprises a serine at a position corresponding to position 186 according to SEQ ID NO:9.

In some embodiments, the cDNA comprises or consists of a nucleic acid sequence comprising a guanine at a position corresponding to position 557 according to SEQ ID NO:6. In contrast, the wild type *SIGIRR* cDNA comprises an adenine at a position corresponding to position 557 according to SEQ ID NO:5. The alteration in the variant *SIGIRR* cDNA is due to the deletion of this adenine, which produces a one nucleotide base frameshift, thereby resulting in the guanine at a position corresponding to position 557 of SEQ ID NO:6. In some embodiments, the cDNA comprises or consists of a nucleic acid sequence comprising the codons CTA and AGC at positions corresponding to positions 553 to 555 and 556 to 558, respectively, according to SEQ ID NO:6. In contrast, the wild type *SIGIRR* cDNA comprises the codons CTA and AAG at positions corresponding to positions 553 to 555 and 556 to 558, respectively, according to SEQ ID NO:5. In some embodiments, the cDNA comprises or consists of a nucleic acid sequence that has at least about 90%, at least about 91%, at least about 92%, at least about 93%, at least about 94%, at least about 95%, at least about 96%, at least about 97%, at least about 98%, or at least about 99% sequence identity to SEQ ID NO:6. In some embodiments, the cDNA comprises or consists of a nucleic acid sequence according to SEQ ID NO:6.

In some embodiments, the cDNA molecules comprise less than the entire sequence of the variant *SIGIRR* cDNA molecule. In some embodiments, the cDNA molecules comprise or consist of at least about 5, at least about 8, at least about 10, at least about 12, at least about 15, at least about 20, at least about 25, at least about 30, at least about 35, at least about 40, at least about 45, at least about 50, at least about 60, at least about 70, at least about 80, at least about 90, at least about 100, at least about 200, at least about 300, at least about 400, at least about 500, or at least about 600 contiguous nucleotides of SEQ ID NO:6. In some embodiments, the cDNA molecule comprises or consists of at least about 200 to at least about 500 contiguous nucleotides of SEQ ID NO:6. In this regard, the longer cDNA molecules are preferred over the shorter ones. In some embodiments, the cDNA molecules comprise or consist of at least about 50, at least about 60, at least about 70, at least about 80, at least about 90, at least about 100, at least about 200, at least about 300, at least about 400, or at least about 500 contiguous nucleotides of SEQ ID NO:6. In this regard, the longer cDNA molecules are preferred over the shorter ones. In some embodiments, such cDNA molecules include the codon that encodes the serine at the position that corresponds to position 186 according to SEQ ID NO:9. In some embodiments, such cDNA molecules include the guanine at the position corresponding to position 557 according to SEQ ID NO:6. In some embodiments, such cDNA molecules include the codons CTA and AGC at positions corresponding to positions 553 to 555 and 556 to 558, respectively, according to SEQ ID NO:4.

The present disclosure also provides isolated nucleic acid molecules that hybridize to variant SIGIRR genomic DNA (such as SEQ ID NO:2), variant SIGIRR minigenes, variant SIGIRR mRNA (such as SEQ ID NO:4), and/or variant *SIGIRR* cDNA (such as SEQ ID NO:6). In some embodiments, such isolated nucleic acid molecules comprise or consist of at least about 5, at least about 8, at least about 10, at least about 11, at least about 12, at least about 13, at least about 14, at least about 15, at least about 16, at least about 17, at least about 18, at least about 19, at least about 20, at least about 21, at least about 22, at least about 23, at least about 24, at least about 25, at least about 30, at least about 35, at least about 40, at least about 45, at least about 50, at least about 55, at least about 60, at least about 65, at least about 70, at least about 75, at least about 80, at least about 85, at least about 90, at least about 95, at least about 100, at least about 200, at least about 300, at least about 400, at least about 500, at least about 600, at least about 700, at least about 800, at least about 900, at least about 1000, at least about 2000, at least about 3000, at least about 4000, at least about 5000, at least about 6000, at least about 7000, at least about 8000, at least about 9000, at least about 10000, at least about 11000, or at least about 11500. In some embodiments, the isolated nucleic acid molecule comprises or consists of at least 15 nucleotides. In some embodiments, the isolated nucleic acid molecule comprises or consists of at least 15 nucleotides to at least about 35 nucleotides. In some embodiments, such isolated nucleic acid molecules hybridize to variant SIGIRR genomic DNA (such as SEQ ID NO:2), variant SIGIRR minigenes, variant SIGIRR mRNA (such as SEQ ID NO:4), and/or variant *SIGIRR* cDNA (such as SEQ ID NO:6) under stringent conditions. Such nucleic acid molecules may be used, for example, as probes, as primers, or as alteration-specific probes or primers as described or exemplified herein.

In some embodiments, the isolated nucleic acid molecules hybridize to at least about 15 contiguous nucleotides of a nucleic acid molecule that is at least about 70%, at least about 75%, at least about 80%, at least about 85%, at least about 90%, at least about 95%, at least about 96%, at least about 97%, at least about 98%, at least about 99%, or 100% identical to variant SIGIRR genomic DNA (such as SEQ ID NO:2), variant SIGIRR minigenes, variant SIGIRR mRNA (such as SEQ ID NO:4), and/or variant *SIGIRR* cDNA (such as SEQ ID NO:6). In some embodiments, the isolated nucleic acid molecules comprise or consist of from about 15 to about 100 nucleotides, or from about 15 to about 35 nucleotides. In some embodiments, the isolated nucleic acid molecules comprise or consist of from about 15 to about 100 nucleotides. In some embodiments, the isolated nucleic acid molecules comprise or consist of from about 15 to about 35 nucleotides.

In some embodiments, any of the nucleic acid molecules, genomic DNA molecules, cDNA molecules, or mRNA molecules disclosed herein can be purified, e.g., are at least about 90% pure. In some embodiments, any of the nucleic acid molecules, genomic DNA molecules, cDNA molecules, or mRNA molecules disclosed herein can be purified, e.g., are at least about 95% pure. In some embodiments, any of the nucleic acid molecules, genomic DNA molecules, cDNA molecules, or mRNA molecules disclosed herein can be purified, e.g., are at least about 99% pure. Purification is according to the hands of a human being, with human-made purification techniques.

The present disclosure also provides fragments of any of the isolated nucleic acid molecules, genomic DNA molecules, cDNA molecules, or mRNA molecules disclosed herein. In some embodiments, the fragments comprise or consist of at least about 5, at least about 8, at least about 10, at least about 11, at least about 12, at least about 13, at least about 14, at least about 15, at least about 16, at least about 17, at least about 18, at least about 19, at least about 20, at least about 21, at least about 22, at least about 23, at least about 24, at least about 25, at least about 30, at least about 35, at least about 40, at least about 45, at least about 50, at least about 55, at least about 60, at least about 65, at least about 70, at least about 75, at least about 80, at least about 85, at least about 90, at least about 95, or at least about 100 contiguous residues of any of the nucleic acid sequences disclosed herein, or any complement thereof. In this regard, the longer fragments are preferred over the shorter ones. In some embodiments, the fragments comprise or consist of at least about 5, at least about 8, at least about 10, at least about 11, at least about 12, at least about 13, at least about 14, at least about 15, at least about 16, at least about 17, at least about 18, at least about 19, at least about 20, at least about 21, at least about 22, at least about 23, at least about 24, at least about 25, at least about 30, at least about 35, at least about 40, at least about 45, or at least about 50 contiguous residues. In this regard, the longer fragments are preferred over the shorter ones. In some embodiments, the fragments comprise or consist of at least about 20, at least about 25, at least about 30, or at least about 35 contiguous residues. In some embodiments, the fragments comprise or consist of at least about 20 contiguous residues. In some embodiments, the fragments comprise or consist of at least about 25 contiguous residues. In some embodiments, the fragments comprise or consist of at least about 30 contiguous residues. In some embodiments, the fragments comprise or consist of at least about 35 contiguous residues. It is envisaged that the fragments comprise of consist of the portion of the nucleic acid molecule that encodes a serine at a position corresponding to position 186 according to SEQ ID NO:9, or encodes positions corresponding to positions 186 to 215 according to SEQ ID NO:9. Such fragments may be used, for example, as probes, as primers, or as allele-specific primers as described or exemplified herein.

The present disclosure also provides probes and primers. The probe or primer of the present disclosure have a nucleic acid sequence that specifically hybridizes to any of the nucleic acid molecules disclosed herein, or the complement thereof. In some embodiments, the probe or primer specifically hybridizes to any of the nucleic acid molecules disclosed herein under stringent conditions. The present disclosure also provides nucleic acid molecules having nucleic acid sequences that hybridize under moderate conditions to any of the nucleic acid molecules disclosed herein, or the complement thereof. A probe or primer according to the disclosure preferably encompasses the nucleic acid codon which encodes the serine at a position corresponding to position 186 according to SEQ ID NO:9, or the complement thereof. Thus, in a preferred embodiment, the disclosure provides alteration-specific primers which are defined herein above and below in more detail.

A probe according to the present disclosure may be used to detect the variant SIGIRR nucleic acid molecule (e.g., genomic DNA, mRNA, and/or cDNA) encoding the variant SIGIRR protein (e.g., according to SEQ ID NO:9). In addition, a primer according to the present disclosure may be used to amplify a nucleic acid molecule encoding a variant SIGIRR protein, or fragment thereof. The disclosure also provides a pair of primers comprising one of the primers described above. For genomic polymerase chain reaction (PCR) amplification of the SIGIRR fragment containing the frameshift variant leading to truncation, suitable primer sequences include, but are not limited to: forward primer (5' to 3'): TCAGTGGCTCTGAACTGCAC (SEQ ID NO:12) and reverse primer (5' to 3'): GGTCCTGTTGAGCAGAGGAG (SEQ ID NO:13).

The nucleic acid molecules disclosed herein can comprise a nucleic acid sequence of a naturally occurring SIGIRR genomic DNA, cDNA, or mRNA transcript, or can comprise a non-naturally occurring sequence. In some embodiments, the naturally occurring sequence can differ from the non-naturally occurring sequence due to synonymous mutations or mutations that do not affect the encoded SIGIRR polypeptide. For example, the sequence can be identical with the exception of synonymous mutations or mutations that do not affect the encoded SIGIRR polypeptide. A synonymous mutation or substitution is the substitution of one nucleotide for another in an exon of a gene coding for a protein such that the produced amino acid sequence is not modified. This is possible because of the degeneracy of the genetic code, with some amino acids being coded for by more than one three-base pair codon. Synonymous substitutions are used, for example, in the process of codon optimization. The nucleic acid molecules disclosed herein can be codon optimized.

Also provided herein are functional polynucleotides that can interact with the disclosed nucleic acid molecules. Functional polynucleotides are nucleic acid molecules that have a specific function, such as binding a target molecule or catalyzing a specific reaction. Examples of functional polynucleotides include, but are not limited to, antisense molecules, aptamers, ribozymes, triplex forming molecules, and external guide sequences. The functional polynucleotides can act as effectors, inhibitors, modulators, and stimulators of a specific activity possessed by a target molecule, or the functional polynucleotides can possess a *de novo* activity independent of any other molecules.

Antisense molecules are designed to interact with a target nucleic acid molecule through either canonical or non-canonical base pairing. The interaction of the antisense molecule and the target molecule is designed to promote the destruction of the target molecule through, for example, RNase-H-mediated RNA-DNA hybrid degradation. Alternately, the antisense molecule is designed to interrupt a processing function that normally would take place on the target molecule, such as transcription or replication. Antisense molecules can be designed based on the sequence of the target molecule. Numerous methods for optimization of antisense efficiency by identifying the most accessible regions of the target molecule exist. Exemplary methods include, but are not limited to, *in vitro* selection experiments and DNA modification studies using DMS and DEPC. Antisense molecules generally bind the target molecule with a dissociation constant (k_{d}) less than or equal to about 10⁻⁶, less than or equal to about 10⁻⁸, less than or equal to about 10⁻¹⁰, or less than or equal to about 10⁻¹². A representative sample of methods and techniques which aid in the design and use of antisense molecules can be found in the following non-limiting list of U.S. Patents: 5,135,917; 5,294,533; 5,627,158; 5,641,754; 5,691,317; 5,780,607; 5,786,138; 5,849,903; 5,856,103; 5,919,772; 5,955,590; 5,990,088; 5,994,320; 5,998,602; 6,005,095; 6,007,995; 6,013,522; 6,017,898; 6,018,042; 6,025,198; 6,033,910; 6,040,296; 6,046,004; 6,046,319; and 6,057,437. Examples of antisense molecules include, but are not limited to, antisense RNAs, small interfering RNAs (siRNAs), and short hairpin RNAs (shRNAs).

The isolated nucleic acid molecules disclosed herein can comprise RNA, DNA, or both RNA and DNA. The isolated nucleic acid molecules can also be linked or fused to a heterologous nucleic acid sequence, such as in a vector, or a heterologous label. For example, the isolated nucleic acid molecules disclosed herein can be in a vector or exogenous donor sequence comprising the isolated nucleic acid molecule and a heterologous nucleic acid sequence. The isolated nucleic acid molecules can also be linked or fused to a heterologous label, such as a fluorescent label. Other examples of labels are disclosed elsewhere herein.

The label can be directly detectable *(e.g.,* fluorophore) or indirectly detectable (*e.g.,* hapten, enzyme, or fluorophore quencher). Such labels can be detectable by spectroscopic, photochemical, biochemical, immunochemical, or chemical means. Such labels include, for example, radiolabels that can be measured with radiation-counting devices; pigments, dyes or other chromogens that can be visually observed or measured with a spectrophotometer; spin labels that can be measured with a spin label analyzer; and fluorescent labels (e.g., fluorophores), where the output signal is generated by the excitation of a suitable molecular adduct and that can be visualized by excitation with light that is absorbed by the dye or can be measured with standard fluorometers or imaging systems. The label can also be, for example, a chemiluminescent substance, where the output signal is generated by chemical modification of the signal compound; a metal-containing substance; or an enzyme, where there occurs an enzyme-dependent secondary generation of signal, such as the formation of a colored product from a colorless substrate. The term "label" can also refer to a "tag" or hapten that can bind selectively to a conjugated molecule such that the conjugated molecule, when added subsequently along with a substrate, is used to generate a detectable signal. For example, one can use biotin as a tag and then use an avidin or streptavidin conjugate of horseradish peroxidate (HRP) to bind to the tag, and then use a calorimetric substrate (e.g., tetramethylbenzidine (TMB)) or a fluorogenic substrate to detect the presence of HRP. Exemplary labels that can be used as tags to facilitate purification include, but are not limited to, myc, HA, FLAG or 3XFLAG, 6XHis or polyhistidine, glutathione-S-transferase (GST), maltose binding protein, an epitope tag, or the Fc portion of immunoglobulin. Numerous labels are known and include, for example, particles, fluorophores, haptens, enzymes and their calorimetric, fluorogenic and chemiluminescent substrates and other labels.

The disclosed nucleic acid molecules can comprise, for example, nucleotides or non-natural or modified nucleotides, such as nucleotide analogs or nucleotide substitutes. Such nucleotides include a nucleotide that contains a modified base, sugar, or phosphate group, or that incorporates a non-natural moiety in its structure. Examples of non-natural nucleotides include, but are not limited to, dideoxynucleotides, biotinylated, aminated, deaminated, alkylated, benzylated, and fluorophor-labeled nucleotides.

The nucleic acid molecules disclosed herein can also comprise one or more nucleotide analogs or substitutions. A nucleotide analog is a nucleotide which contains a modification to either the base, sugar, or phosphate moieties. Modifications to the base moiety include, but are not limited to, natural and synthetic modifications of A, C, G, and T/U, as well as different purine or pyrimidine bases such as, for example, pseudouridine, uracil-5-yl, hypoxanthin-9-yl (I), and 2-aminoadenin-9-yl. Modified bases include, but are not limited to, 5-methylcytosine (5-me-C), 5-hydroxymethyl cytosine, xanthine, hypoxanthine, 2-aminoadenine, 6-methyl and other alkyl derivatives of adenine and guanine, 2-propyl and other alkyl derivatives of adenine and guanine, 2-thiouracil, 2-thiothymine and 2-thiocytosine, 5-halouracil and cytosine, 5-propynyl uracil and cytosine, 6-azo uracil, cytosine and thymine, 5-uracil (pseudouracil), 4-thiouracil, 8-halo, 8-amino, 8-thiol, 8-thioalkyl, 8-hydroxyl and other 8-substituted adenines and guanines, 5-halo particularly 5-bromo, 5-trifluoromethyl and other 5-substituted uracils and cytosines, 7-methylguanine and 7-methyladenine, 8-azaguanine and 8-azaadenine, 7-deazaguanine and 7-deazaadenine and 3-deazaguanine and 3-deazaadenine. Certain nucleotide analogs such as, for example, 5-substituted pyrimidines, 6-azapyrimidines, and N-2, N-6 and O-6 substituted purines including, but not limited to, 2-aminopropyladenine, 5-propynyluracil, 5-propynylcytosine, and 5-methylcytosine can increase the stability of duplex formation. Often, base modifications can be combined with, for example, a sugar modification, such as 2'-O-methoxyethyl, to achieve unique properties such as increased duplex stability.

Nucleotide analogs can also include modifications of the sugar moiety. Modifications to the sugar moiety include, but are not limited to, natural modifications of the ribose and deoxy ribose as well as synthetic modifications. Sugar modifications include, but are not limited to, the following modifications at the 2' position: OH; F; O-, S-, or N-alkyl; O-, S-, or N-alkenyl; O-, S- or N-alkynyl; or O-alkyl-O-alkyl, wherein the alkyl, alkenyl, and alkynyl may be substituted or unsubstituted C₁₋₁₀alkyl or C₂₋₁₀alkenyl, and C₂₋₁₀alkynyl. Exemplary 2' sugar modifications also include, but are not limited to, -O[(CH₂)ₙO]ₘCH₃, -O(CH₂)ₙOCH₃, -O(CH₂)ₙNH₂, -O(CH₂)ₙCH₃, -O(CH₂)ₙ-ONH₂, and -O(CH₂)ₙON[(CH₂)ₙCH₃)]₂, where n and m are from 1 to about 10.

Other modifications at the 2' position include, but are not limited to, C₁₋₁₀alkyl, substituted lower alkyl, alkaryl, aralkyl, O-alkaryl or O-aralkyl, SH, SCH₃, OCN, Cl, Br, CN, CF₃, OCF₃, SOCH₃, SO₂CH₃, ONO₂, NO₂, N₃, NH₂, heterocycloalkyl, heterocycloalkaryl, aminoalkylamino, polyalkylamino, substituted silyl, an RNA cleaving group, a reporter group, an intercalator, a group for improving the pharmacokinetic properties of an oligonucleotide, or a group for improving the pharmacodynamic properties of an oligonucleotide, and other substituents having similar properties. Similar modifications may also be made at other positions on the sugar, particularly the 3' position of the sugar on the 3' terminal nucleotide or in 2'-5' linked oligonucleotides and the 5' position of 5' terminal nucleotide. Modified sugars can also include those that contain modifications at the bridging ring oxygen, such as CH₂ and S. Nucleotide sugar analogs can also have sugar mimetics, such as cyclobutyl moieties in place of the pentofuranosyl sugar.

Nucleotide analogs can also be modified at the phosphate moiety. Modified phosphate moieties include, but are not limited to, those that can be modified so that the linkage between two nucleotides contains a phosphorothioate, chiral phosphorothioate, phosphorodithioate, phosphotriester, aminoalkylphosphotriester, methyl and other alkyl phosphonates including 3'-alkylene phosphonate and chiral phosphonates, phosphinates, phosphoramidates including 3'-amino phosphoramidate and aminoalkylphosphoramidates, thionophosphoramidates, thionoalkylphosphonates, thionoalkylphosphotriesters, and boranophosphates. These phosphate or modified phosphate linkage between two nucleotides can be through a 3'-5' linkage or a 2'-5' linkage, and the linkage can contain inverted polarity such as 3'-5' to 5'-3' or 2'-5' to 5'-2'. Various salts, mixed salts, and free acid forms are also included.

Nucleotide substitutes include molecules having similar functional properties to nucleotides, but which do not contain a phosphate moiety, such as peptide nucleic acid (PNA). Nucleotide substitutes include molecules that will recognize nucleic acids in a Watson-Crick or Hoogsteen manner, but which are linked together through a moiety other than a phosphate moiety. Nucleotide substitutes are able to conform to a double helix type structure when interacting with the appropriate target nucleic acid.

Nucleotide substitutes also include nucleotides or nucleotide analogs that have had the phosphate moiety or sugar moieties replaced. In some embodiments, nucleotide substitutes may not contain a standard phosphorus atom. Substitutes for the phosphate can be, for example, short chain alkyl or cycloalkyl internucleoside linkages, mixed heteroatom and alkyl or cycloalkyl internucleoside linkages, or one or more short chain heteroatomic or heterocyclic internucleoside linkages. These include those having morpholino linkages (formed in part from the sugar portion of a nucleoside); siloxane backbones; sulfide, sulfoxide and sulfone backbones; formacetyl and thioformacetyl backbones; methylene formacetyl and thioformacetyl backbones; alkene containing backbones; sulfamate backbones; methyleneimino and methylenehydrazino backbones; sulfonate and sulfonamide backbones; amide backbones; and others having mixed N, O, S, and CH₂ component parts.

It is also understood in a nucleotide substitute that both the sugar and the phosphate moieties of the nucleotide can be replaced by, for example, an amide type linkage (aminoethylglycine) (PNA).

It is also possible to link other types of molecules (conjugates) to nucleotides or nucleotide analogs to enhance, for example, cellular uptake. Conjugates can be chemically linked to the nucleotide or nucleotide analogs. Such conjugates include, for example, lipid moieties such as a cholesterol moiety, cholic acid, a thioether such as hexyl-S-tritylthiol, a thiocholesterol, an aliphatic chain such as dodecandiol or undecyl residues, a phospholipid such as di-hexadecyl-rac-glycerol or triethylammonium 1,2-di-O-hexadecyl-rac-glycero-3-H-phosphonate, a polyamine or a polyethylene glycol chain, adamantane acetic acid, a palmityl moiety, or an octadecylamine or hexylamino-carbonyl-oxycholesterol moiety.

The present disclosure also provides vectors comprising any one or more of the nucleic acid molecules disclosed herein. In some embodiments, the vectors comprise any one or more of the nucleic acid molecules disclosed herein and a heterologous nucleic acid. The vectors can be viral or nonviral vectors capable of transporting a nucleic acid molecule. In some embodiments, the vector is a plasmid or cosmid (e.g., a circular double-stranded DNA into which additional DNA segments can be ligated). In some embodiments, the vector is a viral vector, wherein additional DNA segments can be ligated into the viral genome. In some embodiments, the vector can autonomously replicate in a host cell into which it is introduced (e.g., bacterial vectors having a bacterial origin of replication and episomal mammalian vectors). In some embodiments, the vector (e.g., non-episomal mammalian vectors) can be integrated into the genome of a host cell upon introduction into the host cell and thereby are replicated along with the host genome. Moreover, particular vectors can direct the expression of genes to which they are operatively linked. Such vectors are referred to herein as "recombinant expression vectors" or "expression vectors." Such vectors can also be targeting vectors (i.e., exogenous donor sequences).

In some embodiments, the proteins encoded by the various genetic variants disclosed herein are expressed by inserting nucleic acid molecules encoding the disclosed genetic variants into expression vectors, such that the genes are operatively linked to expression control sequences, such as transcriptional and translational control sequences. Expression vectors include, but are not limited to, plasmids, cosmids, retroviruses, adenoviruses, adeno-associated viruses (AAV), plant viruses such as cauliflower mosaic virus and tobacco mosaic virus, yeast artificial chromosomes (YACs), Epstein-Barr (EBV)-derived episomes, and other expression vectors known in the art. In some embodiments, nucleic acid molecules comprising the disclosed genetic variants can be ligated into a vector such that transcriptional and translational control sequences within the vector serve their intended function of regulating the transcription and translation of the genetic variant. The expression vector and expression control sequences are chosen to be compatible with the expression host cell used. Nucleic acid sequences comprising the disclosed genetic variants can be inserted into separate vectors or into the same expression vector as the variant genetic information. A nucleic acid sequence comprising the disclosed genetic variants can be inserted into the expression vector by standard methods (e.g., ligation of complementary restriction sites on the nucleic acid comprising the disclosed genetic variants and vector, or blunt end ligation if no restriction sites are present).

In addition to a nucleic acid sequence comprising the disclosed genetic variants, the recombinant expression vectors can carry regulatory sequences that control the expression of the genetic variant in a host cell. The design of the expression vector, including the selection of regulatory sequences can depend on such factors as the choice of the host cell to be transformed, the level of expression of protein desired, and so forth. Desired regulatory sequences for mammalian host cell expression can include, for example, viral elements that direct high levels of protein expression in mammalian cells, such as promoters and/or enhancers derived from retroviral LTRs, cytomegalovirus (CMV) (such as the CMV promoter/enhancer), Simian Virus 40 (SV40) (such as the SV40 promoter/enhancer), adenovirus, (e.g., the adenovirus major late promoter (AdMLP)), polyoma and strong mammalian promoters such as native immunoglobulin and actin promoters. Methods of expressing polypeptides in bacterial cells or fungal cells (e.g., yeast cells) are also well known.

A promoter can be, for example, a constitutively active promoter, a conditional promoter, an inducible promoter, a temporally restricted promoter (e.g., a developmentally regulated promoter), or a spatially restricted promoter (e.g., a cell-specific or tissue-specific promoter). Examples of promoters can be found, for example, in WO 2013/176772.

Examples of inducible promoters include, for example, chemically regulated promoters and physically-regulated promoters. Chemically regulated promoters include, for example, alcohol-regulated promoters (e.g., an alcohol dehydrogenase (alcA) gene promoter), tetracycline-regulated promoters (e.g., a tetracycline-responsive promoter, a tetracycline operator sequence (tetO), a tet-On promoter, or a tet-Off promoter), steroid regulated promoters (e.g., a rat glucocorticoid receptor, a promoter of an estrogen receptor, or a promoter of an ecdysone receptor), or metal-regulated promoters (e.g., a metalloprotein promoter). Physically regulated promoters include, for example temperature-regulated promoters *(e.g.,* a heat shock promoter) and light-regulated promoters *(e.g.,* a light-inducible promoter or a light-repressible promoter).

Tissue-specific promoters can be, for example, neuron-specific promoters, glia-specific promoters, muscle cell-specific promoters, heart cell-specific promoters, kidney cell-specific promoters, bone cell-specific promoters, endothelial cell-specific promoters, or immune cell-specific promoters (e.g., a B cell promoter or a T cell promoter).

Developmentally regulated promoters include, for example, promoters active only during an embryonic stage of development, or only in an adult cell.

In addition to a nucleic acid sequence comprising the disclosed genetic variants and regulatory sequences, the recombinant expression vectors can carry additional sequences, such as sequences that regulate replication of the vector in host cells (e.g., origins of replication) and selectable marker genes. A selectable marker gene can facilitate selection of host cells into which the vector has been introduced *(see e.g.,* U.S. Patents 4,399,216; 4,634,665; and 5,179,017). For example, a selectable marker gene can confer resistance to drugs, such as G418, hygromycin, or methotrexate, on a host cell into which the vector has been introduced. Exemplary selectable marker genes include, but are not limited to, the dihydrofolate reductase (DHFR) gene (for use in dhfr-host cells with methotrexate selection/amplification), the neo gene (for G418 selection), and the glutamate synthetase (GS) gene.

Additional vectors are described in, for example, U.S. Provisional Application No. 62/367,973, filed on July 28, 2016.

The present disclosure also provides compositions comprising any one or more of the isolated nucleic acid molecules, genomic DNA molecules, cDNA molecules, or mRNA molecules disclosed herein. In some embodiments, the composition is a pharmaceutical composition.

The present disclosure also provides variant SIGIRR polypeptides. In some embodiments, the variant SIGIRR polypeptide is truncated. In some embodiments, the variant SIGIRR polypeptide is truncated at a position corresponding to position 215 according to SEQ ID NO:9. In some embodiments, the variant SIGIRR polypeptide is truncated at a position corresponding to position 215 according to SEQ ID NO:9, and comprises a serine at a position corresponding to position 186 according to SEQ ID NO:9. In some embodiments, the variant SIGIRR polypeptide is truncated at a position corresponding to position 215 according to SEQ ID NO:9, and comprises a plurality of the amino acids at positions corresponding to positions 186 to 215 according to SEQ ID NO:9. In some embodiments, the variant SIGIRR polypeptide is truncated at a position corresponding to position 215 according to SEQ ID NO:9, and comprises the following amino acid sequence at positions corresponding to positions 186 to 215 according to SEQ ID NO:9: Ser-Arg-Ser-Trp-Ser-Gly-Val-Gly-Ala-Thr-Ser-Ser-Ser-Trp-Thr-Thr-Ala-Thr-Ser-Cys-Arg-Ala-Leu-Ser-Pro-Pro-Pro-Thr-Ser-Trp (SEQ ID NO:11). In some embodiments, the variant SIGIRR polypeptide has at least about 90%, at least about 91%, at least about 92%, at least about 93%, at least about 94%, at least about 95%, at least about 96%, at least about 97%, at least about 98%, or at least about 99% sequence identity to the amino acid sequence according to SEQ ID NO:9. In some embodiments, the variant SIGIRR polypeptide comprises or consists of the amino acid sequence according to SEQ ID NO:9. In some embodiments, the truncated SIGIRR protein comprises or consists of the amino acid sequence according to SEQ ID NO:9, or an amino acid sequence that has at least 90% sequence identity to SEQ ID NO:9 and comprises a serine at a position corresponding to position 186 according to SEQ ID NO:9.

The present disclosure also provides fragments of any of the polypeptides disclosed herein. In some embodiments, the fragments comprise at least about 10, at least about 15, at least about 20, at least about 25, at least about 30, at least about 35, at least about 40, at least about 45, at least about 50, at least about 55, at least about 60, at least about 65, at least about 70, at least about 75, at least about 80, at least about 85, at least about 90, at least about 95, at least about 100, at least about 150, or at least about 200 contiguous amino acid residues of the encoded polypeptide (such as the polypeptide having the amino acid sequence of SEQ ID NO:9). In this regard, the longer fragments are preferred over the shorter ones. In some embodiments, the fragments comprise at least about 10, at least about 15, at least about 20, at least about 25, at least about 30, at least about 35, at least about 40, at least about 45, at least about 50, at least about 55, at least about 60, at least about 65, at least about 70, at least about 75, at least about 80, at least about 85, at least about 90, at least about 95, or at least about 100 contiguous amino acid residues of the encoded polypeptide. In this regard, the longer fragments are preferred over the shorter ones.

The present disclosure also provides dimers comprising an isolated polypeptide comprising a variant SIGIRR polypeptide wherein the polypeptide is selected from any of the polypeptides disclosed herein.

In some embodiments, the isolated polypeptides disclosed herein are linked or fused to heterologous polypeptides or heterologous molecules or labels, numerous examples of which are disclosed elsewhere herein. For example, the proteins can be fused to a heterologous polypeptide providing increased or decreased stability. The fused domain or heterologous polypeptide can be located at the N-terminus, the C-terminus, or internally within the polypeptide. A fusion partner may, for example, assist in providing T helper epitopes (an immunological fusion partner), or may assist in expressing the protein (an expression enhancer) at higher yields than the native recombinant polypeptide. Certain fusion partners are both immunological and expression enhancing fusion partners. Other fusion partners may be selected to increase the solubility of the polypeptide or to facilitate targeting the polypeptide to desired intracellular compartments. Some fusion partners include affinity tags, which facilitate purification of the polypeptide.

In some embodiments, a fusion protein is directly fused to the heterologous molecule or is linked to the heterologous molecule via a linker, such as a peptide linker. Suitable peptide linker sequences may be chosen, for example, based on the following factors: 1) the ability to adopt a flexible extended conformation; 2) the resistance to adopt a secondary structure that could interact with functional epitopes on the first and second polypeptides; and 3) the lack of hydrophobic or charged residues that might react with the polypeptide functional epitopes. For example, peptide linker sequences may contain Gly, Asn and Ser residues. Other near neutral amino acids, such as Thr and Ala may also be used in the linker sequence. Amino acid sequences which may be usefully employed as linkers include those disclosed in, for example, Maratea et al., Gene, 1985, 40, 39-46; Murphy et al., Proc. Natl. Acad. Sci. USA, 1986, 83, 8258-8262; and U.S. Patents 4,935,233 and 4,751,180. A linker sequence may generally be, for example, from 1 to about 50 amino acids in length. Linker sequences are generally not required when the first and second polypeptides have non-essential N-terminal amino acid regions that can be used to separate the functional domains and prevent steric interference.

In some embodiments, the polypeptides are operably linked to a cell-penetrating domain. For example, the cell-penetrating domain can be derived from the HIV-1 TAT protein, the TLM cell-penetrating motif from human hepatitis B virus, MPG, Pep-1, VP22, a cell-penetrating peptide from Herpes simplex virus, or a polyarginine peptide sequence. *See, e.g.,* WO 2014/089290. The cell-penetrating domain can be located at the N-terminus, the C-terminus, or anywhere within the protein.

In some embodiments, the polypeptides are operably linked to a heterologous polypeptide for ease of tracking or purification, such as a fluorescent protein, a purification tag, or an epitope tag. Examples of fluorescent proteins include, but are not limited to, green fluorescent proteins (e.g., GFP, GFP-2, tagGFP, turboGFP, eGFP, Emerald, Azami Green, Monomeric Azami Green, CopGFP, AceGFP, ZsGreenl), yellow fluorescent proteins (e.g., YFP, eYFP, Citrine, Venus, YPet, PhiYFP, ZsYellowl), blue fluorescent proteins (e.g., eBFP, eBFP2, Azurite, mKalamal, GFPuv, Sapphire, T-sapphire), cyan fluorescent proteins (e.g., eCFP, Cerulean, CyPet, AmCyanl, Midoriishi-Cyan), red fluorescent proteins (e.g., mKate, mKate2, mPlum, DsRed monomer, mCherry, mRFP1, DsRed-Express, DsRed2, DsRed-Monomer, HcRed-Tandem, HcRedl, AsRed2, eqFP611, mRaspberry, mStrawberry, Jred), orange fluorescent proteins (e.g., mOrange, mKO, Kusabira-Orange, Monomeric Kusabira-Orange, mTangerine, tdTomato), and any other suitable fluorescent protein. Examples of tags include, but are not limited to, glutathione-S-transferase (GST), chitin binding protein (CBP), maltose binding protein, thioredoxin (TRX), poly(NANP), tandem affinity purification (TAP) tag, myc, AcV5, AU1, AU5, E, ECS, E2, FLAG, hemagglutinin (HA), nus, Softag 1, Softag 3, Strep, SBP, Glu-Glu, HSV, KT3, S, S1, T7, V5, VSV-G, histidine (His), biotin carboxyl carrier protein (BCCP), and calmodulin. In some embodiments, the heterologous molecule is an immunoglobulin Fc domain, a peptide tag, a transduction domain, poly(ethylene glycol), polysialic acid, or glycolic acid.

In some embodiments, isolated polypeptides comprise non-natural or modified amino acids or peptide analogs. For example, there are numerous D-amino acids or amino acids which have a different functional substituent than the naturally occurring amino acids. The opposite stereo isomers of naturally occurring peptides are disclosed, as well as the stereo isomers of peptide analogs. These amino acids can readily be incorporated into polypeptide chains by charging tRNA molecules with the amino acid of choice and engineering genetic constructs that utilize, for example, amber codons, to insert the analog amino acid into a peptide chain in a site-specific way.

In some embodiments, the isolated polypeptides are peptide mimetics, which can be produced to resemble peptides, but which are not connected via a natural peptide linkage. For example, linkages for amino acids or amino acid analogs include, but are not limited to, -CH₂NH-, -CH₂S-, -CH₂-, -CH=CH- (cis and trans), -COCH₂-, -CH(OH)CH₂-, and -CHH₂SO-. Peptide analogs can have more than one atom between the bond atoms, such as b-alanine, gaminobutyric acid, and the like. Amino acid analogs and peptide analogs often have enhanced or desirable properties, such as, more economical production, greater chemical stability, enhanced pharmacological properties (half-life, absorption, potency, efficacy, and so forth), altered specificity (e.g., a broad-spectrum of biological activities), reduced antigenicity, and others desirable properties.

In some embodiments, the isolated polypeptides comprise D-amino acids, which can be used to generate more stable peptides because D amino acids are not recognized by peptidases. Systematic substitution of one or more amino acids of a consensus sequence with a D-amino acid of the same type (e.g., D-lysine in place of L-lysine) can be used to generate more stable peptides. Cysteine residues can be used to cyclize or attach two or more peptides together. This can be beneficial to constrain peptides into particular conformations *(see, e.g.,* Rizo and Gierasch, Ann. Rev. Biochem., 1992, 61, 387).

The present disclosure also provides nucleic acid molecules encoding any of the polypeptides disclosed herein. This includes all degenerate sequences related to a specific polypeptide sequence (all nucleic acids having a sequence that encodes one particular polypeptide sequence as well as all nucleic acids, including degenerate nucleic acids, encoding the disclosed variants and derivatives of the protein sequences). Thus, while each particular nucleic acid sequence may not be written out herein, each and every sequence is in fact disclosed and described herein through the disclosed polypeptide sequences.

Percent identity (or percent complementarity) between particular stretches of nucleic acid sequences within nucleic acids or amino acid sequences within polypeptides can be determined routinely using BLAST programs (basic local alignment search tools) and PowerBLAST programs (Altschul et al., J. Mol. Biol., 1990, 215, 403-410; Zhang and Madden, Genome Res., 1997, 7, 649-656) or by using the Gap program (Wisconsin Sequence Analysis Package, Version 8 for Unix, Genetics Computer Group, University Research Park, Madison Wis.), using default settings, which uses the algorithm of Smith and Waterman (Adv. Appl. Math., 1981, 2, 482-489). Herein, if reference is made to percent sequence identity, the higher percentages of sequence identity are preferred over the lower ones.

The present disclosure also provides compositions comprising any one or more of the nucleic acid molecules and/or any one or more of the polypeptides disclosed herein and a carrier and/or excipient. In some embodiments, the carrier increases the stability of the nucleic acid molecule and/or polypeptide (e.g., prolonging the period under given conditions of storage *(e.g.,* -20°C, 4°C, or ambient temperature) for which degradation products remain below a threshold, such as below 0.5% by weight of the starting nucleic acid or protein; or increasing the stability *in vivo).* Examples of carriers include, but are not limited to, poly(lactic acid) (PLA) microspheres, poly(D,L-lactic-coglycolic-acid) (PLGA) microspheres, liposomes, micelles, inverse micelles, lipid cochleates, and lipid microtubules. A carrier may comprise a buffered salt solution such as PBS, HBSS, etc.

The present disclosure also provides methods of producing any of the polypeptides or fragments thereof disclosed herein. Such polypeptides or fragments thereof can be produced by any suitable method. For example, polypeptides or fragments thereof can be produced from host cells comprising nucleic acid molecules (e.g., recombinant expression vectors) encoding such polypeptides or fragments thereof. Such methods can comprise culturing a host cell comprising a nucleic acid molecule (e.g., recombinant expression vector) encoding a polypeptide or fragment thereof under conditions sufficient to produce the polypeptide or fragment thereof, thereby producing the polypeptide or fragment thereof. The nucleic acid can be operably linked to a promoter active in the host cell, and the culturing can be carried out under conditions whereby the nucleic acid is expressed. Such methods can further comprise recovering the expressed polypeptide or fragment thereof. The recovering can further comprise purifying the polypeptide or fragment thereof.

Examples of suitable systems for protein expression include host cells such as, for example: bacterial cell expression systems *(e.g., Escherichia coli, Lactococcus lactis),* yeast cell expression systems *(e.g., Saccharomyces cerevisiae, Pichia pastoris),* insect cell expression systems (e.g., baculovirus-mediated protein expression), and mammalian cell expression systems.

Examples of nucleic acid molecules encoding polypeptides or fragments thereof are disclosed in more detail elsewhere herein. In some embodiments, the nucleic acid molecules are codon optimized for expression in the host cell. In some embodiments, the nucleic acid molecules are operably linked to a promoter active in the host cell. The promoter can be a heterologous promoter (e.g., a promoter than is not a naturally occurring promoter). Examples of promoters suitable for *Escherichia coli* include, but are not limited to, arabinose, *lac, tac,* and T7 promoters. Examples of promoters suitable for *Lactococcus lactis* include, but are not limited to, P170 and nisin promoters. Examples of promoters suitable for *Saccharomyces cerevisiae* include, but are not limited to, constitutive promoters such as alcohol dehydrogenase (ADHI) or enolase (ENO) promoters or inducible promoters such as PHO, CUP1, GAL1, and G10. Examples of promoters suitable for *Pichia pastoris* include, but are not limited to, the alcohol oxidase I (AOX I) promoter, the glyceraldehyde 3 phosphate dehydrogenase (GAP) promoter, and the glutathione dependent formaldehyde dehydrogenase (FLDI) promoter. An example of a promoter suitable for a baculovirus-mediated system is the late viral strong polyhedrin promoter.

In some embodiments, the nucleic acid molecules encode a tag in frame with the polypeptide or fragment thereof to facilitate protein purification. Examples of tags are disclosed elsewhere herein. Such tags can, for example, bind to a partner ligand (e.g., immobilized on a resin) such that the tagged protein can be isolated from all other proteins (e.g., host cell proteins). Affinity chromatography, high performance liquid chromatography (HPLC), and size exclusion chromatography (SEC) are examples of methods that can be used to improve the purity of the expressed protein.

Other methods can also be used to produce polypeptides or fragments thereof. For example, two or more peptides or polypeptides can be linked together by protein chemistry techniques. For example, peptides or polypeptides can be chemically synthesized using either Fmoc (9-fluorenylmethyloxycarbonyl) or Boc (tert -butyloxycarbonoyl) chemistry. Such peptides or polypeptides can be synthesized by standard chemical reactions. For example, a peptide or polypeptide can be synthesized and not cleaved from its synthesis resin, whereas the other fragment of a peptide or protein can be synthesized and subsequently cleaved from the resin, thereby exposing a terminal group which is functionally blocked on the other fragment. By peptide condensation reactions, these two fragments can be covalently joined via a peptide bond at their carboxyl and amino termini, respectively. Alternately, the peptide or polypeptide can be independently synthesized *in vivo* as described herein. Once isolated, these independent peptides or polypeptides may be linked to form a peptide or fragment thereof via similar peptide condensation reactions.

In some embodiments, enzymatic ligation of cloned or synthetic peptide segments allow relatively short peptide fragments to be joined to produce larger peptide fragments, polypeptides, or whole protein domains (Abrahmsen et al., Biochemistry, 1991, 30, 4151). Alternately, native chemical ligation of synthetic peptides can be utilized to synthetically construct large peptides or polypeptides from shorter peptide fragments. This method can consist of a two-step chemical reaction (Dawson et al., Science, 1994, 266, 776-779). The first step can be the chemoselective reaction of an unprotected synthetic peptide-thioester with another unprotected peptide segment containing an amino-terminal Cys residue to give a thioester-linked intermediate as the initial covalent product. Without a change in the reaction conditions, this intermediate can undergo spontaneous, rapid intramolecular reaction to form a native peptide bond at the ligation site.

In some embodiments, unprotected peptide segments can be chemically linked where the bond formed between the peptide segments as a result of the chemical ligation is an unnatural (non-peptide) bond (Schnolzer et al., Science, 1992, 256, 221).

In some embodiments, the polypeptides can possess post-expression modifications such as, for example, glycosylations, acetylations, and phosphorylations, as well as other modifications known in the art, both naturally occurring and non-naturally occurring. A polypeptide may be an entire protein, or a subsequence thereof.

The present disclosure also provides methods of producing any of the polypeptides disclosed herein, comprising culturing a host cell comprising a recombinant expression vectors comprising nucleic acid molecules comprising a polynucleotide capable of encoding one or more of the polypeptides disclosed herein, or its complement, thereby producing the polypeptide.

The present disclosure also provides cells (e.g., recombinant host cells) comprising any one or more of the nucleic acid molecules, including vectors comprising the nucleic acid molecules, and/or any one or more of the polypeptides disclosed herein. The cells can be *in vitro, ex vivo,* or *in vivo.* Nucleic acid molecules can be linked to a promoter and other regulatory sequences so they are expressed to produce an encoded protein. Cell lines of such cells are further provided.

In some embodiments, the cell is a totipotent cell or a pluripotent cell *(e.g.,* an embryonic stem (ES) cell such as a rodent ES cell, a mouse ES cell, or a rat ES cell). Totipotent cells include undifferentiated cells that can give rise to any cell type, and pluripotent cells include undifferentiated cells that possess the ability to develop into more than one differentiated cell types. Such pluripotent and/or totipotent cells can be, for example, ES cells or ES-like cells, such as an induced pluripotent stem (iPS) cells. ES cells include embryo-derived totipotent or pluripotent cells that are capable of contributing to any tissue of the developing embryo upon introduction into an embryo. ES cells can be derived from the inner cell mass of a blastocyst and are capable of differentiating into cells of any of the three vertebrate germ layers (endoderm, ectoderm, and mesoderm). In accordance with the present disclosure, the embryonic stem cells may be non-human embryonic stem cells.

In some embodiments, the cell is a primary somatic cell, or a cell that is not a primary somatic cell. Somatic cells can include any cell that is not a gamete, germ cell, gametocyte, or undifferentiated stem cell. In some embodiments, the cell can also be a primary cell. Primary cells include cells or cultures of cells that have been isolated directly from an organism, organ, or tissue. Primary cells include cells that are neither transformed nor immortal. Primary cells include any cell obtained from an organism, organ, or tissue which was not previously passed in tissue culture or has been previously passed in tissue culture but is incapable of being indefinitely passed in tissue culture. Such cells can be isolated by conventional techniques and include, for example, somatic cells, hematopoietic cells, endothelial cells, epithelial cells, fibroblasts, mesenchymal cells, keratinocytes, melanocytes, monocytes, mononuclear cells, adipocytes, preadipocytes, neurons, glial cells, hepatocytes, skeletal myoblasts, and smooth muscle cells. For example, primary cells can be derived from connective tissues, muscle tissues, nervous system tissues, or epithelial tissues.

In some embodiments, the cells may normally not proliferate indefinitely but, due to mutation or alteration, have evaded normal cellular senescence and instead can keep undergoing division. Such mutations or alterations can occur naturally or be intentionally induced. Examples of immortalized cells include, but are not limited to, Chinese hamster ovary (CHO) cells, human embryonic kidney cells (e.g., HEK 293 cells), and mouse embryonic fibroblast cells (e.g., 3T3 cells). Numerous types of immortalized cells are well known. Immortalized or primary cells include cells that are typically used for culturing or for expressing recombinant genes or proteins. In some embodiments, the cell is a differentiated cell, such as a liver cell *(e.g.,* a human liver cell).

The cell can be from any source. For example, the cell can be a eukaryotic cell, an animal cell, a plant cell, or a fungal (e.g., yeast) cell. Such cells can be fish cells or bird cells, or such cells can be mammalian cells, such as human cells, non-human mammalian cells, rodent cells, mouse cells or rat cells. Mammals include, but are not limited to, humans, non-human primates, monkeys, apes, cats dogs, horses, bulls, deer, bison, sheep, rodents (e.g., mice, rats, hamsters, guinea pigs), livestock (e.g., bovine species such as cows, steer, etc.; ovine species such as sheep, goats, etc.; and porcine species such as pigs and boars). Birds include, but are not limited to, chickens, turkeys, ostrich, geese, ducks, etc. Domesticated animals and agricultural animals are also included. The term "non-human animal" excludes humans.

Additional host cells are described in, for example, U.S. Patent Application Publication No. US2018/0030114,

The nucleic acid molecules and polypeptides disclosed herein can be introduced into a cell by any means. Transfection protocols as well as protocols for introducing nucleic acids or proteins into cells may vary. Non-limiting transfection methods include chemical-based transfection methods using liposomes, nanoparticles, calcium, dendrimers, and cationic polymers such as DEAE-dextran or polyethylenimine. Non-chemical methods include electroporation, sono-poration, and optical transfection. Particle-based transfection includes the use of a gene gun, or magnet-assisted transfection. Viral methods can also be used for transfection.

Introduction of nucleic acids or proteins into a cell can also be mediated by electroporation, by intracytoplasmic injection, by viral infection, by adenovirus, by adeno-associated virus, by lentivirus, by retrovirus, by transfection, by lipid-mediated transfection, or by nucleofection. Nucleofection is an improved electroporation technology that enables nucleic acid substrates to be delivered not only to the cytoplasm but also through the nuclear membrane and into the nucleus. In addition, use of nucleofection in the methods disclosed herein typically requires much fewer cells than regular electroporation (e.g., only about 2 million compared with 7 million by regular electroporation). In some embodiments, nucleofection is performed using the LONZA^{®} NUCLEOFECTOR^{™} system.

Introduction of nucleic acids or proteins into a cell can also be accomplished by microinjection. Microinjection of an mRNA is usually into the cytoplasm (e.g., to deliver mRNA directly to the translation machinery), while microinjection of a protein or a DNA is usually into the nucleus. Alternately, microinjection can be carried out by injection into both the nucleus and the cytoplasm: a needle can first be introduced into the nucleus and a first amount can be injected, and while removing the needle from the cell a second amount can be injected into the cytoplasm. If a nuclease agent protein is injected into the cytoplasm, the protein may comprise a nuclear localization signal to ensure delivery to the nucleus/pronucleus.

Other methods for introducing nucleic acid or proteins into a cell can include, for example, vector delivery, particle-mediated delivery, exosome-mediated delivery, lipid-nanoparticle-mediated delivery, cell-penetrating-peptide-mediated delivery, or implantable-device-mediated delivery. Methods of administering nucleic acids or proteins to a subject to modify cells *in vivo* are disclosed elsewhere herein. Introduction of nucleic acids and proteins into cells can also be accomplished by hydrodynamic delivery (HDD).

Other methods for introducing nucleic acid or proteins into a cell can include, for example, vector delivery, particle-mediated delivery, exosome-mediated delivery, lipid-nanoparticle-mediated delivery, cell-penetrating-peptide-mediated delivery, or implantabledevice-mediated delivery. In some embodiments, a nucleic acid or protein can be introduced into a cell in a carrier such as a poly(lactic acid) (PLA) microsphere, a poly(D,L-lactic-coglycolic-acid) (PLGA) microsphere, a liposome, a micelle, an inverse micelle, a lipid cochleate, or a lipid microtubule.

The present disclosure also provides probes and primers. Examples of probes and primers are disclosed above for example. The present disclosure provides probes and primers comprising a nucleic acid sequence that specifically hybridizes to any of the nucleic acid molecules disclosed herein. For example, the probe or primer may comprise a nucleic acid sequence which hybridizes to any of the nucleic acid molecules described herein that encode a variant SIGIRR protein that is truncated at a position corresponding to position 215 according to SEQ ID NO:9, or which hybridizes to the complement of the nucleic acid molecule. In some embodiments, the probe or primer comprises a nucleic acid sequence which hybridizes to a nucleic acid molecule encoding a variant SIGIRR protein according to SEQ ID NO:9, or which hybridizes to the complement of this nucleic acid molecule. In some embodiments, the probe or primer comprises a nucleic acid sequence which hybridizes to a nucleic acid molecule encoding a variant SIGIRR polypeptide that is truncated at a position corresponding to position 215 according to SEQ ID NO:9, and comprises a serine at a position corresponding to position 186 according to SEQ ID NO:9, or which hybridizes to the complement of this nucleic acid molecule. In some embodiments, the probe or primer comprises a nucleic acid sequence which hybridizes to a nucleic acid molecule encoding a variant SIGIRR polypeptide that is truncated at a position corresponding to position 215 according to SEQ ID NO:9, and comprises a plurality of the amino acids at positions corresponding to positions 186 to 215 according to SEQ ID NO:9, or which hybridizes to the complement of this nucleic acid molecule. In some embodiments, the probe or primer comprises a nucleic acid sequence which hybridizes to a nucleic acid molecule encoding a variant SIGIRR polypeptide that is truncated at a position corresponding to position 215 according to SEQ ID NO:9, and comprises the following amino acid sequence at positions corresponding to positions 186 to 215 according to SEQ ID NO:9: Ser-Arg-Ser-Trp-Ser-Gly-Val-Gly-Ala-Thr-Ser-Ser-Ser-Trp-Thr-Thr-Ala-Thr-Ser-Cys-Arg-Ala-Leu-Ser-Pro-Pro-Pro-Thr-Ser-Trp (SEQ ID NO:11), or which hybridizes to the complement of this nucleic acid molecule. In some embodiments, the probe or primer comprises a nucleic acid sequence which hybridizes to a nucleic acid molecule encoding a variant SIGIRR polypeptide that has at least about 90%, at least about 91%, at least about 92%, at least about 93%, at least about 94%, at least about 95%, at least about 96%, at least about 97%, at least about 98%, or at least about 99% sequence identity to the amino acid sequence according to SEQ ID NO:9, or which hybridizes to the complement of this nucleic acid molecule. In some embodiments, the probe or primer comprises a nucleic acid sequence which hybridizes to a nucleic acid molecule encoding a variant SIGIRR polypeptide that comprises or consists of the amino acid sequence according to SEQ ID NO:9, or which hybridizes to the complement of this nucleic acid molecule. In some embodiments, the probe or primer specifically hybridizes to a portion of the nucleic acid molecule encompassing the codon which encodes a serine at the position corresponding to the position 186 according to SEQ ID NO:9.

The probe or primer may comprise any suitable length, non-limiting examples of which include at least about 5, at least about 8, at least about 10, at least about 11, at least about 12, at least about 13, at least about 14, at least about 15, at least about 16, at least about 17, at least about 18, at least about 19, at least about 20, at least about 21, at least about 22, at least about 23, at least about 24, or at least about 25 nucleotides in length. In preferred embodiments, the probe or primer comprises at least about 18 nucleotides in length. The probe or primer may comprise from about 10 to about 35, from about 10 to about 30, from about 10 to about 25, from about 12 to about 30, from about 12 to about 28, from about 12 to about 24, from about 15 to about 30, from about 15 to about 25, from about 18 to about 30, from about 18 to about 25, from about 18 to about 24, or from about 18 to about 22 nucleotides in length. In preferred embodiments, the probe or primer is from about 18 to about 30 nucleotides in length.

The present disclosure also provides alteration-specific probes and alteration-specific primers. The alteration-specific probe or alteration-specific primer comprises a nucleic acid sequence which is complementary to and/or hybridizes, or specifically hybridizes, to a nucleic acid sequence encoding a variant SIGIRR protein that is truncated at a position corresponding to position 215 according to SEQ ID NO:9, or to the complement thereof. In the context of the disclosure "specifically hybridizes" means that the probe or primer (e.g., the alteration-specific probe or alteration-specific primer) does not hybridize to a nucleic acid molecule encoding a wild type SIGIRR protein. In some embodiments, the alteration-specific probe specifically hybridizes to the nucleic acid codon which encodes the serine at a position corresponding to position 186 according to SEQ ID NO:9, or the complement thereof. In some embodiments, the alteration-specific primer, or primer pair, specifically hybridizes to a region(s) of the nucleic acid molecule encoding a variant SIGIRR protein such that the codon which encodes the serine at a position corresponding to position 186 according to SEQ ID NO:9 is encompassed within any transcript produced therefrom. In some embodiments, the probe or primer specifically hybridizes to a portion of the nucleic acid molecule encompassing the codon which encodes a serine at the position corresponding to the position 186 according to SEQ ID NO:9.

In some embodiments, the alteration-specific probe or alteration-specific primer comprises a nucleic acid sequence which is complementary to and/or hybridizes, or specifically hybridizes, to a nucleic acid sequence encoding a variant SIGIRR protein, wherein the protein comprises a truncation at a position corresponding to position 215 according to SEQ ID NO:9, or the complement thereof.

In some embodiments, the alteration-specific probe or alteration-specific primer comprises a nucleic acid sequence which is complementary to and/or hybridizes, or specifically hybridizes, to a genomic DNA molecule encoding a variant SIGIRR protein truncated at a position corresponding to position 215 according to SEQ ID NO:9, or the complement thereof. In some embodiments, the alteration-specific probe or alteration-specific primer comprises a nucleic acid sequence which is complementary to and/or hybridizes, or specifically hybridizes, to a genomic DNA molecule encoding a variant SIGIRR protein truncated at a position corresponding to position 215 according to SEQ ID NO:9, and that comprises a serine at a position corresponding to position 186 according to SEQ ID NO:9. In some embodiments, the alteration-specific probe or alteration-specific primer comprises a nucleic acid sequence which is complementary to and/or hybridizes, or specifically hybridizes, to a genomic DNA molecule encoding a variant SIGIRR protein truncated at a position corresponding to position 215 according to SEQ ID NO:9, and that comprises the following amino acid sequence at positions corresponding to positions 186 to 215 according to SEQ ID NO:9: Ser-Arg-Ser-Trp-Ser-Gly-Val-Gly-Ala-Thr-Ser-Ser-Ser-Trp-Thr-Thr-Ala-Thr-Ser-Cys-Arg-Ala-Leu-Ser-Pro-Pro-Pro-Thr-Ser-Trp (SEQ ID NO:11). In some embodiments, the alteration-specific probe or alteration-specific primer comprises a nucleic acid sequence which is complementary to and/or hybridizes, or specifically hybridizes, to a genomic DNA molecule encoding a variant SIGIRR protein having at least about 90%, at least about 91%, at least about 92%, at least about 93%, at least about 94%, at least about 95%, at least about 96%, at least about 97%, at least about 98%, or at least about 99% sequence identity to SEQ ID NO:9. In some embodiments, the alteration-specific probe or alteration-specific primer comprises a nucleic acid sequence which is complementary to and/or hybridizes, or specifically hybridizes, to a genomic DNA molecule encoding a variant SIGIRR protein having SEQ ID NO:9. In some embodiments, the probe or primer specifically hybridizes to a portion of the genomic DNA encompassing the codon which encodes a serine at the position corresponding to the position 186 according to SEQ ID NO:9.

In some embodiments, the alteration-specific probe or alteration-specific primer comprises a nucleic acid sequence which is complementary to and/or hybridizes, or specifically hybridizes, to a genomic DNA molecule that comprises or consists of a nucleic acid sequence comprising a guanine at a position corresponding to position 9962 according to SEQ ID NO:2. In some embodiments, the alteration-specific probe or alteration-specific primer comprises a nucleic acid sequence which is complementary to and/or hybridizes, or specifically hybridizes, to a genomic DNA molecule that comprises or consists of a nucleic acid sequence that has at least about 90%, at least about 91%, at least about 92%, at least about 93%, at least about 94%, at least about 95%, at least about 96%, at least about 97%, at least about 98%, or at least about 99% sequence identity to SEQ ID NO:2. In some embodiments, the alteration-specific probe or alteration-specific primer comprises a nucleic acid sequence which is complementary to and/or hybridizes, or specifically hybridizes, to a genomic DNA molecule that comprises or consists of a nucleic acid sequence according to SEQ ID NO:2.

In some embodiments, the alteration-specific probe or alteration-specific primer comprises a nucleic acid sequence which is complementary to and/or hybridizes, or specifically hybridizes, to an mRNA molecule encoding a variant SIGIRR protein truncated at a position corresponding to position 215 according to SEQ ID NO:9. In some embodiments, the alteration-specific probe or alteration-specific primer comprises a nucleic acid sequence which is complementary to and/or hybridizes, or specifically hybridizes, to an mRNA molecule encoding a variant SIGIRR protein truncated at a position corresponding to position 215 according to SEQ ID NO:9, and that comprises a serine at a position corresponding to position 186 according to SEQ ID NO:9. In some embodiments, the alteration-specific probe or alteration-specific primer comprises a nucleic acid sequence which is complementary to and/or hybridizes, or specifically hybridizes, to an mRNA molecule encoding a variant SIGIRR protein truncated at a position corresponding to position 215 according to SEQ ID NO:9, and that comprises the following amino acid sequence at positions corresponding to positions 186 to 215 according to SEQ ID NO:9: Ser-Arg-Ser-Trp-Ser-Gly-Val-Gly-Ala-Thr-Ser-Ser-Ser-Trp-Thr-Thr-Ala-Thr-Ser-Cys-Arg-Ala-Leu-Ser-Pro-Pro-Pro-Thr-Ser-Trp (SEQ ID NO:11). In some embodiments, the alteration-specific probe or alteration-specific primer comprises a nucleic acid sequence which is complementary to and/or hybridizes, or specifically hybridizes, to an mRNA molecule encoding a variant SIGIRR protein having at least about 90%, at least about 91%, at least about 92%, at least about 93%, at least about 94%, at least about 95%, at least about 96%, at least about 97%, at least about 98%, or at least about 99% sequence identity to SEQ ID NO:9. In some embodiments, the alteration-specific probe or alteration-specific primer comprises a nucleic acid sequence which is complementary to and/or hybridizes, or specifically hybridizes, to an mRNA molecule encoding a variant SIGIRR protein having SEQ ID NO:9. In some embodiments, the probe or primer specifically hybridizes to a portion of the mRNA molecule encompassing the codon which encodes a serine at the position corresponding to the position 186 according to SEQ ID NO:9.

In some embodiments, the alteration-specific probe or alteration-specific primer comprises a nucleic acid sequence which is complementary to and/or hybridizes, or specifically hybridizes, to an mRNA molecule that comprises or consists of a nucleic acid sequence comprising a guanine at a position corresponding to position 557 according to SEQ ID NO:4. In some embodiments, the alteration-specific probe or alteration-specific primer comprises a nucleic acid sequence which is complementary to and/or hybridizes, or specifically hybridizes, to an mRNA molecule that comprises the codons CUA and AGC at positions corresponding to positions 553 to 555 and 556 to 558, respectively, according to SEQ ID NO:4. In some embodiments, the alteration-specific probe or alteration-specific primer comprises a nucleic acid sequence which is complementary to and/or hybridizes, or specifically hybridizes, to an mRNA molecule that comprises or consists of a nucleic acid sequence that has at least about 90%, at least about 91%, at least about 92%, at least about 93%, at least about 94%, at least about 95%, at least about 96%, at least about 97%, at least about 98%, or at least about 99% sequence identity to SEQ ID NO:4. In some embodiments, the alteration-specific probe or alteration-specific primer comprises a nucleic acid sequence which is complementary to and/or hybridizes, or specifically hybridizes, to an mRNA molecule that comprises or consists of a nucleic acid sequence according to SEQ ID NO:4.

In some embodiments, the alteration-specific probe or alteration-specific primer comprises a nucleic acid sequence which is complementary to and/or hybridizes, or specifically hybridizes, to an cDNA molecule encoding a variant SIGIRR protein truncated at a position corresponding to position 215 according to SEQ ID NO:9. In some embodiments, the alteration-specific probe or alteration-specific primer comprises a nucleic acid sequence which is complementary to and/or hybridizes, or specifically hybridizes, to an cDNA molecule encoding a variant SIGIRR protein truncated at a position corresponding to position 215 according to SEQ ID NO:9, and that comprises a serine at a position corresponding to position 186 according to SEQ ID NO:9. In some embodiments, the alteration-specific probe or alteration-specific primer comprises a nucleic acid sequence which is complementary to and/or hybridizes, or specifically hybridizes, to an cDNA molecule encoding a variant SIGIRR protein truncated at a position corresponding to position 215 according to SEQ ID NO:9, and that comprises the following amino acid sequence at positions corresponding to positions 186 to 215 according to SEQ ID NO:9: Ser-Arg-Ser-Trp-Ser-Gly-Val-Gly-Ala-Thr-Ser-Ser-Ser-Trp-Thr-Thr-Ala-Thr-Ser-Cys-Arg-Ala-Leu-Ser-Pro-Pro-Pro-Thr-Ser-Trp (SEQ ID NO:11). In some embodiments, the alteration-specific probe or alteration-specific primer comprises a nucleic acid sequence which is complementary to and/or hybridizes, or specifically hybridizes, to an cDNA molecule encoding a variant SIGIRR protein having at least about 90%, at least about 91%, at least about 92%, at least about 93%, at least about 94%, at least about 95%, at least about 96%, at least about 97%, at least about 98%, or at least about 99% sequence identity to SEQ ID NO:9. In some embodiments, the alteration-specific probe or alteration-specific primer comprises a nucleic acid sequence which is complementary to and/or hybridizes, or specifically hybridizes, to an cDNA molecule encoding a variant SIGIRR protein having SEQ ID NO:9. In some embodiments, the probe or primer specifically hybridizes to a portion of the cDNA molecule encompassing the codon which encodes a serine at the position corresponding to the position 186 according to SEQ ID NO:9.

In some embodiments, the alteration-specific probe or alteration-specific primer comprises a nucleic acid sequence which is complementary to and/or hybridizes, or specifically hybridizes, to an cDNA molecule that comprises or consists of a nucleic acid sequence comprising a guanine at a position corresponding to position 557 according to SEQ ID NO:6. In some embodiments, the alteration-specific probe or alteration-specific primer comprises a nucleic acid sequence which is complementary to and/or hybridizes, or specifically hybridizes, to a cDNA molecule that comprises the codons CUA and AGC at positions corresponding to positions 553 to 555 and 556 to 558, respectively, according to SEQ ID NO:6. In some embodiments, the alteration-specific probe or alteration-specific primer comprises a nucleic acid sequence which is complementary to and/or hybridizes, or specifically hybridizes, to an cDNA molecule that comprises or consists of a nucleic acid sequence that has at least about 90%, at least about 91%, at least about 92%, at least about 93%, at least about 94%, at least about 95%, at least about 96%, at least about 97%, at least about 98%, or at least about 99% sequence identity to SEQ ID NO:6. In some embodiments, the alteration-specific probe or alteration-specific primer comprises a nucleic acid sequence which is complementary to and/or hybridizes, or specifically hybridizes, to an cDNA molecule that comprises or consists of a nucleic acid sequence according to SEQ ID NO:6.

The length which is described above with regard to the probe or primer of the disclosure applies, mutatis mutandis, also for the alteration-specific probe or alteration-specific primer of the disclosure.

The disclosure also provides a pair of alteration-specific primers comprising two of the alteration-specific primers as described above.

In some embodiments, the probe or primer (e.g., the alteration-specific probe or alteration-specific primer) comprises DNA. In some embodiments, the probe or primer (e.g., alteration-specific probe or alteration-specific primer) comprises RNA. In some embodiments, the probe or primer (e.g., the alteration-specific probe or alteration-specific primer) hybridizes to a nucleic acid sequence encoding the variant SIGIRR protein under stringent conditions, such as high stringent conditions.

In some embodiments, the probe comprises a label. In some embodiments, the label is a fluorescent label, a radiolabel, or biotin. In some embodiments, the length of the probe is described above. Alternately, in some embodiments, the probe comprises or consists of at least about 20, at least about 25, at least about 30, at least about 35, at least about 40, at least about 45, at least about 50, at least about 55, at least about 60, at least about 65, at least about 70, at least about 75, at least about 80, at least about 85, at least about 90, at least about 95, or at least about 100 nucleotides. The probe (e.g., the allele-specific probe) may be used, for example, to detect any of the nucleic acid molecules disclosed herein. In preferred embodiments, the probe comprises at least about 18 nucleotides in length. The probe may comprise from about 10 to about 35, from about 10 to about 30, from about 10 to about 25, from about 12 to about 30, from about 12 to about 28, from about 12 to about 24, from about 15 to about 30, from about 15 to about 25, from about 18 to about 30, from about 18 to about 25, from about 18 to about 24, or from about 18 to about 22 nucleotides in length. In preferred embodiments, the probe is from about 18 to about 30 nucleotides in length.

The present disclosure also provides supports comprising a substrate to which any one or more of the probes disclosed herein is attached. Solid supports are solid-state substrates or supports with which molecules, such as any of the probes disclosed herein, can be associated. A form of solid support is an array. Another form of solid support is an array detector. An array detector is a solid support to which multiple different probes have been coupled in an array, grid, or other organized pattern.

Solid-state substrates for use in solid supports can include any solid material to which molecules can be coupled. This includes materials such as acrylamide, agarose, cellulose, nitrocellulose, glass, polystyrene, polyethylene vinyl acetate, polypropylene, polymethacrylate, polyethylene, polyethylene oxide, polysilicates, polycarbonates, teflon, fluorocarbons, nylon, silicon rubber, polyanhydrides, polyglycolic acid, polylactic acid, polyorthoesters, polypropylfumerate, collagen, glycosaminoglycans, and polyamino acids. Solid-state substrates can have any useful form including thin film, membrane, bottles, dishes, fibers, woven fibers, shaped polymers, particles, beads, microparticles, or a combination. Solid-state substrates and solid supports can be porous or non-porous. A form for a solid-state substrate is a microtiter dish, such as a standard 96-well type. In some embodiments, a multiwell glass slide can be employed that normally contain one array per well. This feature allows for greater control of assay reproducibility, increased throughput and sample handling, and ease of automation. In some embodiments, the support is a microarray.

Any of the polypeptides disclosed herein can further have one or more substitutions (such as conservative amino acid substitutions), insertions, or deletions. Insertions include, for example, amino or carboxyl terminal fusions as well as intrasequence insertions of single or multiple amino acid residues. Techniques for making substitutions at predetermined sites in DNA having a known sequence are well known, for example M13 primer mutagenesis and PCR mutagenesis. Amino acid substitutions are typically of single residues, but can occur at a number of different locations at once; insertions usually will be on the order of about from 1 to 10 amino acid residues; and deletions will range about from 1 to 30 residues. Deletions or insertions can be made in adjacent pairs, i.e. a deletion of 2 residues or insertion of 2 residues. Substitutions, deletions, insertions or any combination thereof may be combined to arrive at a final construct. In some embodiments, the mutations do not place the sequence out of reading frame and do not create complementary regions that could produce secondary mRNA structure.

The present disclosure also provides kits for making the compositions and utilizing the methods described herein. The kits described herein can comprise an assay or assays for detecting one or more genetic variants in a sample of a subject.

In some embodiments, the kits for human identification of SIGIRR variants utilize the compositions and methods described above. In some embodiments, a basic kit can comprise a container having at least one pair of oligonucleotide primers or probes, such as alteration-specific probes or alteration-specific primers, for a locus in any of the nucleic acid molecules disclosed herein (such as, for example, SEQ ID NO:2, SEQ ID NO:4, and/or SEQ ID NO:6). A kit can also optionally comprise instructions for use. A kit can also comprise other optional kit components, such as, for example, one or more of an allelic ladder directed to each of the loci amplified, a sufficient quantity of enzyme for amplification, amplification buffer to facilitate the amplification, divalent cation solution to facilitate enzyme activity, dNTPs for strand extension during amplification, loading solution for preparation of the amplified material for electrophoresis, genomic DNA as a template control, a size marker to insure that materials migrate as anticipated in the separation medium, and a protocol and manual to educate the user and limit error in use. The amounts of the various reagents in the kits also can be varied depending upon a number of factors, such as the optimum sensitivity of the process. It is within the scope of these teachings to provide test kits for use in manual applications or test kits for use with automated sample preparation, reaction set-up, detectors or analyzers.

In some embodiments, the kits comprise at least one pair of oligonucleotide primers (e.g., alteration-specific primers) for amplification, or at least one labeled oligonucleotide probe (e.g., alteration-specific probe) for detection, of a genomic DNA molecule encoding a variant SIGIRR protein truncated at a position corresponding to position 215 according to SEQ ID NO:9, or the complement thereof. In some embodiments, the kits comprise at least one pair of oligonucleotide primers (e.g., alteration-specific primers) for amplification, or at least one labeled oligonucleotide probe (e.g., alteration-specific probe) for detection, of a genomic DNA molecule encoding a variant SIGIRR protein truncated at a position corresponding to position 215 according to SEQ ID NO:9, and that comprises a serine at a position corresponding to position 186 according to SEQ ID NO:9. In some embodiments, the kits comprise at least one pair of oligonucleotide primers (e.g., alteration-specific primers) for amplification, or at least one labeled oligonucleotide probe (e.g., alteration-specific probe) for detection, of a genomic DNA molecule encoding a variant SIGIRR protein truncated at a position corresponding to position 215 according to SEQ ID NO:9, and that comprises the following amino acid sequence at positions corresponding to positions 186 to 215 according to SEQ ID NO:9: Ser-Arg-Ser-Trp-Ser-Gly-Val-Gly-Ala-Thr-Ser-Ser-Ser-Trp-Thr-Thr-Ala-Thr-Ser-Cys-Arg-Ala-Leu-Ser-Pro-Pro-Pro-Thr-Ser-Trp (SEQ ID NO:11). In some embodiments, the kits comprise at least one pair of oligonucleotide primers (e.g., alteration-specific primers) for amplification, or at least one labeled oligonucleotide probe (e.g., alteration-specific probe) for detection, of a genomic DNA molecule encoding a variant SIGIRR protein having at least about 90%, at least about 91%, at least about 92%, at least about 93%, at least about 94%, at least about 95%, at least about 96%, at least about 97%, at least about 98%, or at least about 99% sequence identity to SEQ ID NO:9. In some embodiments, the kits comprise at least one pair of oligonucleotide primers (e.g., alteration-specific primers) for amplification, or at least one labeled oligonucleotide probe (e.g., alteration-specific probe) for detection, of a genomic DNA molecule encoding a variant SIGIRR protein having SEQ ID NO:9.

In some embodiments, the kits comprise at least one pair of oligonucleotide primers (e.g., alteration-specific primers) for amplification, or at least one labeled oligonucleotide probe (e.g., alteration-specific probe) for detection, of a genomic DNA molecule that comprises or consists of a nucleic acid sequence comprising a guanine at a position corresponding to position 9962 according to SEQ ID NO:2. In some embodiments, the kits comprise at least one pair of oligonucleotide primers (e.g., alteration-specific primers) for amplification, or at least one labeled oligonucleotide probe (e.g., alteration-specific probe) for detection, of a genomic DNA molecule that comprises or consists of a nucleic acid sequence that has at least about 90%, at least about 91%, at least about 92%, at least about 93%, at least about 94%, at least about 95%, at least about 96%, at least about 97%, at least about 98%, or at least about 99% sequence identity to SEQ ID NO:2. In some embodiments, the kits comprise at least one pair of oligonucleotide primers (e.g., alteration-specific primers) for amplification, or at least one labeled oligonucleotide probe (e.g., alteration-specific probe) for detection, of a genomic DNA molecule that comprises or consists of a nucleic acid sequence according to SEQ ID NO:2.

In some embodiments, the kits comprise at least one pair of oligonucleotide primers (e.g., alteration-specific primers) for amplification, or at least one labeled oligonucleotide probe (e.g., alteration-specific probe) for detection, of an mRNA molecule encoding a variant SIGIRR protein truncated at a position corresponding to position 215 according to SEQ ID NO:9. In some embodiments, the kits comprise at least one pair of oligonucleotide primers (e.g., alteration-specific primers) for amplification, or at least one labeled oligonucleotide probe (e.g., alteration-specific probe) for detection, of an mRNA molecule encoding a variant SIGIRR protein truncated at a position corresponding to position 215 according to SEQ ID NO:9, and that comprises a serine at a position corresponding to position 186 according to SEQ ID NO:9. In some embodiments, the kits comprise at least one pair of oligonucleotide primers (e.g., alteration-specific primers) for amplification, or at least one labeled oligonucleotide probe (e.g., alteration-specific probe) for detection, of an mRNA molecule encoding a variant SIGIRR protein truncated at a position corresponding to position 215 according to SEQ ID NO:9, and that comprises the following amino acid sequence at positions corresponding to positions 186 to 215 according to SEQ ID NO:9: Ser-Arg-Ser-Trp-Ser-Gly-Val-Gly-Ala-Thr-Ser-Ser-Ser-Trp-Thr-Thr-Ala-Thr-Ser-Cys-Arg-Ala-Leu-Ser-Pro-Pro-Pro-Thr-Ser-Trp (SEQ ID NO:11). In some embodiments, the kits comprise at least one pair of oligonucleotide primers (e.g., alteration-specific primers) for amplification, or at least one labeled oligonucleotide probe (e.g., alteration-specific probe) for detection, of an mRNA molecule encoding a variant SIGIRR protein having at least about 90%, at least about 91%, at least about 92%, at least about 93%, at least about 94%, at least about 95%, at least about 96%, at least about 97%, at least about 98%, or at least about 99% sequence identity to SEQ ID NO:9. In some embodiments, the kits comprise at least one pair of oligonucleotide primers (e.g., alteration-specific primers) for amplification, or at least one labeled oligonucleotide probe (e.g., alteration-specific probe) for detection, of an mRNA molecule encoding a variant SIGIRR protein having SEQ ID NO:9.

In some embodiments, the kits comprise at least one pair of oligonucleotide primers (e.g., alteration-specific primers) for amplification, or at least one labeled oligonucleotide probe (e.g., alteration-specific probe) for detection, of an mRNA molecule that comprises or consists of a nucleic acid sequence comprising a guanine at a position corresponding to position 557 according to SEQ ID NO:4. In some embodiments, the kits comprise at least one pair of oligonucleotide primers (e.g., alteration-specific primers) for amplification, or at least one labeled oligonucleotide probe (e.g., alteration-specific probe) for detection, of an mRNA molecule that comprises the codons CUA and AGC at positions corresponding to positions 553 to 555 and 556 to 558, respectively, according to SEQ ID NO:4. In some embodiments, the kits comprise at least one pair of oligonucleotide primers (e.g., alteration-specific primers) for amplification, or at least one labeled oligonucleotide probe (e.g., alteration-specific probe) for detection, of an mRNA molecule that comprises or consists of a nucleic acid sequence that has at least about 90%, at least about 91%, at least about 92%, at least about 93%, at least about 94%, at least about 95%, at least about 96%, at least about 97%, at least about 98%, or at least about 99% sequence identity to SEQ ID NO:4. In some embodiments, the kits comprise at least one pair of oligonucleotide primers (e.g., alteration-specific primers) for amplification, or at least one labeled oligonucleotide probe (e.g., alteration-specific probe) for detection, of an mRNA molecule that comprises or consists of a nucleic acid sequence according to SEQ ID NO:4.

In some embodiments, the kits comprise at least one pair of oligonucleotide primers (e.g., alteration-specific primers) for amplification, or at least one labeled oligonucleotide probe (e.g., alteration-specific probe) for detection, of an cDNA molecule encoding a variant SIGIRR protein truncated at a position corresponding to position 215 according to SEQ ID NO:9. In some embodiments, the kits comprise at least one pair of oligonucleotide primers (e.g., alteration-specific primers) for amplification, or at least one labeled oligonucleotide probe (e.g., alteration-specific probe) for detection, of an cDNA molecule encoding a variant SIGIRR protein truncated at a position corresponding to position 215 according to SEQ ID NO:9, and that comprises a serine at a position corresponding to position 186 according to SEQ ID NO:9. In some embodiments, the kits comprise at least one pair of oligonucleotide primers (e.g., alteration-specific primers) for amplification, or at least one labeled oligonucleotide probe (e.g., alteration-specific probe) for detection, of an cDNA molecule encoding a variant SIGIRR protein truncated at a position corresponding to position 215 according to SEQ ID NO:9, and that comprises the following amino acid sequence at positions corresponding to positions 186 to 215 according to SEQ ID NO:9: Ser-Arg-Ser-Trp-Ser-Gly-Val-Gly-Ala-Thr-Ser-Ser-Ser-Trp-Thr-Thr-Ala-Thr-Ser-Cys-Arg-Ala-Leu-Ser-Pro-Pro-Pro-Thr-Ser-Trp (SEQ ID NO:11). In some embodiments, the kits comprise at least one pair of oligonucleotide primers (e.g., alteration-specific primers) for amplification, or at least one labeled oligonucleotide probe (e.g., alteration-specific probe) for detection, of an cDNA molecule encoding a variant SIGIRR protein having at least about 90%, at least about 91%, at least about 92%, at least about 93%, at least about 94%, at least about 95%, at least about 96%, at least about 97%, at least about 98%, or at least about 99% sequence identity to SEQ ID NO:9. In some embodiments, the kits comprise at least one pair of oligonucleotide primers (e.g., alteration-specific primers) for amplification, or at least one labeled oligonucleotide probe (e.g., alteration-specific probe) for detection, of an cDNA molecule encoding a variant SIGIRR protein having SEQ ID NO:9.

In some embodiments, the kits comprise at least one pair of oligonucleotide primers (e.g., alteration-specific primers) for amplification, or at least one labeled oligonucleotide probe (e.g., alteration-specific probe) for detection, of an cDNA molecule that comprises or consists of a nucleic acid sequence comprising a guanine at a position corresponding to position 557 according to SEQ ID NO:6. In some embodiments, the kits comprise at least one pair of oligonucleotide primers (e.g., alteration-specific primers) for amplification, or at least one labeled oligonucleotide probe (e.g., alteration-specific probe) for detection, of a cDNA molecule that comprises the codons CTA and AGC at positions corresponding to positions 553 to 555 and 556 to 558, respectively, according to SEQ ID NO:6. In some embodiments, the kits comprise at least one pair of oligonucleotide primers (e.g., alteration-specific primers) for amplification, or at least one labeled oligonucleotide probe (e.g., alteration-specific probe) for detection, of an cDNA molecule that comprises or consists of a nucleic acid sequence that has at least about 90%, at least about 91%, at least about 92%, at least about 93%, at least about 94%, at least about 95%, at least about 96%, at least about 97%, at least about 98%, or at least about 99% sequence identity to SEQ ID NO:6. In some embodiments, the kits comprise at least one pair of oligonucleotide primers (e.g., alteration-specific primers) for amplification, or at least one labeled oligonucleotide probe (e.g., alteration-specific probe) for detection, of an cDNA molecule that comprises or consists of a nucleic acid sequence according to SEQ ID NO:6.

In some embodiments, any of the kits disclosed herein may further comprise any one or more of: a nucleotide ladder, protocol, an enzyme (such as an enzyme used for amplification, such as polymerase chain reaction (PCR)), dNTPs, a buffer, a salt or salts, and a control nucleic acid sample. In some embodiments, any of the kits disclosed herein may further comprise any one or more of: a detectable label, products and reagents required to carry out an annealing reaction, and instructions.

In some embodiments, the kits disclosed herein can comprise a primer or probe or an alteration-specific primer or an alteration-specific probe comprising a 3' terminal nucleotide that hybridizes directly to a guanine at a position corresponding to position 9962 of SEQ ID NO:2, or at a position corresponding to position 557 of SEQ ID NO:4 and/or SEQ ID NO:6.

Those in the art understand that the detection techniques employed are generally not limiting. Rather, a wide variety of detection means are within the scope of the disclosed methods and kits, provided that they allow the presence or absence of an amplicon to be determined.

In some aspects, a kit can comprise one or more of the primers or probes disclosed herein. For example, a kit can comprise one or more probes that hybridize to one or more of the disclosed genetic variants.

In some aspects, a kit can comprise one of the disclosed cells or cell lines. In some aspects, a kit can comprise the materials necessary to create a transgenic cell or cell line. For example, in some aspects a kit can comprise a cell and a vector comprising a nucleic acid sequence comprising one or more of the disclosed genetic variants. A kit can further comprise media for cell culture.

The present disclosure also provides methods for detecting the presence of a *SIGIRR* variant genomic DNA, mRNA, cDNA, and/or polypeptide in a biological sample from a subject human. It is understood that gene sequences within a population and mRNAs and proteins encoded by such genes can vary due to polymorphisms such as single-nucleotide polymorphisms. The sequences provided herein for the SIGIRR genomic DNA, mRNA, cDNA, and polypeptide are only exemplary sequences. Other sequences for the *SIGIRR* genomic DNA, mRNA, cDNA, and polypeptide are also possible.

The biological sample can be derived from any cell, tissue, or biological fluid from the subject. The sample may comprise any clinically relevant tissue, such as a bone marrow sample, a tumor biopsy, a fine needle aspirate, or a sample of bodily fluid, such as blood, gingival crevicular fluid, plasma, serum, lymph, ascitic fluid, cystic fluid, or urine. In some cases, the sample comprises a buccal swab. The sample used in the methods disclosed herein will vary based on the assay format, nature of the detection method, and the tissues, cells, or extracts that are used as the sample. A biological sample can be processed differently depending on the assay being employed. For example, when detecting a variant SIGIRR nucleic acid molecule, preliminary processing designed to isolate or enrich the sample for the genomic DNA can be employed. A variety of known techniques may be used for this purpose. When detecting the level of variant SIGIRR mRNA, different techniques can be used enrich the biological sample with mRNA. Various methods to detect the presence or level of a mRNA or the presence of a particular variant genomic DNA locus can be used.

In some embodiments, the disclosure provides methods of detecting the presence or absence of a variant SIGIRR protein comprising sequencing at least a portion of a protein in a biological sample to determine whether the protein comprises an amino acid sequence encoding a truncated SIGIRR protein. In some embodiments, the disclosure provides methods of detecting the presence or absence of a variant SIGIRR protein comprising sequencing at least a portion of a protein in a biological sample to determine whether the protein comprises an amino acid sequence encoding a SIGIRR protein truncated at a position corresponding to position 215 according to SEQ ID NO:9. In some embodiments, the disclosure provides methods of detecting the presence or absence of a variant SIGIRR protein comprising sequencing at least a portion of a protein in a biological sample to determine whether the protein comprises an amino acid sequence encoding a SIGIRR protein truncated at a position corresponding to position 215 according to SEQ ID NO:9, and comprises a serine at the position corresponding to position 186 according to SEQ ID NO:9.

In some embodiments, the disclosure provides methods of detecting the presence or absence of a variant SIGIRR nucleic acid molecule comprising sequencing at least a portion of a nucleic acid in a biological sample to determine whether the nucleic acid comprises a nucleic acid sequence encoding a truncated SIGIRR protein. In some embodiments, the disclosure provides methods of detecting the presence or absence of a variant SIGIRR nucleic acid molecule comprising sequencing at least a portion of a nucleic acid in a biological sample to determine whether the nucleic acid comprises a nucleic acid sequence encoding a SIGIRR protein truncated at a position corresponding to position 215 according to SEQ ID NO:9. In some embodiments, the disclosure provides methods of detecting the presence or absence of a variant SIGIRR nucleic acid molecule comprising sequencing at least a portion of a nucleic acid in a biological sample to determine whether the nucleic acid comprises a nucleic acid sequence encoding a SIGIRR protein truncated at a position corresponding to position 215 according to SEQ ID NO:9, and comprises a serine at the position corresponding to position 186 according to SEQ ID NO:9. Any of the variant nucleic acid molecules disclosed herein can be detected using any of the probes and primers described herein.

In some embodiments, the methods of detecting the presence or absence of an inflammatory bowel disease-associated variant SIGIRR nucleic acid molecule or an early-onset inflammatory bowel disease-associated variant SIGIRR nucleic acid molecule (e.g., genomic DNA, mRNA, or cDNA) in a subject, comprising: performing an assay on a biological sample obtained from the subject, which assay determines whether a nucleic acid molecule in the biological sample comprises any of the variant SIGIRR nucleic acid sequences disclosed herein *(e.g.,* a nucleic acid molecule that encodes a truncated SIGIRR protein, a nucleic acid molecule that encodes a SIGIRR protein truncated at a position corresponding to position 215 according to SEQ ID NO:9, a nucleic acid molecule that encodes a SIGIRR protein truncated at a position corresponding to position 215 according to SEQ ID NO:9 and comprising a serine at the position corresponding to position 186 according to SEQ ID NO:9). In some embodiments, the biological sample comprises a cell or cell lysate. Such methods can further comprise, for example, obtaining a biological sample from the subject comprising a *SIGIRR* genomic DNA or mRNA, and if mRNA, optionally reverse transcribing the mRNA into cDNA, and performing an assay on the biological sample that determine whether a position of the SIGIRR genomic DNA, mRNA, or cDNA encodes a truncated SIGIRR protein. Such methods can further comprise, for example, obtaining a biological sample from the subject comprising a *SIGIRR* genomic DNA or mRNA, and if mRNA, optionally reverse transcribing the mRNA into cDNA, and performing an assay on the biological sample that determine whether a position of the SIGIRR genomic DNA, mRNA, or cDNA encodes a SIGIRR protein truncated at a position corresponding to position 215 according to SEQ ID NO:9, or performing an assay on the biological sample that determine whether a position of the *SIGIRR* genomic DNA, mRNA, or cDNA encodes a SIGIRR protein truncated at a position corresponding to position 215 according to SEQ ID NO:9 and comprising a serine at the position corresponding to position 186 according to SEQ ID NO:9. Such assays can comprise, for example determining the identity of these positions of the particular SIGIRR nucleic acid molecule. In some embodiments, the subject is a human.

In some embodiments, the assay comprises: sequencing at least a portion of the *SIGIRR* genomic DNA sequence of a nucleic acid molecule in the biological sample from the subject, wherein the portion sequenced includes the position corresponding to the position encoding a serine at position 186 in the SIGIRR protein according to SEQ ID NO:9; sequencing at least a portion of the *SIGIRR* mRNA sequence of a nucleic acid molecule in the biological sample from the subject, wherein the portion sequenced includes the position corresponding to the position encoding a serine at position 186 in the SIGIRR protein according to SEQ ID NO:9; or sequencing at least a portion of the *SIGIRR* cDNA sequence of a nucleic acid molecule in the biological sample from the subject, wherein the portion sequenced includes the position corresponding to the position encoding a serine at position 186 in the SIGIRR protein according to SEQ ID NO:9.

In some embodiments, the assay comprises: a) contacting the biological sample with a primer hybridizing to: i) a portion of the SIGIRR genomic DNA sequence that is proximate to a position of the *SIGIRR* genomic sequence at the position corresponding to the position encoding a serine at position 186 according to SEQ ID NO:9; ii) a portion of the SIGIRR mRNA sequence that is proximate to a position of the *SIGIRR* mRNA sequence at the position corresponding to the position encoding a serine at position 186 according to SEQ ID NO:9; or iii) a portion of the *SIGIRR* cDNA sequence that is proximate to a position of the *SIGIRR* cDNA sequence at the position corresponding to the position encoding a serine at position 186 according to SEQ ID NO:9; b) extending the primer at least through: i) the position of the *SIGIRR* genomic DNA sequence corresponding to nucleotide positions beyond the codon encoding a serine at position 186 according to SEQ ID NO:9; ii) the position of the *SIGIRR* mRNA sequence corresponding to nucleotide positions beyond the codon encoding a serine at position 186 according to SEQ ID NO:9; or iii) the position of the *SIGIRR* cDNA sequence corresponding to nucleotide positions beyond the codon encoding a serine at position 186 according to SEQ ID NO:9; and c) determining whether the extension product of the primer comprises nucleotides encoding a serine at the position corresponding to position 186 according to SEQ ID NO:9. In some embodiments, only *SIGIRR* genomic DNA is analyzed. In some embodiments, only *SIGIRR* mRNA is analyzed. In some embodiments, only *SIGIRR* cDNA obtained from *SIGIRR* mRNA is analyzed.

In some embodiments, the assay comprises: a) contacting the biological sample with an alteration-specific primer hybridizing to i) a portion of the *SIGIRR* genomic DNA sequence including the nucleotides encoding a serine at the position corresponding to position 186 according to SEQ ID NO:9; ii) a portion of the *SIGIRR* mRNA sequence including the nucleotides encoding a serine at the position corresponding to position 186 according to SEQ ID NO:9; or iii) a portion of the *SIGIRR* cDNA sequence including the nucleotides encoding a serine at the position corresponding to position 186 according to SEQ ID NO:9; b) extending the primer using an alteration-specific polymerase chain reaction technique; and c) determining whether extension occurred. Alteration-specific polymerase chain reaction techniques can be used to detect mutations such as deletions in a nucleic acid sequence. Alteration-specific primers are used because the DNA polymerase will not extend when a mismatch with the template is present. A number of variations of the basic alteration-specific polymerase chain reaction technique are at the disposal of the skilled artisan.

The alteration-specific primer may comprise a nucleic acid sequence which is complementary to a nucleic acid sequence encoding the SIGIRR protein comprising a serine at the position corresponding to position 186 according to SEQ ID NO:9, or the complement to the nucleic acid sequence. For example, the alteration-specific primer may comprise a nucleic acid sequence which is complementary to the nucleic acid sequence encoding SEQ ID NO:9, or to the complement to this nucleic acid sequence. The alteration-specific primer preferably specifically hybridizes to the nucleic acid sequence encoding the variant SIGIRR protein when the nucleic acid sequence encodes a serine at the position corresponding to position 186 according to SEQ ID NO:9.

In some embodiments, the assay comprises contacting the biological sample with a primer or probe that specifically hybridizes to a variant SIGIRR genomic DNA sequence, mRNA sequence, or cDNA sequence and not the corresponding wild type SIGIRR sequence under stringent conditions, and determining whether hybridization has occurred.

In some embodiments, the assay comprises RNA sequencing (RNA-Seq). In some embodiments, the assays also comprise reverse transcribing mRNA into cDNA via the reverse transcriptase polymerase chain reaction (RT-PCR).

In some embodiments, the methods utilize probes and primers of sufficient nucleotide length to bind to the target nucleic acid sequence and specifically detect and/or identify a polynucleotide comprising a variant SIGIRR genomic DNA, mRNA, or cDNA. The hybridization conditions or reaction conditions can be determined by the operator to achieve this result. This nucleotide length may be any length that is sufficient for use in a detection method of choice, including any assay described or exemplified herein. Generally, for example, primers or probes having about 8, about 10, about 11, about 12, about 14, about 15, about 16, about 18, about 20, about 22, about 24, about 26, about 28, about 30, about 40, about 50, about 75, about 100, about 200, about 300, about 400, about 500, about 600, or about 700 nucleotides, or more, or from about 11 to about 20, from about 20 to about 30, from about 30 to about 40, from about 40 to about 50, from about 50 to about 100, from about 100 to about 200, from about 200 to about 300, from about 300 to about 400, from about 400 to about 500, from about 500 to about 600, from about 600 to about 700, or from about 700 to about 800, or more nucleotides in length are used. In preferred embodiments, the probe or primer comprises at least about 18 nucleotides in length. The probe or primer may comprise from about 10 to about 35, from about 10 to about 30, from about 10 to about 25, from about 12 to about 30, from about 12 to about 28, from about 12 to about 24, from about 15 to about 30, from about 15 to about 25, from about 18 to about 30, from about 18 to about 25, from about 18 to about 24, or from about 18 to about 22 nucleotides in length. In preferred embodiments, the probe or primer is from about 18 to about 30 nucleotides in length.

Such probes and primers can hybridize specifically to a target sequence under high stringency hybridization conditions. Probes and primers may have complete nucleic acid sequence identity of contiguous nucleotides with the target sequence, although probes differing from the target nucleic acid sequence and that retain the ability to specifically detect and/or identify a target nucleic acid sequence may be designed by conventional methods. Accordingly, probes and primers can share about 80%, about 85%, about 90%, about 91%, about 92%, about 93%, about 94%, about 95%, about 96%, about 97%, about 98%, about 99%, or 100% sequence identity or complementarity to the target nucleic acid molecule.

In some embodiments, specific primers can be used to amplify the variant *SIGIRR* locus and/or SIGIRR variant mRNA or cDNA to produce an amplicon that can be used as a specific probe or can itself be detected for identifying the variant *SIGIRR* locus or for determining the level of specific SIGIRR mRNA or cDNA in a biological sample. The *SIGIRR* variant locus can be used to denote a genomic nucleic acid sequence including positions corresponding to positions encoding a serine at position 186 according to SEQ ID NO:9. When the probe is hybridized with a nucleic acid molecule in a biological sample under conditions that allow for the binding of the probe to the nucleic acid molecule, this binding can be detected and allow for an indication of the presence of the variant *SIGIRR* locus or the presence or the level of variant *SIGIRR* mRNA or cDNA in the biological sample. Such identification of a bound probe has been described. The specific probe may comprise a sequence of at least about 80%, from about 80% to about 85%, from about 85% to about 90%, from about 90% to about 95%, and from about 95% to about 100% identical (or complementary) to a specific region of a variant *SIGIRR* gene. The specific probe may comprise a sequence of at least about 80%, from about 80% to about 85%, from about 85% to about 90%, from about 90% to about 95%, and from about 95% to about 100% identical (or complementary) to a specific region of a variant *SIGIRR* mRNA. The specific probe may comprise a sequence of at least about 80%, from about 80% to about 85%, from about 85% to about 90%, from about 90% to about 95%, and from about 95% to about 100% identical (or complementary) to a specific region of a variant *SIGIRR* cDNA.

In some embodiments, to determine whether the nucleic acid complement of a biological sample comprises a nucleic acid sequence encoding the variant SIGIRR protein (e.g., a truncated SIGIRR protein, or a variant SIGIRR protein having a serine at the position corresponding to position 186 according to SEQ ID NO:9), the biological sample may be subjected to a nucleic acid amplification method using a primer pair that includes a first primer derived from the 5' flanking sequence adjacent to positions encoding the serine at the position corresponding to position 186 according to SEQ ID NO:9, and a second primer derived from the 3' flanking sequence adjacent to positions encoding the serine at the position corresponding to position 186 according to SEQ ID NO:9, to produce an amplicon that is diagnostic for the presence of the nucleotides at positions encoding the serine at the position corresponding to position 186 according to SEQ ID NO:9. In some embodiments, the amplicon may range in length from the combined length of the primer pairs plus one nucleotide base pair to any length of amplicon producible by a DNA amplification protocol. This distance can range from one nucleotide base pair up to the limits of the amplification reaction, or about twenty thousand nucleotide base pairs. Optionally, the primer pair flanks a region including positions encoding the serine at position 186 according to SEQ ID NO:9 and at least 1, 2, 3, 4, 5, 6, 7, 8, 9, 10, or more nucleotides on each side of positions encoding the serine at position 186 according to SEQ ID NO:9. Similar amplicons can be generated from the mRNA and/or cDNA sequences.

Representative methods for preparing and using probes and primers are described, for example, in Molecular Cloning: A Laboratory Manual, 2nd Ed., Vol. 1-3, ed. Sambrook et al., Cold Spring Harbor Laboratory Press, Cold Spring Harbor, N.Y. 1989 (hereinafter, "Sambrook *et al.,* 1989"); Current Protocols in Molecular Biology, ed. Ausubel et al., Greene Publishing and Wiley-Interscience, New York, 1992 (with periodic updates) (hereinafter, "Ausubel *et al.,* 1992"); and Innis et al., PCR Protocols: A Guide to Methods and Applications, Academic Press: San Diego, 1990). PCR primer pairs can be derived from a known sequence, for example, by using computer programs intended for that purpose, such as the PCR primer analysis tool in Vector NTI version 10 (Informax Inc., Bethesda Md.); PrimerSelect (DNASTAR Inc., Madison, Wis.); and Primer3 (Version 0.4.0.COPYRGT., 1991, Whitehead Institute for Biomedical Research, Cambridge, Mass.). Additionally, the sequence can be visually scanned and primers manually identified using known guidelines.

Any nucleic acid hybridization or amplification or sequencing method can be used to specifically detect the presence of the variant *SIGIRR* gene locus and/or the level of variant *SIGIRR* mRNA or cDNA produced from mRNA. In some embodiments, the nucleic acid molecule can be used either as a primer to amplify a region of the *SIGIRR* nucleic acid or the nucleic acid molecule can be used as a probe that specifically hybridizes, for example, under stringent conditions, to a nucleic acid molecule comprising the variant *SIGIRR* gene locus or a nucleic acid molecule comprising a variant *SIGIRR* mRNA or cDNA produced from mRNA.

A variety of techniques are available in the art including, for example, nucleic acid sequencing, nucleic acid hybridization, and nucleic acid amplification. Illustrative examples of nucleic acid sequencing techniques include, but are not limited to, chain terminator (Sanger) sequencing and dye terminator sequencing.

Other methods involve nucleic acid hybridization methods other than sequencing, including using labeled primers or probes directed against purified DNA, amplified DNA, and fixed cell preparations (fluorescence *in situ* hybridization (FISH)). In some methods, a target nucleic acid may be amplified prior to or simultaneous with detection. Illustrative examples of nucleic acid amplification techniques include, but are not limited to, polymerase chain reaction (PCR), ligase chain reaction (LCR), strand displacement amplification (SDA), and nucleic acid sequence based amplification (NASBA). Other methods include, but are not limited to, ligase chain reaction, strand displacement amplification, and thermophilic SDA (tSDA).

Any method can be used for detecting either the non-amplified or amplified polynucleotides including, for example, Hybridization Protection Assay (HPA), quantitative evaluation of the amplification process in real-time, and determining the quantity of target sequence initially present in a sample, but which is not based on a real-time amplification.

Also provided are methods for identifying nucleic acids which do not necessarily require sequence amplification and are based on, for example, the known methods of Southern (DNA:DNA) blot hybridizations, *in situ* hybridization (ISH), and fluorescence *in situ* hybridization (FISH) of chromosomal material. Southern blotting can be used to detect specific nucleic acid sequences. In such methods, nucleic acid that is extracted from a sample is fragmented, electrophoretically separated on a matrix gel, and transferred to a membrane filter. The filter bound nucleic acid is subject to hybridization with a labeled probe complementary to the sequence of interest. Hybridized probe bound to the filter is detected. In any such methods, the process can include hybridization using any of the probes described or exemplified herein.

In hybridization techniques, stringent conditions can be employed such that a probe or primer will specifically hybridize to its target. In some embodiments, a polynucleotide primer or probe under stringent conditions will hybridize to its target sequence (*e.g.,* the variant *SIGIRR* gene locus, variant *SIGIRR* mRNA, or variant *SIGIRR* cDNA) to a detectably greater degree than to other sequences (*e.g.,* the corresponding wild type *SIGIRR* locus, wild type mRNA, or wild type cDNA), such as, at least 2-fold, at least 3-fold, at least 4-fold, or more over background, including over 10-fold over background. In some embodiments, a polynucleotide primer or probe under stringent conditions will hybridize to its target sequence to a detectably greater degree than to other sequences by at least 2-fold. In some embodiments, a polynucleotide primer or probe under stringent conditions will hybridize to its target sequence to a detectably greater degree than to other sequences by at least 3-fold. In some embodiments, a polynucleotide primer or probe under stringent conditions will hybridize to its target sequence to a detectably greater degree than to other sequences by at least 4-fold. In some embodiments, a polynucleotide primer or probe under stringent conditions will hybridize to its target sequence to a detectably greater degree than to other sequences by over 10-fold over background. Stringent conditions are sequence-dependent and will be different in different circumstances. By controlling the stringency of the hybridization and/or washing conditions, target sequences that are 100% complementary to the probe can be identified (homologous probing). Alternately, stringency conditions can be adjusted to allow some mismatching in sequences so that lower degrees of identity are detected (heterologous probing).

Appropriate stringency conditions which promote DNA hybridization, for example, 6X sodium chloride/sodium citrate (SSC) at about 45°C., followed by a wash of 2X SSC at 50°C, are known or can be found in Current Protocols in Molecular Biology, John Wiley & Sons, N.Y. (1989), 6.3.1-6.3.6. Typically, stringent conditions for hybridization and detection will be those in which the salt concentration is less than about 1.5 M Na ion, typically about 0.01 to 1.0 M Na ion concentration (or other salts) at pH 7.0 to 8.3 and the temperature is at least about 30°C for short probes *(e.g.,* 10 to 50 nucleotides) and at least about 60°C for longer probes *(e.g.,* greater than 50 nucleotides). Stringent conditions may also be achieved with the addition of destabilizing agents such as formamide. Exemplary low stringency conditions include hybridization with a buffer solution of 30 to 35% formamide, 1 M NaCl, 1% SDS (sodium dodecyl sulfate) at 37°C, and a wash in 1X to 2X SSC (20X SSC = 3.0 M NaCl/0.3 M trisodium citrate) at 50 to 55°C. Exemplary moderate stringency conditions include hybridization in 40 to 45% formamide, 1.0 M NaCl, 1% SDS at 37°C, and a wash in 0.5X to 1X SSC at 55 to 60°C. Exemplary high stringency conditions include hybridization in 50% formamide, 1 M NaCl, 1% SDS at 37°C, and a wash in 0.1X SSC at 60 to 65°C. Optionally, wash buffers may comprise about 0.1% to about 1% SDS. Duration of hybridization is generally less than about 24 hours, usually about 4 to about 12 hours. The duration of the wash time will be at least a length of time sufficient to reach equilibrium.

In hybridization reactions, specificity is typically the function of post-hybridization washes, the critical factors being the ionic strength and temperature of the final wash solution. For DNA-DNA hybrids, the Tₘ can be approximated from the equation of Meinkoth and Wahl, Anal. Biochem., 1984, 138, 267-284: Tₘ = 81.5°C + 16.6 (log M) + 0.41 (% GC) - 0.61 (% form) - 500/L; where M is the molarity of monovalent cations, %GC is the percentage of guanosine and cytosine nucleotides in the DNA, % form is the percentage of formamide in the hybridization solution, and L is the length of the hybrid in base pairs. The Tₘ is the temperature (under defined ionic strength and pH) at which 50% of a complementary target sequence hybridizes to a perfectly matched probe. Tₘ is reduced by about 1°C for each 1% of mismatching; thus, Tₘ, hybridization, and/or wash conditions can be adjusted to hybridize to sequences of the desired identity. For example, if sequences with ≥90% identity are sought, the Tₘ can be decreased 10°C. Generally, stringent conditions are selected to be about 5°C lower than the thermal melting point (Tₘ) for the specific sequence and its complement at a defined ionic strength and pH. However, severely stringent conditions can utilize a hybridization and/or wash at 1°C, 2°C, 3°C, or 4°C lower than the thermal melting point (Tₘ); moderately stringent conditions can utilize a hybridization and/or wash at 6°C, 7°C, 8°C, 9°C, or 10°C lower than the thermal melting point (Tₘ); low stringency conditions can utilize a hybridization and/or wash at 11°C, 12°C, 13°C, 14°C, 15°C, or 20°C lower than the thermal melting point (Tₘ). Using the equation, hybridization and wash compositions, and desired Tₘ, those of ordinary skill will understand that variations in the stringency of hybridization and/or wash solutions are inherently described. If the desired degree of mismatching results in a Tₘ of less than 45°C (aqueous solution) or 32°C (formamide solution), it is optimal to increase the SSC concentration so that a higher temperature can be used.

Also provided are methods for detecting the presence or quantifying the levels of variant SIGIRR polypeptide in a biological sample, including, for example, protein sequencing and immunoassays. In some embodiments, the method of detecting the presence of variant SIGIRR protein (*e.g.,* SEQ D NO:9) in a human subject comprises performing an assay on a biological sample from the human subject that detects the presence of the variant SIGIRR protein (*e.g.,* SEQ ID NO:4) in the biological sample.

Illustrative non-limiting examples of protein sequencing techniques include, but are not limited to, mass spectrometry and Edman degradation. Illustrative examples of immunoassays include, but are not limited to, immunoprecipitation, Western blot, immunohistochemistry, ELISA, immunocytochemistry, flow cytometry, and immuno-PCR. Polyclonal or monoclonal antibodies detectably labeled using various known techniques (*e.g.,* calorimetric, fluorescent, chemiluminescent, or radioactive) are suitable for use in the immunoassays. Regarding immunoassays, the variant SIGIRR protein has a different size as compared to the wild type SIGIRR protein and, therefore, runs at a different molecular weight on a protein gel. Thus, by using the same antibody, the wild type SIGIRR protein can be distinguished from the variant SIGIRR protein in, for example, a Western Blot assay.

The present disclosure also provides methods for diagnosing inflammatory bowel disease or early-onset inflammatory bowel disease or detecting a risk of developing inflammatory bowel disease or early-onset inflammatory bowel disease in a human subject, comprising: detecting in a nucleic acid molecule obtained from the human subject any of the alterations in any of the SIGIRR nucleic acid molecules described herein; and diagnosing the human subject with inflammatory bowel disease or early-onset inflammatory bowel disease if the subject has one or more symptoms of inflammatory bowel disease or early-onset inflammatory bowel disease, or diagnosing the human subject as at risk for developing inflammatory bowel disease or early-onset inflammatory bowel disease if the subject does not have one or more symptoms of inflammatory bowel disease or early-onset inflammatory bowel disease. In some embodiments, the human subject is in need of such diagnosis. In some embodiments, the human subject may have relatives that have been diagnosed with inflammatory bowel disease or early-onset inflammatory bowel disease.

Symptoms of inflammatory bowel disease or early-onset inflammatory bowel disease include, but are not limited to, diarrhea, fever, fatigue, abdominal pain, abdominal cramping, nausea, vomiting, the presence of blood in the stool, anemia, reduced appetite, and unintended weight loss, or any combination thereof.

In some embodiments, the methods comprise detecting the presence of the variant *SIGIRR* genomic DNA, mRNA, or cDNA obtained from mRNA obtained from a biological sample obtained from the subject. It is understood that gene sequences within a population and mRNAs encoded by such genes can vary due to polymorphisms such as single nucleotide polymorphisms (SNPs). The sequences provided herein for the *SIGIRR* genomic DNA, mRNA, cDNA, and polypeptide are only exemplary sequences and other such sequences, including additional *SIGIRR* alleles are also possible.

In some embodiments, the detecting step comprises sequencing at least a portion of the nucleic acid molecule that encodes a truncated SIGIRR protein. In some embodiments, the detecting step comprises sequencing at least a portion of the nucleic acid molecule that encodes a SIGIRR protein, wherein the sequenced nucleic acid molecule encodes an amino acid sequence which comprises the position corresponding to position 186 according to SEQ ID NO:9. Any of the nucleic acid molecules disclosed herein (*e.g.,* genomic DNA, mRNA, or cDNA) can be sequenced. In some embodiments, the detecting step comprises sequencing the entire nucleic acid molecule.

In some embodiments, the detecting step comprises: amplifying at least a portion of the nucleic acid molecule that encodes a truncated SIGIRR protein; labeling the nucleic acid molecule with a detectable label; contacting the labeled nucleic acid with a support comprising a probe, wherein the probe comprises a nucleic acid sequence which hybridizes under stringent conditions to a nucleic acid sequence encoding the truncated SIGIRR protein; and detecting the detectable label. In some embodiments, the detecting step comprises: amplifying at least a portion of the nucleic acid molecule that encodes a SIGIRR protein, wherein the amplified nucleic acid molecule encodes an amino acid sequence which comprises the position corresponding to position 186 according to SEQ ID NO:9; labeling the nucleic acid molecule with a detectable label; contacting the labeled nucleic acid with a support comprising a probe, wherein the probe comprises a nucleic acid sequence which hybridizes under stringent conditions to a nucleic acid sequence encoding aspartic acid at the position corresponding to position at 186 according to SEQ ID NO:9; and detecting the detectable label. Any of the nucleic acid molecules disclosed herein can be amplified. For example, any of the genomic DNA, cDNA, or mRNA molecules disclosed herein can be amplified. In some embodiments, the nucleic acid molecule is mRNA and the method further comprises reverse-transcribing the mRNA into a cDNA prior to the amplifying step.

In some embodiments, the detecting step comprises: contacting the nucleic acid molecule that encodes a SIGIRR protein with a probe comprising a detectable label, wherein the probe comprises a nucleic acid sequence which hybridizes under stringent conditions to a nucleic acid sequence encoding a truncated SIGIRR protein, and detecting the detectable label. In some embodiments, the detecting step comprises: contacting the nucleic acid molecule that encodes a SIGIRR protein with a probe comprising a detectable label, wherein the probe comprises a nucleic acid sequence which hybridizes under stringent conditions to a nucleic acid sequence encoding an amino acid sequence which comprises a serine at the position corresponding to position 186 according to SEQ ID NO:9, and detecting the detectable label. In some embodiments, the nucleic acid molecule is present within a cell obtained from the human subject, such that the detection is according to an *in situ* hybridization technique.

In some embodiments, the detecting step comprises contacting the nucleic acid molecule that encodes a SIGIRR protein with an alteration-specific primer, and amplifying the nucleic acid molecule using alteration-specific PCR techniques. The alteration-specific primer may be any such primer described herein, and may be specific to variant SIGIRR proteins that encode a serine at the position corresponding to position 186 according to SEQ ID NO:9.

Other assays that can be used in the methods disclosed herein include, for example, reverse transcription polymerase chain reaction (RT-PCR) or quantitative RT-PCR (qRT-PCR). Yet other assays that can be used in the methods disclosed herein include, for example, RNA sequencing (RNA-Seq) followed by detection of the presence and quantity of variant mRNA or cDNA in the biological sample.

The present disclosure also provides methods for identifying a human subject having inflammatory bowel disease or early-onset inflammatory bowel disease or a risk for developing inflammatory bowel disease or early-onset inflammatory bowel disease. The methods generally comprise detecting in a sample obtained from the subject the presence or absence of a variant SIGIRR protein; and/or the presence or absence of any of the nucleic acid molecules described herein encoding a variant SIGIRR protein. The presence of a truncated SIGIRR protein, indicates that the subject has inflammatory bowel disease or early-onset inflammatory bowel disease or a risk for developing inflammatory bowel disease or early-onset inflammatory bowel disease. The presence of a guanine (due to the deletion of the adenine) at a position corresponding to position 9962 according to SEQ ID NO:2 *(e.g.,* the genomic DNA), or at a position corresponding to position 557 according to SEQ ID NO:4 *(e.g.,* the mRNA), or at a position corresponding to position 557 according to SEQ ID NO:6 (e.g., the cDNA), each resulting in a variant SIGIRR protein truncated at a position corresponding to position 215 according to SEQ ID NO:9 and containing a serine at a position corresponding to position 186 according to SEQ ID NO:9, indicates that the subject has inflammatory bowel disease or early-onset inflammatory bowel disease or a risk for developing inflammatory bowel disease or early-onset inflammatory bowel disease. The method may be carried out *in vitro, in situ,* or *in vivo.*

The present disclosure also provides methods for identifying a human subject having early-onset inflammatory bowel disease or a risk for developing early-onset inflammatory bowel disease, wherein the method comprises detecting in a sample obtained from the subject the presence or absence of: a SIGIRR protein having a serine at the position corresponding to position 186 according to SEQ ID NO:9 and being truncated at the position corresponding to position 215 according to SEQ ID NO:9; and/or a nucleic acid molecule encoding a SIGIRR protein having a serine at the position corresponding to position 186 according to SEQ ID NO:9 and being truncated at the position corresponding to position 215 according to SEQ ID NO:9; wherein the presence of the truncated SIGIRR protein and/or the nucleic acid molecule encoding the truncated SIGIRR protein indicates that the subject has early-onset inflammatory bowel disease or a risk for developing early-onset inflammatory bowel disease. In some embodiments, the truncated SIGIRR protein comprises a different amino acid compared to the wild type SIGIRR protein at any one of the positions corresponding to positions 186 to 209 and 211 to 215 according to SEQ ID NO:9. In some embodiments, the truncated SIGIRR protein comprises the amino acid sequence of SEQ ID NO:11 at the positions corresponding to positions 186 to 215 according to SEQ ID NO:9.

In some embodiments, the presence or absence of the truncated SIGIRR protein in the sample is detected with an antibody which is specific for truncated SIGIRR. In some embodiments, the antibody which is specific for truncated SIGIRR is specific for: i) serine at the position corresponding to position 186 according to SEQ ID NO:9; or ii) an epitope created in the SIGIRR protein because of a frameshift mutation which results in a serine at the position corresponding to position 186 according to SEQ ID NO:9. In some embodiments, the detection further comprises comparing the reaction of the antibody which is specific for truncated SIGIRR with the reaction of an antibody that is specific for wild type SIGIRR. In some embodiments, the presence or absence of said truncated SIGIRR protein in said sample is detected by an enzyme-linked immunosorbent assay (ELISA). In some embodiments, the presence or absence of said nucleic acid molecule encoding said truncated SIGIRR protein in the sample is detected by determining whether there is a frameshift mutation in the nucleic acid molecule creating a codon encoding a serine at the position corresponding to position 186 according to SEQ ID NO:9. In some embodiments, the portion of the nucleic acid molecule sequenced comprises a plurality of positions encompassing the codon encoding the position corresponding to the position 186 according to SEQ ID NO:9.

In some embodiments of the method, the detecting step comprises sequencing at least a portion of the nucleic acid molecule that encodes a SIGIRR protein. In some embodiments of the method, the detecting step comprises sequencing at least a portion of the nucleic acid molecule that encodes a truncated SIGIRR protein. The sequenced nucleic acid molecule may encode an amino acid sequence which comprises a position corresponding to position 186 according to SEQ ID NO:9. The presence of a guanine (due to the deletion of the adenine) at a position corresponding to position 9962 according to SEQ ID NO:2 *(e.g.,* the genomic DNA), or at a position corresponding to position 557 according to SEQ ID NO:4 (*e.g.,* the mRNA), or at a position corresponding to position 557 according to SEQ ID NO:6 (*e.g.,* the cDNA), each resulting in a variant SIGIRR protein truncated at a position corresponding to position 215 according to SEQ ID NO:9 and containing a serine at a position corresponding to position 186 according to SEQ ID NO:9. The detecting step may comprise sequencing the nucleic acid molecule encoding the entire SIGIRR protein.

In some embodiments of the method, the detecting step comprises amplifying at least a portion of the nucleic acid molecule that encodes a truncated SIGIRR protein, labeling the amplified nucleic acid molecule with a detectable label, contacting the labeled nucleic acid molecule with a support comprising a probe, wherein the probe comprises a nucleic acid sequence which specifically hybridizes, including, for example, under stringent conditions, to a nucleic acid sequence encoding the truncated SIGIRR protein, and detecting the detectable label. In some embodiments of the method, the detecting step comprises amplifying at least a portion of the nucleic acid molecule that encodes a SIGIRR protein, labeling the amplified nucleic acid molecule with a detectable label, contacting the labeled nucleic acid molecule with a support comprising a probe, wherein the probe comprises a nucleic acid sequence which specifically hybridizes, including, for example, under stringent conditions, to a nucleic acid sequence encoding a serine at the position corresponding to position 186 according to SEQ ID NO:9 (or a nucleic acid sequence having a guanine (due to the deletion of the adenine) at a position corresponding to position 9962 according to SEQ ID NO:2 *(e.g.,* the genomic DNA), or at a position corresponding to position 557 according to SEQ ID NO:4 *(e.g.,* the mRNA), or at a position corresponding to position 557 according to SEQ ID NO:6 (*e.g.,* the cDNA), each resulting in a variant SIGIRR protein truncated at a position corresponding to position 215 according to SEQ ID NO:9 and containing a serine at a position corresponding to position 186 according to SEQ ID NO:9), and detecting the detectable label. The amplified nucleic acid molecule preferably encodes an amino acid sequence which comprises the position corresponding to position 186 according to SEQ ID NO:9. If the nucleic acid includes mRNA, the method may further comprise reverse-transcribing the mRNA into a cDNA prior to the amplifying step. In some embodiments, the determining step comprises contacting the nucleic acid molecule that encodes a SIGIRR protein with a probe comprising a detectable label and detecting the detectable label. The probe preferably comprises a nucleic acid sequence which specifically hybridizes, including, for example, under stringent conditions, to a nucleic acid sequence encoding an amino acid sequence which comprises a serine at the position corresponding to position at 186 according to SEQ ID NO:9 (or a nucleic acid sequence having a guanine (due to the deletion of the adenine) at a position corresponding to position 9962 according to SEQ ID NO:2 *(e.g.,* the genomic DNA), or at a position corresponding to position 557 according to SEQ ID NO:4 *(e.g.,* the mRNA), or at a position corresponding to position 557 according to SEQ ID NO:6 (*e.g.,* the cDNA), each resulting in a variant SIGIRR protein truncated at a position corresponding to position 215 according to SEQ ID NO:9 and containing a serine at a position corresponding to position 186 according to SEQ ID NO:9). The nucleic acid molecule may be present within a cell obtained from the human subject.

In some embodiments, the detecting step comprises: amplifying at least a portion of the nucleic acid molecule that encodes a SIGIRR protein, wherein the amplified nucleic acid molecule encompasses the codon encoding the amino acid at the position corresponding to position 186 according to SEQ ID NO:9; labeling the amplified nucleic acid molecule with a detectable label; contacting the labeled nucleic acid molecule with a support comprising a probe, wherein the probe comprises a nucleic acid sequence which specifically hybridizes under stringent conditions to a nucleic acid sequence encompassing the codon encoding a serine at the position corresponding to position 186 according to SEQ ID NO:9; and detecting the detectable label.

In some embodiments, the detecting step comprises: contacting a nucleic acid molecule that encodes a SIGIRR protein with a probe comprising a detectable label, wherein the probe comprises a nucleic acid sequence which specifically hybridizes under stringent conditions to a nucleic acid sequence encompassing the codon encoding serine at the position corresponding to position 186 according to SEQ ID NO:9; and detecting the detectable label. In some embodiments, the human subject is younger than 18 years. In some embodiments, the human subject is identified as having Crohn's disease or a risk for developing Crohn's disease.

The present disclosure also provides methods for diagnosing inflammatory bowel disease or early-onset inflammatory bowel disease or detecting a risk of developing inflammatory bowel disease or early-onset inflammatory bowel disease in a human subject, comprising: detecting a truncated SIGIRR protein obtained from the human subject; and diagnosing the human subject with inflammatory bowel disease or early-onset inflammatory bowel disease if the subject has one or more symptoms of inflammatory bowel disease or early-onset inflammatory bowel disease, or diagnosing the human subject as at risk for developing inflammatory bowel disease or early-onset inflammatory bowel disease if the subject does not have one or more symptoms of inflammatory bowel disease or early-onset inflammatory bowel disease. In some embodiments, the human subject is in need of such diagnosis. The present disclosure also provides methods for diagnosing inflammatory bowel disease or early-onset inflammatory bowel disease or detecting a risk of developing inflammatory bowel disease or early-onset inflammatory bowel disease in a human subject, comprising: detecting a variant SIGIRR protein, such as a protein comprising SEQ ID NO:9, obtained from the human subject; and diagnosing the human subject with inflammatory bowel disease or early-onset inflammatory bowel disease if the subject has one or more symptoms of inflammatory bowel disease or early-onset inflammatory bowel disease, or diagnosing the human subject as at risk for developing inflammatory bowel disease or early-onset inflammatory bowel disease if the subject does not have one or more symptoms of inflammatory bowel disease or early-onset inflammatory bowel disease. In some embodiments, the human subject is in need of such diagnosis. In some embodiments, the human subject may have relatives that have been diagnosed with inflammatory bowel disease or early-onset inflammatory bowel disease.

The present disclosure also provides methods for diagnosing early-onset inflammatory bowel disease or detecting a risk of early-onset inflammatory bowel disease in a human subject, comprising: detecting a nucleic acid molecule encoding a SIGIRR protein obtained from the human subject, wherein the a SIGIRR protein has a serine at the position corresponding to position 186 according to SEQ ID NO:9 and is truncated at a position corresponding to position 215 according to SEQ ID NO:9; and/or detecting a SIGIRR protein obtained from the human subject, wherein the SIGIRR protein has a serine at the position corresponding to position 186 according to SEQ ID NO:9 and is truncated at the position corresponding to position 215 according to SEQ ID NO:9; and diagnosing the human subject with early-onset inflammatory bowel disease if the subject has one or more symptoms of early-onset inflammatory bowel disease, or diagnosing the human subject as at risk for early-onset inflammatory bowel disease if the subject does not have one or more symptoms of early-onset inflammatory bowel disease. In some embodiments, the truncated SIGIRR protein comprises a different amino acid compared to the wild type SIGIRR protein at any one of the positions corresponding to positions 186 to 209 and 211 to 215 according to SEQ ID NO:9. In some embodiments, the truncated SIGIRR protein comprises the amino acid sequence of SEQ ID NO:13 at the positions corresponding to positions 186 to 215 according to SEQ ID NO:9. In some embodiments, the truncated SIGIRR protein is detected with an antibody which is specific for truncated SIGIRR. In some embodiments, the antibody which is specific for truncated SIGIRR is specific for: i) serine at the position corresponding to position 186 according to SEQ ID NO:9; or ii) an epitope created in the SIGIRR protein because of a frameshift mutation which results in a serine at the position corresponding to position 186 according to SEQ ID NO:9. In some embodiments, the detection further comprises comparing the reaction of the antibody which is specific for truncated SIGIRR with the reaction of an antibody that is specific for wild type SIGIRR. In some embodiments, the truncated SIGIRR protein is detected by an enzyme-linked immunosorbent assay (ELISA). In some embodiments, the nucleic acid molecule encoding said truncated SIGIRR protein is detected by detecting a frameshift mutation in said nucleic acid molecule creating a codon encoding a serine at the position corresponding to position 186 according to SEQ ID NO:9. In some embodiments, the portion of the nucleic acid molecule sequenced comprises a plurality of positions encompassing the codon encoding the position corresponding to the position 186 according to SEQ ID NO:9.

In some embodiments, the detecting step comprises: amplifying at least a portion of the nucleic acid molecule that encodes a SIGIRR protein, wherein the amplified nucleic acid molecule encompasses the codon encoding the amino acid at the position corresponding to position 186 according to SEQ ID NO:9; labeling the amplified nucleic acid molecule with a detectable label; contacting the labeled nucleic acid molecule with a support comprising a probe, wherein the probe comprises a nucleic acid sequence which specifically hybridizes under stringent conditions to a nucleic acid sequence encompassing the codon encoding a serine at the position corresponding to position 186 according to SEQ ID NO:9; and detecting the detectable label.

In some embodiments, the detecting step comprises: contacting the nucleic acid molecule that encodes a SIGIRR protein with a probe comprising a detectable label, wherein the probe comprises a nucleic acid sequence which specifically hybridizes under stringent conditions to a nucleic acid sequence encompassing the codon encoding serine at the position corresponding to position 186 according to SEQ ID NO:9; and detecting the detectable label.

In some embodiments, the human subject described herein from which a sample is obtained (e.g., the human subject being diagnosed and/or treated) is not an adult. In some embodiments, the human subject described herein from which a sample is obtained is 18 years old or younger. In some embodiments, the human subject described herein from which a sample is obtained is 15 years old or younger. In some embodiments, the human subject described herein from which a sample is obtained is 13 years old or younger. In some embodiments, the human subject described herein from which a sample is obtained is 10 years old or younger. In some embodiments, the human subject that is 6 years old or younger may have very early-onset inflammatory bowel disease. In some embodiments, the human subject does not have necrotizing enterocolitis.

In some embodiments, any of the methods described herein can further comprise treating the subject with an agent effective to treat inflammatory bowel disease or early-onset inflammatory bowel disease. In some embodiments, the methods further comprise treating the subject with an agent effective to treat inflammatory bowel disease or early-onset inflammatory bowel disease when the alteration is detected in the subject and the subject is diagnosed as having inflammatory bowel disease or early-onset inflammatory bowel disease.

The present disclosure also provides uses of any of the variant *SIGIRR* genomic DNA, mRNA, cDNA, polypeptides, and hybridizing nucleic acid molecules disclosed herein in the diagnosis of early-onset inflammatory bowel disease or diagnosing the risk of developing early-onset inflammatory bowel disease.

If different versions of a sequence are associated with an accession number at different times, the version associated with the accession number at the effective filing date of this application is meant. The effective filing date means the earlier of the actual filing date or filing date of a priority application referring to the accession number if applicable. Likewise, if different versions of a publication, website or the like are published at different times, the version most recently published at the effective filing date of the application is meant unless otherwise indicated. Any feature, step, element, embodiment, or aspect of the present disclosure can be used in combination with any other feature, step, element, embodiment, or aspect unless specifically indicated otherwise. Although the present disclosure has been described in some detail by way of illustration and example for purposes of clarity and understanding, it will be apparent that certain changes and modifications may be practiced within the scope of the appended claims, which define the invention.

The nucleotide and amino acid sequences recited herein are shown using standard letter abbreviations for nucleotide bases, and one-letter code for amino acids. The nucleotide sequences follow the standard convention of beginning at the 5' end of the sequence and proceeding forward (i.e., from left to right in each line) to the 3' end. Only one strand of each nucleotide sequence is shown, but the complementary strand is understood to be included by any reference to the displayed strand. The amino acid sequences follow the standard convention of beginning at the amino terminus of the sequence and proceeding forward (i.e., from left to right in each line) to the carboxy terminus.

The following examples are provided to describe the embodiments in greater detail. They are intended to illustrate, not to limit, the claimed embodiments.

### Examples

The following examples are put forth so as to provide those of ordinary skill in the art with a complete disclosure and description of how the subject-matters claimed herein are made and evaluated, and are intended to be purely exemplary and are not intended to limit the scope of the invention, as defined by the appended claims.

Efforts have been made to ensure accuracy with respect to numbers (*e.g.,* amounts, temperature, etc.), but some errors and deviations should be accounted for. Unless indicated otherwise, parts are parts by weight, temperature is in °C or is at ambient temperature, and pressure is at or near atmospheric.

### Example 1: Patient Recruitment and Phenotyping

Whole exome sequencing and trio-based variant analysis was performed on a 13-year-old EO-IBD patient, his IBD-affected mother, and his unaffected father. This family had been ascertained for genetic evaluation of EO-IBD in the patient.

### Example 2: Genomic Samples

Genomic DNA was extracted from peripheral blood samples and transferred to the Regeneron Genetics Center (RGC) for whole exome sequencing, and stored in automated biobanks at -80°C. Fluorescence-based quantification was performed to ensure appropriate DNA quantity and quality for sequencing purposes.

1 µg of DNA was sheared to an average fragment length of 150 base pairs (Covaris LE220) and prepared for exome capture with a custom reagent kit from Kapa Biosystems. Samples were captured using the NimbleGen SeqCap VCRome 2.1 or the Integrated DNA Technologies xGen exome target designs. Samples were barcoded, pooled, and multiplexed for sequenced using 75 bp paired-end sequencing on an Illumina HiSeq 2500 with v4 chemistry. Captured fragments were sequenced to achieve a minimum of 85% of the target bases covered at 20x or greater coverage. Following sequencing, data was processed using a cloud-based pipeline developed at the RGC that uses DNAnexus and AWS to run standard tools for sample-level data production and analysis. Briefly, sequence data were generated and de-multiplexed using Illumina's CASAVA software. Sequence reads were mapped and aligned to the GRCh37/hg19 human genome reference assembly using BWA-mem. After alignment, duplicate reads were marked and flagged using Picard tools and indels were realigned using GATK to improve variant call quality. SNP and INDEL variants and genotypes were called using GATK's HaplotypeCaller and Variant Quality Score Recalibration (VQSR) from GATK was applied to annotate the overall variant quality scores. Sequencing and data quality metric statistics were captured for each sample to evaluate capture performance, alignment performance, and variant calling.

### Example 3: Genomic Data Analyses

Standard quality-control filters for minimum read depth (>10), genotype quality (>30), and allelic balance (>20%) were applied to called variants. Passing variants were classified and annotated based on their potential functional effects (whether synonymous, nonsynonymous, splicing, frameshift, or nonframeshift variants) using an RGC developed annotation and analysis pipeline. Familial relationships were verified through identity by descent derived metrics from genetic data to infer relatedness and relationships in the cohort using PRIMUS (Staples et al., Amer. J. Human Genet., 2014, 95, 553-564) and cross-referencing with the reported pedigree for this family.

Pedigree-based variant analyses and segregation were performed to identify candidate disease genes under an autosomal dominant inheritance pattern given the reported family history. Shared variants between the affected proband and his affected mother but not shared with the unaffected father were subsequently annotated and filtered by their observed frequencies in population control databases such as dbSNP, the 1000 Genomes Project, the NHLBI Exome Sequencing Project, the Exome Aggregation Consortium Database (ExAc), and internal RGC databases to filter out common polymorphisms and high frequency, likely benign variants. Algorithms for bioinformatic prediction of functional effects of variants, such as LRT, Poly-phen2, SIFT, CADD, and Mutation Taster, along with conservation scores based on multiple species alignments (i.e. GERP, PhastCons, PhyloP) were incorporated as part of the annotation process of variants and used to inform on the potential deleteriousness of identified candidate variants.

A rare, truncating indel variant was identified in the SIGIRR gene (SIGIRR: c.557delA; p.K186fs*31) segregating with the early-onset inflammatory bowel disease in the 13-year-old patient, which was inherited from his IBD-affected mother. Referring to Figure 1 (panels A, B, and C), identification of a truncating variant in the *SIGIRR* gene with dominant segregation in a family with Crohn's Disease (CD) is shown. Panel A shows a table describing the truncating variant in SIGIRR at c.557delA/p.K186fs*31 with maternal inheritance; the variant site has a neutral conservation score across species and is predicted damaging to protein function; this variant has an alternate allele frequency of 0.000471 in the ExAC browser. Panel B shows a pedigree from the affected EO-IBD patient (Utah81427), his Crohn's Disease-affected mother (Utah81428), and unaffected father (Utah81429); filled symbols indicate CD-affected individuals, unfilled symbols indicate unaffected individuals; circles denote females and squares denote males. Panel C shows visual confirmation of the identified SIGIRR truncating variant segregating in CD-affected Utah81427 and his CD-affected mother, but is not observed in the unaffected father.

### Example 4: Detection

The presence of a certain genetic variant in a subject can indicate that the subject has an increased risk of having or developing early-onset inflammatory bowel disease. A sample, such as a blood sample, can be obtained from a subject. Nucleic acids can be isolated from the sample using common nucleic acid extraction kits. After isolating the nucleic acid from the sample obtained from the subject, the nucleic acid is sequenced to determine if there is a genetic variant present. The sequence of the nucleic acid can be compared to a control sequence (wild type sequence). Finding a difference between the nucleic acid obtained from the sample obtained from the subject and the control sequence indicates the presence of a genetic variant. These steps can be performed as described in the examples above and throughout the present disclosure. The presence of one or more genetic variants is indicative of the subject's increased risk for having or developing early-onset inflammatory bowel disease.

### Example 5: Studies

### Materials and methods

### Cell lines and Culture conditions:

Epstein-Barr Virus-transformed lymphoblastoid cell lines (LCLs) were generated from pediatric IBD patients with IRB-approval at the University of Utah Health Sciences Center. Healthy LCLs were purchased from American Type Cell Culture (ATCC; Manassas, VA). Cells were cultured in RPMI Medium 1640 (Gibco product ID: 12633) supplemented with 10% fetal bovine serum (Gibco product ID: 10438026), 1X pen-strep (Gibco product ID: 15140122), and 1X L-glutamine (Gibco product ID: 25030081).

### Stimulation conditions:

LCLs generated from healthy controls, the SIGIRR LoF patient, and LCLs from 4 EO IBD patients not harboring the SIGIRR LoF were stimulated with either 2mg/ml LPS (InvivoGen, San Diego, CA) for 72 hours or with 2mg/ml αIgM/αCD40 (Affymetrix, Santa Clara, CA) for 16 hours. Following stimulation, cell culture supernatants were collected and used to quantify secretion of pro-inflammatory cytokines using a Mesoscale Discovery V-Plex Human Pro-Inflammatory Cytokine panel (K15049D), and quantified using Mesoscale Discovery QuickPlex SQ 120, and was subsequently complemented with RNA-sequencing.

### Statistics:

Significance was determine using 2-way ANOVA test and S.E.M. was calculated using results from 3 independent experiments using GraphPad PRISM (La Jolla, CA)

### Results:

Referring to Figure 2, LCLs generated from the SIGIRR LoF patient produced more IFN-y, IL-2, IL-4, IL-6, IL-8, IL-10, IL-12p70, and TNF-α than healthy LCLs or LCLs from EO IBD patients not harboring SIGIRR LoFs. Further, unstimulated LCLs generated from the SIGIRR LoF patient secreted elevated levels of IFN-y, IL-1β, IL-2, IL-4, IL-6, IL-8, IL-10, IL-12p70, and TNF-α than healthy LCLs or some LCLs from EO IBD patients, indicating SIGIRR LoF LCLs are constitutively active and are refractory to LPS stimulation. Referring to Figure 2, blue bars indicate supernatants isolated from unstimulated cells; red bars indicate supernatants isolated from LPS-stimulated cells. "*" indicates p<0.05 by two-way Anova; error bars indicate S.E.M. from 3 independent experiments.

Referring to Figure 3, following αIgM/αCD40 stimulation, LCLs generated from the SIGIRR LoF patient produced more IFN-y, IL-2, IL-4, IL-6, IL-8, IL-10, IL-12p70, and TNF-α than healthy LCLs or some LCLs from EO IBD patients not harboring SIGIRR LoFs. Further, unstimulated LCLs generated from the SIGIRR LoF patient secreted more elevated levels of IFN-y, IL-2, IL-4, IL-6, IL-8, IL-10, and TNF-α than healthy LCLs or some LCLs from EO IBD patients, indicating SIGIRR LoF LCLs are constitutively active and are refractory to anti-IgM/anti-CD40 stimulation. Referring to Figure 3, blue bars indicate supernatants isolated from unstimulated cells; red bars indicate supernatants isolated from anti-IgM/anti-CD40 -stimulated cells; "*" indicates p<0.05 by two-way Anova; error bars indicate S.E.M. from 3 independent experiments.

In unstimulated cells, upregulation of key immune modulators, including IL-1β, IL-8, and IL-6 in the truncated (c.557delA; p.K186fs*31) SIGIRR EO-IBD patient LCLs relative to both LCLs generated from healthy controls and to LCLs generated from EO-IBD patients not harboring SIGIRR loss-of-function (LoF) variants was observed. This observation supports a unique inflammatory signature in IBD patients carrying SIGIRR LoF variants. Further, SIGIRR EO-IBD patient-derived LCLs were observed to be refractory to stimulation with either IL-1 β or TLR stimulation, supporting constitutive activation of these pro-inflammatory pathways in the absence of SIGIRR.

## Claims

1. A method for identifying a human subject having a risk for developing early-onset inflammatory bowel disease, wherein the method comprises detecting in a sample obtained from the subject the presence or absence of:
a truncated SIGIRR protein comprising an amino acid sequence that is at least 90% identical to SEQ ID NO:9, wherein the truncated SIGIRR protein comprises a serine at a position corresponding to position 186 according to SEQ ID NO:9 and is truncated at a position corresponding to position 215 according to SEQ ID NO:9; and/or
a nucleic acid molecule encoding a truncated SIGIRR protein comprising an amino acid sequence that is at least 90% identical to SEQ ID NO:9, wherein the truncated SIGIRR protein comprises a serine at a position corresponding to position 186 according to SEQ ID NO:9 and is truncated at a position corresponding to position 215 according to SEQ ID NO:9;
wherein the presence of the truncated SIGIRR protein and/or the nucleic acid molecule encoding the truncated SIGIRR protein indicates that the subject has a risk for developing early-onset inflammatory bowel disease.

2. A method for diagnosing early-onset inflammatory bowel disease in a human subject, comprising:
detecting a nucleic acid molecule encoding a truncated SIGIRR protein obtained from the human subject, wherein the truncated SIGIRR protein comprises an amino acid sequence that is at least 90% identical to SEQ ID NO:9, wherein the truncated SIGIRR protein comprises a serine at a position corresponding to position 186 according to SEQ ID NO:9 and is truncated at a position corresponding to position 215 according to SEQ ID NO:9; and/or
detecting a truncated SIGIRR protein obtained from the human subject, wherein the truncated SIGIRR protein comprises an amino acid sequence that is at least 90% identical to SEQ ID NO:9, wherein the truncated SIGIRR protein comprises a serine at a position corresponding to position 186 according to SEQ ID NO:9 and is truncated at a position corresponding to position 215 according to SEQ ID NO:9; and
diagnosing the human subject with early-onset inflammatory bowel disease if the subject has one or more symptoms of early-onset inflammatory bowel disease.

3. The method according to claims 1 or claim 2, wherein the truncated SIGIRR protein comprises the amino acid sequence of SEQ ID NO:11 at the positions corresponding to positions 186 to 215 according to SEQ ID NO:9.

4. The method according to any one of claims 1 to 3, wherein said nucleic acid molecule encoding said truncated SIGIRR protein is detected by detecting a frameshift mutation in said nucleic acid molecule creating a codon encoding a serine at the position corresponding to position 186 according to SEQ ID NO:9.

5. The method according to any one of claims 1 to 4, wherein the detecting step comprises sequencing at least a portion of the nucleic acid molecule that encodes a SIGIRR protein.

6. An isolated nucleic acid molecule comprising a nucleic acid sequence encoding a truncated human Single Immunoglobulin Interleukin-1 Receptor Related (SIGIRR) protein, wherein the truncated SIGIRR comprises an amino acid sequence that is at least 90% identical to SEQ ID NO:9, wherein the truncated SIGIRR protein comprises a serine at a position corresponding to position 186 according to SEQ ID NO:9 and is truncated at a position corresponding to position 215 according to SEQ ID NO:9, or the complement of the nucleic acid sequence.

7. The isolated nucleic acid molecule according to claim 6, wherein the nucleic acid molecule is mRNA and comprises a guanine at a position corresponding to position 557 according to SEQ ID NO:4.

8. The isolated nucleic acid molecule according to claim 6 or claim 7, wherein the truncated SIGIRR protein comprises the amino acid sequence of SEQ ID NO:11 at the positions corresponding to positions 186 to 215 according to SEQ ID NO:9.

9. A cDNA comprising a nucleic acid sequence encoding a truncated SIGIRR protein, wherein the truncated SIGIRR protein comprises an amino acid sequence that is at least 90% identical to SEQ ID NO:9, wherein the truncated SIGIRR protein comprises a serine at a position corresponding to position 186 according to SEQ ID NO:9 and is truncated at a position corresponding to position 215 according to SEQ ID NO:9.

10. The cDNA according to claim 9, wherein the truncated SIGIRR protein comprises the amino acid sequence of SEQ ID NO:11 at the positions corresponding to positions 186 to 215 according to SEQ ID NO:9.

11. The cDNA according to claim 9 or claim 10, wherein the cDNA comprises a guanine at a position corresponding to position 557 according to SEQ ID NO:6.

12. An isolated or recombinant polypeptide comprising a truncated SIGIRR protein, wherein the truncated SIGIRR protein comprising an amino acid sequence that is at least 90% identical to SEQ ID NO:9, wherein the truncated SIGIRR protein comprises a serine at a position corresponding to position 186 according to SEQ ID NO:9 and is truncated at a position corresponding to position 215 according to SEQ ID NO:9.

13. The isolated or recombinant polypeptide according to claim 12, wherein the truncated SIGIRR protein comprises the amino acid sequence of SEQ ID NO:11 at the positions corresponding to positions 186 to 215 according to SEQ ID NO:9.

14. An alteration-specific probe or primer comprising at least 15 nucleotides and having a nucleic acid sequence which is complementary to a nucleic acid sequence of a nucleic acid molecule encoding a truncated SIGIRR protein comprising an amino acid sequence that is at least 90% identical to SEQ ID NO:9, wherein the alteration-specific probe or primer comprises a nucleic acid sequence which is complementary to a portion of the nucleic acid molecule encompassing the codon which encodes a serine at the position corresponding to the position 186 according to SEQ ID NO:9.

## Patentansprüche

1. Verfahren zur Identifizierung eines menschlichen Individuums, bei dem ein Risiko für die Entwicklung einer früh einsetzenden chronisch-entzündlichen Darmerkrankung besteht, wobei das Verfahren den Nachweis des Vorhandenseins oder Fehlens von Folgendem in einer von dem Individuum entnommenen Probe umfasst:
ein verkürztes SIGIRR-Protein, das eine Aminosäuresequenz umfasst, die zu mindestens 90% mit SEQ ID NO:9 identisch ist, wobei das verkürzte SIGIRR-Protein ein Serin an einer Position umfasst, die der Position 186 gemäß SEQ ID NO:9 entspricht, und an einer Position verkürzt ist, die der Position 215 gemäß SEQ ID NO:9 entspricht; und/oder
ein Nukleinsäuremolekül, das ein verkürztes SIGIRR-Protein kodiert, das eine Aminosäuresequenz umfasst, die zu mindestens 90% mit SEQ ID NO:9 identisch ist, wobei das verkürzte SIGIRR-Protein ein Serin an einer Position umfasst, die der Position 186 gemäß SEQ ID NO:9 entspricht, und an einer Position verkürzt ist, die der Position 215 gemäß SEQ ID NO:9 entspricht;
wobei das Vorhandensein des verkürzten SIGIRR-Proteins und/oder des Nukleinsäuremoleküls, das das verkürzte SIGIRR-Protein kodiert, darauf hinweist, dass bei dem Individuum ein Risiko für die Entwicklung einer früh einsetzenden chronisch-entzündlichen Darmerkrankung besteht.

2. Verfahren zur Diagnose einer früh einsetzenden chronisch-entzündlichen Darmerkrankung bei einem menschlichen Individuum, umfassend:
Nachweis eines Nukleinsäuremoleküls, das ein verkürztes SIGIRR-Protein kodiert, das von dem menschlichen Individuum erhalten wurde, wobei das verkürzte SIGIRR-Protein eine Aminosäuresequenz umfasst, die zu mindestens 90% mit SEQ ID NO:9 identisch ist, wobei das verkürzte SIGIRR-Protein ein Serin an einer Position umfasst, die der Position 186 gemäß SEQ ID Nr. 9 entspricht, und an einer Position verkürzt ist, die der Position 215 gemäß SEQ ID Nr. 9 entspricht; und/oder
Nachweis eines verkürzten SIGIRR-Proteins, das von dem menschlichen Individuum erhalten wurde, wobei das verkürzte SIGIRR-Protein eine Aminosäuresequenz umfasst, die zu mindestens 90% mit SEQ ID NO:9 identisch ist, wobei das verkürzte SIGIRR-Protein ein Serin an einer Position umfasst, die der Position 186 gemäß SEQ ID NO:9 entspricht, und an einer Position verkürzt ist, die der Position 215 gemäß SEQ ID NO:9 entspricht; und
Diagnostizieren des menschlichen Individuums mit einer früh einsetzenden chronisch-entzündlichen Darmerkrankung, wenn das menschliche Individuum ein oder mehrere Symptom/e einer früh einsetzenden chronisch-entzündlichen Darmerkrankung aufweist.

3. Verfahren nach Anspruch 1 oder Anspruch 2, wobei das verkürzte SIGIRR-Protein die Aminosäuresequenz der SEQ ID NO:11 an den Positionen umfasst, die den Positionen 186 bis 215 gemäß SEQ ID NO:9 entsprechen.

4. Verfahren nach einem der Ansprüche 1 bis 3, wobei das Nukleinsäuremolekül, das das verkürzte SIGIRR-Protein kodiert, durch den Nachweis einer Frameshift-Mutation in dem Nukleinsäuremolekül nachgewiesen wird, die ein Codon erzeugt, das ein Serin an der Position kodiert, die der Position 186 gemäß SEQ ID NO:9 entspricht.

5. Verfahren nach einem der Ansprüche 1 bis 4, wobei der Nachweisschritt die Sequenzierung mindestens eines Teils des Nukleinsäuremoleküls umfasst, das ein SIGIRR-Protein kodiert.

6. Isoliertes Nukleinsäuremolekül, das eine Nukleinsäuresequenz umfasst, die für ein verkürztes menschliches Single Immunoglobulin Interleukin-1 Receptor Related (SIGIRR)-Protein kodiert, wobei das verkürzte SIGIRR eine Aminosäuresequenz umfasst, die zu mindestens 90% mit SEQ ID NO:9 identisch ist, wobei das verkürzte SIGIRR-Protein ein Serin an einer Position umfasst, die der Position 186 gemäß SEQ ID NO:9 entspricht, und an einer Position verkürzt ist, die der Position 215 gemäß SEQ ID NO:9 entspricht, oder das Komplement der Nukleinsäuresequenz.

7. Das isolierte Nukleinsäuremolekül nach Anspruch 6, wobei das Nukleinsäuremolekül mRNA ist und ein Guanin an einer Position umfasst, die der Position 557 gemäß SEQ ID NO:4 entspricht.

8. Das isolierte Nukleinsäuremolekül nach Anspruch 6 oder Anspruch 7, wobei das verkürzte SIGIRR-Protein die Aminosäuresequenz der SEQ ID NO:11 an den Positionen umfasst, die den Positionen 186 bis 215 gemäß SEQ ID NO:9 entsprechen.

9. cDNA, die eine Nukleinsäuresequenz umfasst, die ein verkürztes SIGIRR-Protein kodiert, wobei das verkürzte SIGIRR-Protein eine Aminosäuresequenz umfasst, die zu mindestens 90% mit SEQ ID Nr. 9 identisch ist, wobei das verkürzte SIGIRR-Protein ein Serin an einer Position umfasst, die der Position 186 gemäß SEQ ID Nr. 9 entspricht, und an einer Position verkürzt ist, die der Position 215 gemäß SEQ ID Nr. 9 entspricht.

10. Die cDNA nach Anspruch 9, wobei das verkürzte SIGIRR-Protein die Aminosäuresequenz von SEQ ID NO:11 an den Positionen umfasst, die den Positionen 186 bis 215 gemäß SEQ ID NO:9 entsprechen.

11. Die cDNA nach Anspruch 9 oder Anspruch 10, wobei die cDNA ein Guanin an einer Position umfasst, die Position 557 gemäß SEQ ID NO:6 entspricht.

12. Isoliertes oder rekombinantes Polypeptid, das ein verkürztes SIGIRR-Protein umfasst, wobei das verkürzte SIGIRR-Protein eine Aminosäuresequenz umfasst, die zu mindestens 90% mit SEQ ID NO:9 identisch ist, wobei das verkürzte SIGIRR-Protein ein Serin an einer Position umfasst, die der Position 186 gemäß SEQ ID NO:9 entspricht, und an einer Position verkürzt ist, die der Position 215 gemäß SEQ ID NO:9 entspricht.

13. Das isolierte oder rekombinante Polypeptid nach Anspruch 12, wobei das verkürzte SIGIRR-Protein die Aminosäuresequenz der SEQ ID NO:11 an den Positionen umfasst, die den Positionen 186 bis 215 gemäß SEQ ID NO:9 entsprechen.

14. Veränderungsspezifische Sonde oder Primer, die/der mindestens 15 Nukleotide umfasst und eine Nukleinsäuresequenz aufweist, die zu einer Nukleinsäuresequenz eines Nukleinsäuremoleküls komplementär ist, das ein verkürztes SIGIRR-Protein kodiert, das eine Aminosäuresequenz umfasst, die zu mindestens 90% mit SEQ ID NO:9 identisch ist, wobei die/der veränderungsspezifische Sonde oder Primer eine Nukleinsäuresequenz umfasst, die zu einem Teil des Nukleinsäuremoleküls komplementär ist, der das Codon beinhaltet, das ein Serin an der Position kodiert, die der Position 186 gemäß SEQ ID NO:9 entspricht.

## Revendications

1. Méthode pour identifier un sujet humain présentant un risque de développement d'une maladie intestinale inflammatoire précoce, laquelle méthode comprend la détection, dans un échantillon obtenu auprès du sujet, de la présence ou de l'absence de :
une protéine SIGIRR tronquée comprenant une séquence d'acides aminés qui est identique à au moins 90 % à la SEQ ID NO : 9, laquelle protéine SIGIRR tronquée comprend une sérine à une position correspondant à la position 186 de la SEQ ID NO : 9 et est tronquée à une position correspondant à la position 215 de la SEQ ID NO : 9 ; et/ou
une molécule d'acide nucléique codant pour une protéine SIGIRR tronquée comprenant une séquence d'acides aminés qui est identique à au moins 90 % à la SEQ ID NO : 9, dans laquelle la protéine SIGIRR tronquée comprend une sérine à une position correspondant à la position 186 de la SEQ ID NO : 9 et est tronquée à une position correspondant à la position 215 de la SEQ ID NO : 9 ;
dans laquelle la présence de la protéine SIGIRR tronquée et/ou de la molécule d'acide nucléique codant pour la protéine SIGIRR tronquée indique que le sujet présente un risque de développement d'une maladie intestinale inflammatoire précoce.

2. Méthode pour diagnostiquer une maladie intestinale inflammatoire précoce chez un sujet humain, comprenant :
la détection d'une molécule d'acide nucléique codant pour une protéine SIGIRR tronquée obtenue du sujet humain, dans laquelle la protéine SIGIRR tronquée comprend une séquence d'acides aminés qui est identique à au moins 90 % à la SEQ ID NO : 9, dans laquelle la protéine SIGIRR tronquée comprend une sérine à une position correspondant à la position 186 de la SEQ ID NO : 9 et est tronquée à une position correspondant à la position 215 de la SEQ ID NO : 9 ; et/ou
la détection d'une protéine SIGIRR tronquée obtenue du sujet humain, dans laquelle la protéine SIGIRR tronquée comprend une séquence d'acides aminés qui est identique à au moins 90 % à la SEQ ID NO : 9, dans laquelle la protéine SIGIRR tronquée comprend une sérine à une position correspondant à la position 186 de la SEQ ID NO : 9 et est tronquée à une position correspondant à la position 215 de la SEQ ID NO : 9 ; et
le diagnostic du sujet humain avec une maladie intestinale inflammatoire précoce si le sujet a un ou plusieurs symptômes de maladie intestinale inflammatoire précoce.

3. Méthode selon la revendication 1 ou la revendication 2, dans laquelle la protéine SIGIRR tronquée comprend la séquence d'acides aminés de la SEQ ID NO : 11 aux positions correspondant aux positions 186 à 215 de la SEQ ID NO : 9.

4. Méthode selon l'une quelconque des revendications 1 à 3, dans laquelle ladite molécule d'acide nucléique codant pour ladite protéine SIGIRR tronquée est détectée par détection d'un décalage du cadre de lecture dans ladite molécule d'acide nucléique créant un codon codant pour une sérine à la position correspondant à la position 186 de la SEQ ID NO : 9.

5. Méthode selon l'une quelconque des revendications 1 à 4, dans laquelle l'étape de détection comprend un séquençage d'au moins une portion de la molécule d'acide nucléique qui code pour une protéine SIGIRR.

6. Molécule d'acide nucléique isolée comprenant une séquence d'acide nucléique codant pour une protéine associée au récepteur d'interleukine-1 d'immunoglobuline unique (SIGIRR) humaine tronquée, dans laquelle la SIGIRR tronquée comprend une séquence d'acides aminés qui est identique à au moins 90 % à la SEQ ID NO : 9, dans laquelle la protéine SIGIRR tronquée comprend une sérine à une position correspondant à la position 186 de la SEQ ID NO : 9 et est tronquée à une position correspondant à la position 215 de la SEQ ID NO : 9, ou le complément de la séquence d'acide nucléique.

7. Molécule d'acide nucléique isolée selon la revendication 6, laquelle molécule d'acide nucléique est un ARNm et comprend une guanine à une position correspondant à la position 557 de la SEQ ID NO : 4.

8. Molécule d'acide nucléique isolée selon la revendication 6 ou la revendication 7, dans laquelle la protéine SIGIRR tronquée comprend la séquence d'acides aminés de la SEQ ID NO : 11 aux positions correspondant aux positions 186 à 215 de la SEQ ID NO : 9.

9. ADNc comprenant une séquence d'acide nucléique codant pour une protéine SIGIRR tronquée, dans lequel la protéine SIGIRR tronquée comprend une séquence d'acides aminés qui est identique à au moins 90 % à la SEQ ID NO : 9, dans lequel la protéine SIGIRR tronquée comprend une sérine à une position correspondant à la position 186 de la SEQ ID NO : 9 et est tronquée à une position correspondant à la position 215 de la SEQ ID NO : 9.

10. ADNc selon la revendication 9, dans lequel la protéine SIGIRR tronquée comprend la séquence d'acides aminés de la SEQ ID NO : 11 aux positions correspondant aux positions 186 à 215 de la SEQ ID NO : 9.

11. ADNc selon la revendication 9 ou la revendication 10, lequel ADNc comprend une guanine à une position correspondant à la position 557 de la SEQ ID NO : 6.

12. Polypeptide isolé ou recombiné comprenant une protéine SIGIRR tronquée, dans lequel la protéine SIGIRR tronquée comprend une séquence d'acides aminés qui est identique à au moins 90 % à la SEQ ID NO : 9, dans lequel la protéine SIGIRR tronquée comprend une sérine à une position correspondant à la position 186 de la SEQ ID NO : 9 et est tronquée à une position correspondant à la position 215 de la SEQ ID NO : 9.

13. Polypeptide isolé ou recombiné selon la revendication 12, dans lequel la protéine SIGIRR tronquée comprend la séquence d'acides aminés de la SEQ ID NO : 11 aux positions correspondant aux positions 186 à 215 de la SEQ ID NO : 9.

14. Amorce ou sonde spécifique d'une altération comprenant au moins 15 nucléotides et ayant une séquence d'acide nucléique qui est complémentaire d'une séquence d'acide nucléique d'une molécule d'acide nucléique codant pour une protéine SIGIRR tronquée comprenant une séquence d'acides aminés qui est identique à au moins 90 % à la SEQ ID NO : 9, laquelle amorce ou sonde spécifique d'une altération comprend une séquence d'acide nucléique qui est complémentaire d'une portion de la molécule d'acide nucléique englobant le codon qui code pour une sérine à la position correspondant à la position 186 de la SEQ ID NO : 9.
